# EUROPEAN PATENT SPECIFICATION

(11) **EP 3 148 537 B1**
(45) Date of publication and mention of the grant of the patent: **26.09.2018**
(21) Application number: 15745154.3
(22) Date of filing: 29.05.2015
(51) Int. Cl.: A61K 31/4192, A61K 31/445, A61K 31/451, A61K 31/454, A61P 31/18

(54) **CYCLIC COMPOUNDS HAVING A 1,3 DIAMINO-FUNCTIONALITY FOR USE IN THE TREATMENT OF HIV INFECTION**
CYCLISCHE VERBINDUNGEN MIT EINER 1,3-DIAMINO-FUNKTIONALITÄT ZUR VERWENDUNG BEI DER BEHANDLUNG VON HIV-INFEKTIONEN
COMPOSÉS CYCLIQUES PRÉSENTANT UNE FONCTIONNALITÉ 1,3 DIAMINO POUR UTILISATION DANS LE TRAITEMENT DE L'INFECTION PAR LE VIH

(30) Priority: 30.05.2014 EP 14305808
(43) Date of publication of application: 05.04.2017
(73) Proprietor: Université Paris Descartes, 75006 Paris (FR); Centre National de la Recherche Scientifique (CNRS), 75016 Paris (FR); Université Pierre et Marie Curie (Paris 6), 75005 Paris (FR); Institut National de la Santé et de la Recherche Médicale (INSERM), 75013 Paris (FR); Assistance Publique Hôpitaux de Paris, 75004 Paris (FR)
(72) Inventor: MICOUIN, Laurent, 75013 Paris (FR); BLOND, Aurélie, 92110 Clichy (FR); CALVEZ, Vincent, 75005 Paris (FR); MARCELLIN, Anne-Geneviève, 75005 Paris (FR); SOULIE, Cathia, 75012 Paris (FR); CORROT, Emilie, 27300 Bernay (FR)
(74) Representative: Regimbeau
(86) International application number: PCT/EP2015/062046
(87) International publication number: WO 2015/181387

(56) References cited:
- WO-A1-2013/129929
- WO-A2-2007/121429
- WO-A2-2010/003133
- US-B1- 6 750 348
- AURÉLIE BLOND ET AL: "Modular Access to N-Substituted cis -3,5-Diaminopiperidines", THE JOURNAL OF ORGANIC CHEMISTRY, vol. 78, no. 23, 6 December 2013 (2013-12-06), pages 12236-12242, XP055137715, ISSN: 0022-3263, DOI: 10.1021/jo401994y
- ROBA MOUMNÉ ET AL: "Tether influence on the binding properties of tRNALys3 ligands designed by a fragment-based approach", ORGANIC & BIOMOLECULAR CHEMISTRY, vol. 8, no. 5, 1 January 2010 (2010-01-01) , page 1154, XP055137718, ISSN: 1477-0520, DOI: 10.1039/b921232a
- MAIA CARNEVALI ET AL: "A Modular Approach to Synthetic RNA Binders of the Hepatitis C Virus Internal Ribosome Entry Site", CHEMBIOCHEM, vol. 11, no. 10, 16 June 2010 (2010-06-16) , pages 1364-1367, XP055137734, ISSN: 1439-4227, DOI: 10.1002/cbic.201000177
- ZHOU Y ET AL: "Antibacterial activity in serum of the 3,5-diamino-piperidine translation inhibitors", BIOORGANIC & MEDICINAL CHEMISTRY LETTERS, PERGAMON, AMSTERDAM, NL, vol. 18, no. 11, 1 June 2008 (2008-06-01), pages 3369-3375, XP022711230, ISSN: 0960-894X, DOI: 10.1016/J.BMCL.2008.04.023 [retrieved on 2008-04-13]

## Description

### Field of the invention:

The present invention concerns cyclic compounds having a 1,3 diamino-functionality, capable of reactivating HIV expression, for use in the treatment of HIV infection.

### Background Art:

At the end of 2011, an estimated 34 million people were living with Human Immunodeficiency Virus (HIV) and approximately 1.7 million people died of acquired immunodeficiency syndrome (AIDS).

HIV is an RNA-retrovirus replicating through a DNA intermediate that is integrated into the host cell's DNA. HIV infects and kills the cells of the immune system, including CD4+ T cells and macrophages, which are critical for mounting effective immune response against invading pathogens. Once

the viral DNA is integrated in the cell's chromosomes, HIV replicates directly into proteins or into RNA and infects new cells.

While significant progresses have been made in the treatment of the infection by HIV, in particular in the field of antiretroviral therapies, current treatments are not curative and must be taken by HIV-infected patients for the rest of their life in order to prevent progression of the infection to AIDS. Highly active antiretroviral therapies (HAART) have been developed in recent years. These HAART consist of drugs acting at different levels of the life cycle of HIV, inhibiting virus entry, reverse transcription, integration and/or maturation. These HAART are capable of eliminating the circulating viruses, with a viral load below the detection limits of modern assays (*i.e.* 50 copies of virion RNA / mL of plasma).

Yet, when the HAART is stopped, plasma viral load increases again. This rebound has been attributed to HIV reservoirs in which the pro-virus is present in a non-expressing state. The best understood reservoirs of HIV are CD4+ T cells: the cells exist in a resting state in which HIV-RNA and HIV-proteins are not expressed or expressed at very low level. As a consequence, these cells are not recognized by the immune system and form a reservoir of HIV, which, upon activation of these cells results in the proliferation of the virus and infection of other cells.

One of the approaches for eradicating these HIV reservoir cells is called the "activation/elimination" approach. The aim of this approach is to induce expression of viral HIV proteins inside the latent cells. The cells may then be killed by the damaging effects of virus production (viral cytopathic effect), apoptosis, or may be recognized by the immune system or therapeutic agents directed towards viral proteins.

One approach that has been developed consists in the stimulation of T-cells with interleukin-2 (IL-2). This approach however led to a rebound of viral load when the HAART was stopped. IL-2 in conjunction with an anti-CD3 monoclonal antibody resulted in toxic side effects.

Clinical trials are currently on-going to establish the safety and efficacy of compounds used to date as anticancer drugs, such as vorinostat (Zolinza®,Merck), LBH589 (Panibinostat®, Novartis) and Romidepsin. The first results indicate that these molecules result in important adverse effects.

Drugs intended for treating alcohol dependency have also been investigated (Disulfirame, Esperal® Sanofi-Aventis), but these compounds, studied in clinical trial, show low capacity to induce HIV replication.

Other preclinical trials are also on-going with NF-KB activators such as prostatin and bryostatin analogs, but the in-vivo safety and efficacy of these compounds has not been established to date.

To date, none of these drug classes has obtained its market authorization and there is therefore a need for new drugs capable of inducing HIV expression in latently infected reservoir cells.

Cyclic compounds having a 1,3 diamino-functionality are known from prior art. The 3,5-diamino-piperidyl scaffold has been shown to possess antiviral activity. These compounds target a structured RNA of the Hepatitis C Virus (HCV) that is essential for the initiation of viral protein synthesis, and inhibit virus replication. These compounds have also been described as antibacterial and antifungal agents. 1,3 diamino-cyclopentanes have also been described as antibacterial agents.

These cyclic 3,5 diamino-piperidines and 1,3 diamino-cyclopentanes share the property of being inhibitors of viral, bacterial and/or fungal proliferation, thereby reducing the load of pathogenic agents.

In a totally surprising and unexpected manner, the inventors of the present invention have discovered that cyclic compounds having a 1,3-diamino functionality act as promoters of HIV replication.

The unexpected potency of these compounds to induce the reactivation of HIV expression is particularly useful for the treatment of HIV infection, as it enables eradicating the reservoirs.

The present invention therefore concerns a compound of formula (I), a pharmaceutically acceptable salt, solvate or hydrate thereof, enantiomers, mixture of enantiomers, diastereoisomers and mixture of diasteroisomers thereof for use in the treatment of HIV infection of the following formula (I): 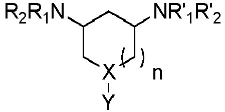 wherein:
n is 0 or 1,
X is CH or N,
Y is OR₃; NR₄R₅, or R₆,
R₁ and R'₁ are H, or R₁ and R₂ and/or R'₁ and R'₂ form together a (C₃-C₈)heterocyclyl,
R₂ and R'₂ are independently one from the other H, (C₁-C₆)alkyl, aryl, heteroaryl, (C₃-C₈)heterocyclyl, (C₃-C₈)carbocyclyl, (C₁-C₆)alkyl-aryl, (C₁-C₆)alkyl-heteroaryl, C(O)-Q, or SO₂-Z,
   Q is H, (C₁-C₆)alkyl, aryl, heteroaryl, (C₃-C₈)carbocyclyl, (C₃-C₈)heterocyclyl, (C₁-C₆)alkyl-aryl, (C₁-C₆)alkyl-heteroaryl, (C₁-C₆)alkyl-(C₃-C₈)heterocyclyl, (C₁-C₆)alkyl-(C₃-C₈)carbocyclyl, NRₐR_{b} or OR_{c},
      Rₐ and R_{b} are independently one from the other H, (C₁-C₆)alkyl, aryl, heteroaryl, (C₃-C₈)heterocyclyl, (C₃-C₈)carbocyclyl, (C₁-C₆)alkyl-aryl, (C₁-C₆)alkyl-heteroaryl, or Rₐ and R_{b} form together a (C₃-C₈)heterocyclyl,
      R_{c} is (C₁-C₆)alkyl, (C₃-C₈)heterocyclyl, (C₃-C₈)carbocyclyl, aryl, heteroaryl, (C₁-C₆)alkyl-aryl, or (C₁-C₆)alkyl-heteroaryl,
   Z is (C₁-C₆)-alkyl, aryl, heteroaryl, NRₐR_{b}, or CF₃,
R₃ is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, or (C₁-C₆)alkyl-heteroaryl-(C₁-C₆)alkyl-C(O)-aryl,
R₄ and R₅ are independently one from the other H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-V,
R₄ and R₅ form together a (C₃-C₈)heterocyclyl or a heteroaryl, or one of R₄ or R₅ is-CH(R₇)-CO-V,
   V is H, (C₁-C₆)alkyl, aryl, heteroaryl, (C₃-C₈)carbocyclyl, (C₃-C₈)heterocyclyl, (C₁-C₆)alkyl-aryl, (C₁-C₆)alkyl-heteroaryl, (C₁-C₆)alkyl-(C₃-C₈)heterocyclyl, (C₁-C₆)alkyl-(C₃-C₈)carbocyclyl, NR_{f}R_{g}, OR₁₀ or CH(R₁₁)-NH-COR₁₂,
   R₁₀ is as defined for R_{c},
   R₇ is the side chain of an amino-acid,
   R₁₁ is (C1-C6) alkylamine,
   R₁₂ is aryl,
R₆ is H, (C₁-C₆)alkyl, (C₃-C₈)heterocyclyl, (C₃-C₈)carbocyclyl, (C₉-C₁₀)carbocyclyl, aryl, heteroaryl, (C₁-C₆)alkyl-aryl, (C₁-C₆)alkyl-heteroaryl, , NR_{d}Rₑ, OR₉, C(O)-V, SO₂-W, or-CH(R₇)-CO-V,
   R_{d} and Rₑ are independently one from the other H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, C(O)-(C₁-C₆)alkyl-(C₃-C₈)carbocyclyl, or R_{d} and Rₑ form together a (C₃-C₈)heterocyclyl,
   R_{f} is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, or C(O)-(C₁-C₆)alkyl-(C₃-C₈)carbocyclyl,
   R_{g} is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, or R_{f} and R_{g} form together a (C₃-C₈)heterocyclyl,
   R₉ is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, or C(O)-(C₁-C₆)alkyl-(C₃-C₈)carbocyclyl,
   W is as defined for Z,
   V is as defined above.
for all radicals R₁ to R₁₂, R_{1'}, R_{2'}, Rₐ to R_{g}, Q, V, W and Z:
said (C₃-C₈)heterocyclyl can be substituted by one or more groups such as methyl, ethyl, isopropyl, hydroxy, methoxy, amino, fluoro, chloro, bromo and iodo,
said aryl may be substituted with one or more groups independently selected from the group consisting of alkyl, alkoxy, halogen, hydroxyl, amino, nitro, cyano, trifluoro, carboxylic acid or carboxylic ester, and
said heteroaryl may be substituted with one or more groups independently selected from the group consisting of alkyl, alkoxy, halogen, hydroxyl, amino, nitro, cyano, trifluoro, carboxylic acid or carboxylic ester,

Advantageously:
n is 0 or 1,
X is CH or N,
Y is OR₃; NR₄R₅, or R₆,
R₁ and R'₁ are H, or R₁ and R₂ and/or R'₁ and R'₂ form together a (C₃-C₈)heterocyclyl,
R₂ and R'₂ are independently one from the other H, (C₁-C₆)alkyl, aryl, heteroaryl, (C₃-C₈)heterocyclyl, (C₃-C₈)carbocyclyl, (C₁-C₆)alkyl-aryl, (C₁-C₆)alkyl-heteroaryl, C(O)-Q, or SO₂-Z,
   Q is H, (C₁-C₆)alkyl, aryl, heteroaryl, (C₃-C₈)carbocyclyl, (C₃-C₈)heterocyclyl, (C₁-C₆)alkyl-aryl, (C₁-C₆)alkyl-heteroaryl, (C₁-C₆)alkyl-(C₃-C₈)heterocyclyl, (C₁-C₆)alkyl-(C₃-C₈)carbocyclyl, NRₐR_{b} or OR_{c},
   Rₐ and R_{b} are independently one from the other H, (C₁-C₆)alkyl, aryl, heteroaryl, (C₃-C₈)heterocyclyl, (C₃-C₈)carbocyclyl, (C₁-C₆)alkyl-aryl, (C₁-C₆)alkyl-heteroaryl, or Rₐ and R_{b} form together a (C₃-C₈)heterocyclyl,
   R_{c} is (C₁-C₆)alkyl, (C₃-C₈)heterocyclyl, (C₃-C₈)carbocyclyl, aryl, heteroaryl, (C₁-C₆)alkyl-aryl, or (C₁-C₆)alkyl-heteroaryl,
   Z is (C₁-C₆)-alkyl, aryl, heteroaryl, NRₐR_{b}, or CF₃,
R₃ is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, or (C₁-C₆)alkyl-heteroaryl-(C₁-C₆)alkyl-C(O)-aryl,
R₄ and R₅ are independently one from the other H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-V, R₄ and R₅ form together a (C₃-C₈)heterocyclyl or a heteroaryl, or one of R₄ or R₅ is -CH(R₇)-CO-V,
   V is H, (C₁-C₆)alkyl, aryl, heteroaryl, (C₃-C₈)carbocyclyl, (C₃-C₈)heterocyclyl, (C₁-C₆)alkyl-aryl, (C₁-C₆)alkyl-heteroaryl, (C₁-C₆)alkyl-(C₃-C₈)heterocyclyl, (C₁-C₆)alkyl-(C₃-C₈)carbocyclyl, NR_{f}R_{g} or OR₁₀,
   R₇ is the side chain of an amino-acid,
R₆ is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, NR_{d}Rₑ, OR₉, C(O)-V, SO₂-W, or -CH(R₇)-CO-V,
   R_{d} and Rₑ are independently one from the other H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, C(O)-(C₁-C₆)alkyl-(C₃-C₈)carbocyclyl, or R_{d} and Rₑ form together a (C₃-C₈)heterocyclyl,
   R_{f} is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, or C(O)-(C₁-C₆)alkyl-(C₃-C₈)carbocyclyl,
   R_{g} is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, or R_{f} and R_{g} form together a (C₃-C₈)heterocyclyl,
   R₉ is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, or C(O)-(C₁-C₆)alkyl-(C₃-C₈)carbocyclyl,
   R₁₀ is as defined for R_{c}, and
   W is as defined for Z.

Advantageously, NR₁R₂ and NR'₁R'₂ are in *cis* configuration.

Advantageously, R₂ and R'₂ are H, C(O)-Q, SO₂-Z as defined above, or R₁ and R₂ and R'₁ and R'₂ form together a (C₃-C₈)heterocyclyl. Advantageously, Q is H, (C₁-C₆)alkyl, such as methyl, (C₁-C₆)alkyl-aryl, such as benzyl, or OR_{c}, where R_{c} is as defined above and is advantageously (C₁-C₆)alkyl or (C₁-C₆)alkyl-aryl. Advantageously, Z is aryl or NRₐR_{b}.

More advantageously, R₂ and R'₂ are H.

Advantageously, R₃ is aryl or (C₁-C₆)alkyl-heteroaryl-(C₁-C₆)alkyl-C(O)-aryl, more advantageously (C₁-C₆)alkyl-heteroaryl-(C₁-C₆)alkyl-C(O)-aryl. Preferred (C₁-C₆)alkyl-heteroaryl-(C₁-C₆)alkyl-C(O)-aryl is CH₂-1,2,3-triazolyl-CH₂-C(O)-p-cyclophanyl.

Advantageously, R₄ and R₅ are independently one from the other H, (C₁-C₆)alkyl, (C₃-C₈)heterocyclyl, (C₃- C₈)carbocyclyl, aryl, heteroaryl, (C₁-C₆)alkyl-aryl, (C₁-C₆)alkyl-heteroaryl, C(O)-V, R₄ and R₅ form together a (C₃-C₈)heterocyclyl or a heteroaryl, or one of R₄ or R₅ is-CH(R₇)-CO-V,

Advantageously, R₆ is C(O)-V, where V is as defined for formula (I), V being advantageously (C₁-C₆)alkyl-aryl or OR₁₀, R₁₀ being advantageously (C₁-C₆)alkyl, preferably *tert*-butyl or (C₁-C₆)alkyl-aryl, preferably benzyl.

The compounds of formula (I) can be prepared according to the methods described in WO 2009/099897, US6316626, WO 2006/024784 and Journal of Organic Chemistry 2013, 78, 12236-12242 (doi: 10.1021/jo401994y*).*

In a first advantageous embodiment, the compounds of formula (I) are 1,3-diaminocyclopentanes, wherein n = 0 and X is CH of formula (I-1): 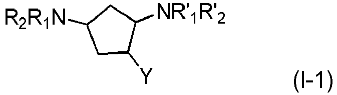 wherein R₁, R'₁, R₂, R'₂ and Y are as defined in formula (I).

Advantageously, Y is OR₃ or NR₄R₅, preferably OR₃, as defined in formula (I).

Advantageously, NR₁R₂ and NR'₁R'₂ are in *cis* configuration.

Advantageously, R₂ and R'₂ are H, C(O)-Q, SO₂-Z as defined above, or R₁ and R₂ and R'₁ and R'₂ form together a (C₃-C₈)heterocyclyl. Advantageously, Q is H, (C₁-C₆)alkyl, such as methyl, (C₁-C₆)alkyl-aryl, such as benzyl, or OR_{c}, where R_{c} is as defined above, and is advantageously (C₁-C₆)alkyl or (C₁-C₆)alkyl-aryl. Advantageously, Z is aryl or NRₐR_{b}.

More advantageously, R₂ and R'₂ are H.

Advantageously, R₃ is aryl or (C₁-C₆)alkyl-heteroaryl-(C₁-C₆)alkyl-C(O)-aryl, more advantageously (C₁-C₆)alkyl-heteroaryl-(C₁-C₆)alkyl-C(O)-aryl. Preferred (C₁-C₆)alkyl-heteroaryl-(C₁-C₆)alkyl-C(O)-aryl is -CH₂-1,2,3-triazolyl-CH₂-C(O)-p-cyclophanyl

Advantageously, R₄ and R₅ are independently one from the other H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-V, R₄ and R₅ form together a (C₃-C₈)heterocyclyl or a heteroaryl, or one of R₄ or R₅ is-CH(R₇)-CO-V,

In a second advantageous embodiment, the compounds of formula (I) are 3, 5-diaminopiperidines wherein n = 1 and X is N of formula (I-2): 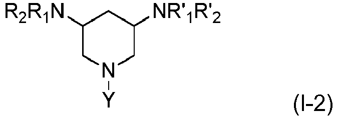 wherein R₁, R'₁, R₂, R'₂, X and Y are as defined for formula (I).

Advantageously, Y is R₆, as defined in formula (I).

Advantageously, NR₁R₂ and NR'₁R'₂ are in *cis* configuration.

Advantageously, R₂ and R'₂ are H, C(O)-Q, SO₂-Z as defined above, or R₁ and R₂ and R'₁ and R'₂ form together a (C₃-C₈)heterocyclyl. Advantageously, Q is H, (C₁-C₆)alkyl, such as methyl, (C₁-C₆)alkyl-aryl, such as benzyl, or OR_{c}, where R_{c} is as defined above. Advantageously, Z is aryl or NRₐR_{b}.

More advantageously, R₂ and R'₂ are H.

Advantageously, R₆ is H, aryl, heteroaryl, (C₁-C₆)alkyl-aryl, (C₁-C₆)alkyl-heteroaryl, C(O)-V, or -CH(R₇)-CO-V, where V is as defined for formula (I), V being advantageously (C₁-C₆)alkyl-aryl, CH(R₁₁)-NH-COR₁₂, R₁₁ being advantageously (C₁-C₆)alkylamine, preferably butylamine and R₁₂ being an aryl, preferably a phenyl or OR₁₀, R₁₀ being advantageously (C₁-C₆)alkyl, preferably *tert*-butyl or (C₁-C₆)alkyl-aryl, preferably benzyl.

Advantageously, when present, the aryl in R₆ is chosen from among methoxyphenyl, advantageously 3-methoxyphenyl or 4-methoxyphenyl, ethoxyphenyl, advantageously 4-ethoxyphenyl dimethoxyphenyl, advantageously, 3,4-dimethoxyphenyl, trimethoxyphenyl, advantageously 3,4,5-trimethoxyphenyl, 9,9'-Spirobi[9H-fluorene], p-cyclophanyl (hydroxyphenyl)amide, advantageously 3-(hydroxyphenyl)-4-benzamide, ethylphenyl, advantageously 4-ethylphenyl and phenylethanol, advantageously 4-phenylethanol.

Advantageously, when present, the heteroaryl is a 3- or 5-indolyl, advantageously substituted with a methoxy group.

Advantageously, the compound of formula (I) is selected in the list consisting of:

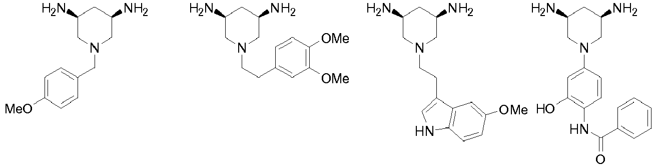

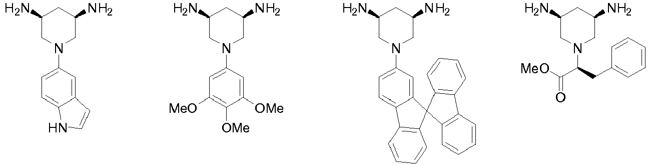

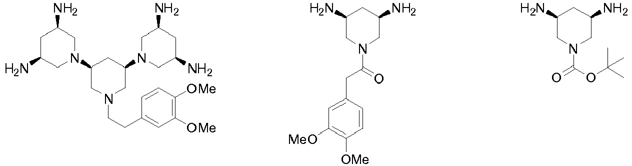

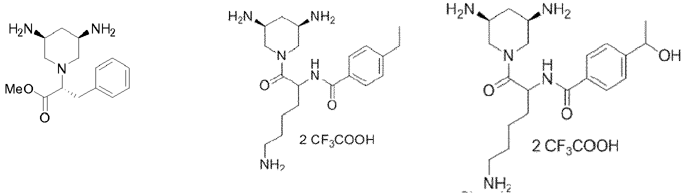

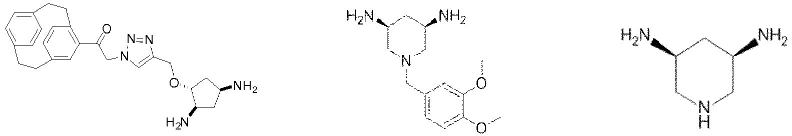

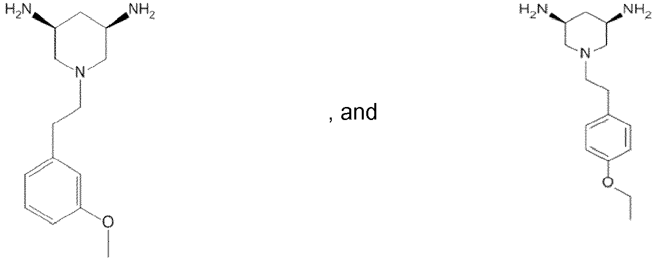

In a third advantageous embodiment, the compounds of formula (I) are 3, 5-diaminopiperidines wherein n = 1 and X is N of formula (I-2):
wherein R₁, R'₁, R₂, R'₂, X and Y are as defined for formula (I).

Advantageously, Y is R₆, as defined in formula (I).

Advantageously, NR₁R₂ and NR'₁R'₂ are in *cis* configuration.

Advantageously, at least one of R₂ and R'₂ is H, C(O)-Q or SO₂-Z as defined in formula (I). Advantageously, Q is OR_{c}, R_{c} being (C₁-C₆)alkyl, advantageously *tert*-butyl. Advantageously, Z is (C₁-C₆)alkyl.

More advantageously, R₂ and R'₂ are C(O)-Q, R₂ is H and R'₂ is SO₂-Z or R₂ is C(O)-Q and R'₂ is H.

Advantageously, R₆ is H, (C₁-C₆)alkyl-aryl, (C₁-C₆)alkyl-heteroaryl or SO₂-W wherein W is a defined above.

Advantageously, when present, the aryl in R₆ is chosen from among phenyl, methoxyphenyl, advantageously 3-methoxyphenyl, N,N-dimethylphenylamine and phenylpyrrolidine.

Advantageously, when present, the heteroaryl is a 1-methyl-5-indolyl, advantageously substituted with a methoxy group.

Advantageously, the compound of formula (I) is selected in the list consisting of:

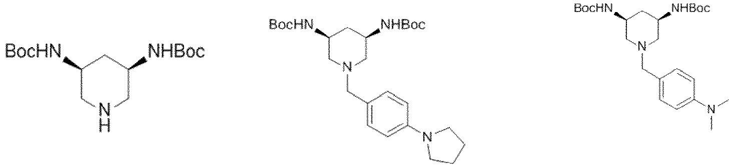

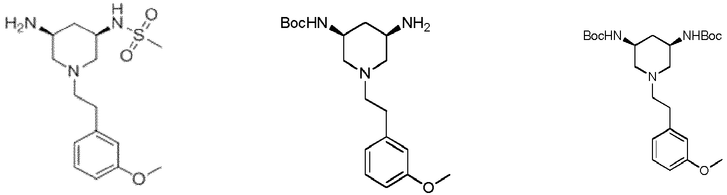

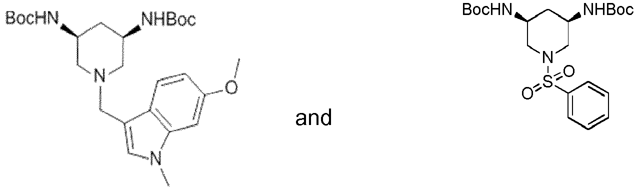

The compound of formula (I) can also be used in the form of a pro-drug. By pro-drug, it is meant in the sense of the present invention, a compound that is administered in an inactive or less active form and that is metabolized *in vivo* into its active form, for example under the action of enzymes or gastric juice. Pro-drugs are useful to improve the physicochemical properties of a molecule, such as solubility or pharmacokinetics (bioavailability for example). In particular, a pro-drug may be obtained by acylation or phosphorylation of an amine or a hydroxyl group.

The compounds of formula (I) can also be used in combination with one or more HIV-1 inducers. HIV inducers are compounds capable of activating HIV-protein expression and include DNA methylation inhibitors, such as 5-azacytidine (azacitidine), 5-aza-2'-deoxycytidine (5-aza-CdR, decitabine), 1-Darabinofuranosyl-5-azacytosine (fazarabine), dihydro-5-azacytidine (DHAC), 5-fluorodeoxycytidine (FdC), oligodeoxynucleotide, duplexes containing 2-H pyrimidinone, zebularine, antisense oligodeoxynucleotides (ODNs), MG98, (-)-epigallocatechin-3-gallate, hydralazine, procaine and procainamide; histone deacetylase inhibitors, such as TSA, SAHA, MS-275, aminosuberoyl hydroxamic acids, M-Carboxycinnamic acid bishydroxamate, LAQ-824, LBH-589, belinostat (PXD-101), Panobinostat (LBH-589), a cinnamic hydroxamic acid analogue of M-carboxycinnamic acid bishydroxamate, IF2357, aryloxyalkanoic acid hydroxamides, depsipeptide, apicidin, cyclic hydroxamic acid-containing peptide group of molecules, FK-228, red FK, cyclic peptide mimic linked by an aliphatic chain to a hydroxamic acid, butyrate, phenylbutyrate, sodium butyrate, valproic acid, pivaloyloxymethyl butyrate, 5 NOX-275, and MGCD0103; or NF-kappa-B-inducers selected from the group comprising: PMA, prostratin, bryostatin and TNF-alpha.

Advantageously, at least one compound of formula (I) is used in combination with an HIV therapy, such as immunotherapy, antiretrovirals, vaccines, such as therapeutic vaccines, and Highly Active Antiretroviral therapies (HAART).

Vaccines are for example therapeutic vaccines, more particularly anti-HIV vaccines, capable of restoring cell-mediated and/or humoral immunity to HIV-infected patients. These vaccines may be combined with cytokines that increase the response to the vaccine and/or restore the immune system by stimulating the growth of certain cells, such as CD4 lymphocytes.

The present invention therefore also concerns at least a compound of formula (I) for use in the treatment of HIV infection in combination with an HIV therapy. HIV therapies are known in the art and include: Lamivudine, Emtricitabine, Abacavir, Zidovudine, Didanosine, Stavudine, Adefovir, Tenofovir, Efavirenz, Etravirine, Nevirapine, Rilpivirine, Amprénavir, Fosamprénavir, Tipranavir, Lopinavir, Ritonavir, Indinavir, Saquinavir, Darunavir, Atazanavir, Nelfinavir, Raltegravir, Eviltegravir, Dolutegravir, Enfuvirtide, Maraviroc, and combinations thereof.

Advantageously, at least one product of formula (I) is used with an HIV therapy chosen, for example, from among: Lamivudine, Emtricitabine, Abacavir, Zidovudine, Didanosine, Stavudine, Adefovir, Tenofovir, Efavirenz, Etravirine, Nevirapine, Rilpivirine, Amprenavir, Fosamprenavir, Tipranavir, Lopinavir, Ritonavir, Indinavir, Saquinavir, Darunavir, Atazanavir, Nelfinavir, Raltegravir, Eviltegravir, Dolutégravir, Enfuvirtide, Maraviroc.

The present invention also concerns a pharmaceutical composition, comprising at least one compound of formula (I) as defined above for use in the treatment of HIV infection, advantageously in combination with an HIV therapy.

The pharmaceutical compositions of the invention can be intended for oral, sublingual, subcutaneous, intramuscular, intravenous, transdermal or rectal administration. The active ingredient can be administered in unit forms for administration, mixed with conventional pharmaceutical carriers, to animals or to humans. The pharmaceutical compositions may be immediate, delayed or sustained release compositions, advantageously sustained release compositions.

When a solid composition is prepared in the form of tablets, the main active ingredient is mixed with a pharmaceutical vehicle and other conventional excipients known to those skilled in the art.

The compounds of the invention can be used in a pharmaceutical composition at a dose ranging from 0.01 mg to 1000 mg a day, administered in only one dose once a day or in several doses along the day, for example twice a day. The daily administered dose is advantageously comprised between 5 mg and 500 mg, and more advantageously between 10 mg and 200 mg. However, it can be necessary to use doses out of these ranges, which could be noticed by the person skilled in the art.

The present invention further concerns the use of at least one compound of formula (I) for the preparation of a medicament intended for the treatment of HIV infection, said medicament being used advantageously in combination with an HIV therapy.

The present invention further concerns a method for treating HIV infection, comprising the administration to a person in need thereof of at least one compound of formula (I), advantageously in combination with an HIV therapy.

The present invention also concerns a combination product comprising:
(i) at least one compound of formula (I) as defined above,
(ii) at least one antiretroviral of HIV,
for simultaneous, separate or sequential use as a medicament.

Advantageously, the combination product is intended for the treatment of HIV infection.

Advantageously, the at least one antiretroviral of HIV is chosen from among entry inhibitors (or fusion inhibitors) such as Maraviroc and Enfuvirtide; nucleoside reverse transcriptase inhibitors (NRTI) and nucleotide reverse transcriptase inhibitors (NtRTI) such as Lamivudine, Emtricitabine, Abacavir, Zidovudine, Didanosine, Stavudine, Adéfovir, Tenofovir; non-Nucleoside reverse transcriptase inhibitors (NNRTI), such as , Efavirenz, Etravirine, Nevirapine, Rilpivirine,; integrase inhibitors, such as Raltegravir,Elvitegravir and Dolutegravir; protease inhibitors, such as Amprenavir, Fosamprenavir, Tipranavir, Lopinavir, Ritonavir, Indinavir, Saquinavir, Darunavir, Atazanavir, Nelfinavir.

Advantageously, the compound of formula (I) is a compound of formula (I-1) or a compound of formula (I-2).

The combination product according to the invention may be administered in the form of a single pharmaceutical composition comprising at least one compound of formula (I) and at least one antiretroviral of HIV. The combination product according to the invention may also be administered in the form of a first pharmaceutical composition comprising the at least one compound of formula (I) and a second pharmaceutical composition comprising the at least one antiretroviral. In that case, the pharmaceutical compositions may be administered by the same or by different routes. For example, one pharmaceutical composition can be administered orally and the second one parenterally.

### Definitions:

The term HIV, in the sense of the present invention, is intended to designate the three types of the human immunodeficiency virus HIV0, HIV 1 and HIV2 and their subtypes.

Within the groups, radicals or fragments defined in the description and the claims, the number of carbon atoms is specified inside the brackets. For example, (C₁-C₆)alkyl designates an alkyl group or radical having 1 to 6 carbon atoms.

For the groups comprising two or more subgroups, the attachment is indicated with "-". For example, "-(C₁-C₆)alkyl-aryl-(C₁-C₆)alkenyl" indicates a radical alkyl bound to a radical aryl itself bound to an alkenyl wherein the alkyl is bound to the rest of the molecule.

In the sense of the present invention, the expression "(C₁-C₆)alkyl" designates an acyclic, saturated, linear or branched hydrocarbon chain comprising 1 to 6 carbon atoms. Examples of (C₁-C₆)alkyl groups include methyl, ethyl, propyl, butyl, pentyl or hexyl. Unless explicitly stated, the definitions propyl, butyl, pentyl and hexyl include all possible isomers. For example, butyl comprises *n*-butyl, *iso*-butyl, *sec*-butyl and *tert*-butyl.

In the sense of the present invention, the expression "(C₁-C₆)alkylamine" designates an amine group bound to the molecule via an "(C₁-C₆)alkyl" as defined above. Examples of (C₁-C₆) alkylamine groups include methylamine, ethylamine, propylamine, butylamine, pentylamine or hexylamine.

In the sense of the present invention, the expression "(C₃-C₈)carbocyclyl" designates a saturated or partially saturated mono-, di- or tri-cyclic structure comprising from 3 to 8 carbon atoms. Examples of "(C₃-C₈)carbocyclyl" include cyclopropyl, cyclobutyl, cyclopentyl, cyclohexyl, cycloheptyl or cyclooctyl. Unless explicitly stated, the carbocyclyl can be substituted by one or more groups selected from methyl, ethyl, isopropyl, hydroxy, methoxy, amino, fluoro, chloro, bromo and iodo.

In the sense of the present invention, the expression "(C₉-C₁₀)carbocyclyl" designates a saturated or partially saturated di- or tri-cyclic structure comprising from 9 to 10 carbon atoms.

In the sense of the present invention, the expression "(C₃-C₈)heterocyclyl" designates saturated heterocycles having 3, 4, 5, 6, 7 or 8 atoms in the ring where 1, 2 or 3 heteroatoms chosen from among N, O and S replace the corresponding number of carbon atoms. Examples of "(C₃-C₈)heterocyclyl" include aziridinyl, oxyranyl, pyrrolidinyl, tetrahydrofuranyl, oxazolyl, piperidinyl, piperazinyl and morpholinyl. Unless explicitly stated, the heterocyclyl can be substituted by one or more groups selected from methyl, ethyl, isopropyl, hydroxy, methoxy, amino, fluoro, chloro, bromo and iodo.

In the sense of the present invention, the expression "Rₓ and R_{y} form together a (C₃-C₈)heterocyclyl" is intended to mean that N, Rₓ and R_{y} represent together an heterocycle. For example, Rₓ and R_{y} can be connected to form a C₄-alkyl chain, forming a pyrrolidinyl ring with the nitrogen atom they are connected to.

The term "aryl" designates an aromatic, monocyclic ring that may be fused with a second saturated, unsaturated or aromatic ring. The term aryl include, without restriction to the following examples, phenyl, indanyl, indenyl, naphtyl, anthracenyl, phenanthrenyl, tetrahydronaphtyl, dihydronaphtyl, 9,9'-Spirobi[9H-fluorene] and p-cyclophanyl. The preferred aryl are those comprising one six-membered aromatic ring. The aryl group may be substituted with one or more groups independently selected from the group consisting of alkyl, alkoxy, halogen, hydroxyl, amino, nitro, cyano, trifluoro, carboxylic acid or carboxylic ester. Examples of substituted phenyl groups are 2-, 3- or 4-methoxyphenyl, 3, 5- or 3, 4-dimethoxyphenyl, 3, 4, 5-trimethoxyphenyl, 2-, 3- or 4-hydroxyphenyl, 4-acylamidophenyl or 4-acylamido-3-hydroxy-phenyl.

The term heteroaryl designates a mono- or polycyclic aryl as defined above where one or more carbon atoms have been replaced with one or more heteroatoms chosen from among N, O and S. Unless explicitly stated, the term "heteroaryl" includes all possible isomers. Examples of heteroaryl groups include furyl, thienyl, imidazolyl, pyridyl, pyrrolyl, N-alkyl pyrrolyl, pyrimidinyl, pyrazinyl, tetrazolyl, triazolyl and triazinyl. The heteroaryl group may be substituted with one or more groups independently selected from the group consisting of alkyl, alkoxy, halogen, hydroxyl, amino, nitro, cyano, trifluoro, carboxylic acid or carboxylic ester. Preferred heteroaryls are those having 5 or 6 atoms in the ring, such as indolyl, pyrrolyl, pyridinyl, pyrrazolyl, triazolyl, furanyl or thienyl.

In the sense of the present invention, the term "halogen" designates a fluorine, chlorine, bromine or iodine atom.

The term "amino acid" as used in the present invention refers to natural α-amino acids (e.g. Alanine (Ala), Arginine (Arg), Asparagine (Asn), Aspartic acid (Asp), Cysteine (Cys), Glutamine (Gln), Glutamic acid (Glu), Glycine (Gly), Histidine (His), Isoleucine (Ile), Leucine (Leu), Lysine (Lys), Methionine (Met), Phenylalanine (Phe), Proline (Pro), Serine (Ser), Threonine (Thr), Tryptophan (Trp), Tyrosine (Tyr) and Valine (Val)) in the D or L form, as well as non-natural amino acid. In the sense of the present invention, the definition "R₇ is the side chain of an aminoacid" is to be understood in its common meaning. By way of illustration, R₇ is a CH₂-phenyl group in phenylalanine.

For the purpose of the invention, the term "pharmaceutically acceptable" is intended to mean what is useful to the preparation of a pharmaceutical composition, and what is generally safe and non-toxic, for a pharmaceutical use.

The term « pharmaceutically acceptable salt, hydrate of solvate » is intended to mean, in the framework of the present invention, a salt of a compound which is pharmaceutically acceptable, as defined above, and which possesses the pharmacological activity of the corresponding compound. Such salts comprise:
(1) hydrates and solvates,
(2) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(3) salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

### Examples:

### • Example 1 : Test of Efficacy and cytotoxicity on Hela and J-Lat cells

### Protocol:

### Hela-p4 cells

HeLa-p4 cells are HeLa CD4 LTR-LacZ wherein LacZ expression is induced by the trans-activating protein Tat of HIV, making possible the precise quantification of HIV-1 infectivity from a single replication cycle. HeLa-CD4 cells growing exponentially at a density of 1 x 104/mL were placed in 96-well plates and infected the following day with 1 ng of HIV p24 antigen in the presence of different concentrations of compounds. The titles for a single cycle of the viruses were determined 48 hours after infection by quantifying the beta-galactosidase activity in lysates P4 by colorimetric test (CPRG, Promega) based on the cleavage of chlorophenol red-beta-D-galactopyranoside (CPRG) by beta-galactosidase. A cell viability assay measuring the absorbance at 690 nm using the yellow tetrazolium reagent MTS [3- (4,5-dimethyl-2-thiazolyl) -2,5-diphenyl tetrazolium bromide] (Promega) was carried out.

### J-Lat cells

The J-Lat were grown in RPMI 1640 medium (Gibco-BRL) supplemented with 10% fetal bovine serum, 50 U/ml of penicillin, 50 mg/ml of streptomycin at 37uC in a humidified 95% air/5% CO2 atmosphere. The cells were plated at 5.105 cells in a 96 well-plate. TNF□ was purchased from Immunosource. SAHA (suberoylanilide hydroxamic acid), prostratin (12-deoxyphorbol-13-acetate) and vorinostat were obtained from Sigma-Aldrich. The J-Lat cells were treated for 24 h with the different compounds alone. The cells were washed twice in PBS, re-suspended in PBS containing 4% paraformaldehyde and fixed for 30 min. Cells were next washed and re-suspended in PBS. The percentage of GFP-positive cells was measured with a LSRFortessa cytometer (Becton-Dickinson) using FACSDiva Version 6.1.3 according to the manufacturer's instructions.

### Results:

For the helap4 cells experiments, the efficacy of the compounds is determined in comparison with control cells containing wild type NL4-3 virus alone (i.e. without any added compound) and is expressed as percentage of control. Then, the viral replication was increased for several compounds at the 50 and 100 µM concentrations with an absence of cytotoxic effects at the same concentrations (MTS test) (table 1).

The results for the J-Lat cells experiments are expressed as a percentage of the living fluorescent cells. In this model, some slight increase of viral production measured by fluorescence are quantified (table 1).

**Table 1**

| **Name** | **Structure** | **Hela-p4** | | | | | | **J-lat** | |
|---|---|---|---|---|---|---|---|---|---|
| | | **CPRG 50 µM** | **CPRG 100 µM** | **CPRG 200µM** | **MTS 50 µM** | **MTS 100 µM** | **MTS 200µM** | **50µ M** | **100µ M** |
| AB-77 | 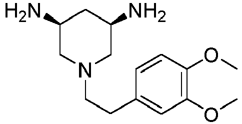 | 165 | 184 | 310 | 93 | 97 | 69 | 0.1 | 0.8 |
| AB-81 | 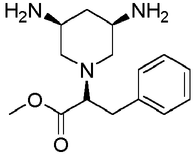 | 151 | 147 | nd | 96 | 94 | nd | 0.1 | 0.8 |
| AB-84 | 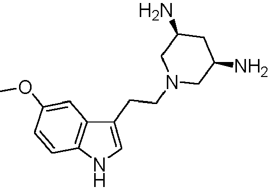 | 112 | 75 | nd | 62 | 60 | nd | 0.1 | 6.9 |
| AB-86 | 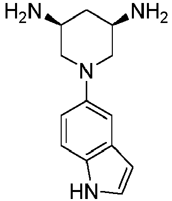 | 80 | 66 | nd | 81 | 74 | nd | 0.1 | 2.6 |
| AB-103 | 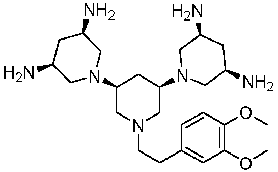 | 125 | 143 | nd | 83 | 84 | nd | 0.2 | 0.4 |
| AB-109 F3 | 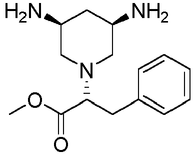 | 164 | 153 | 289 | 110 | 95 | 75 | 0.1 | 4.9 |
| AB-116 | 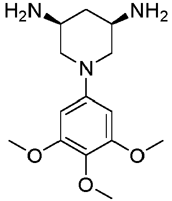 | 135 | 172 | 248 | 96 | 97 | 86 | 0 | 0.5 |
| AB-120 | 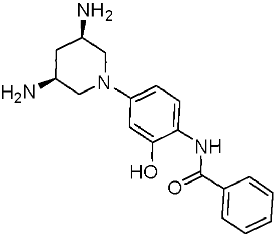 | 144 | 230 | 215 | 94 | 90 | 55 | 0.1 | 12.3 |
| AB-378 | 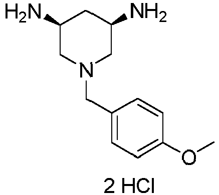 | 169 | 199 | 312 | 98 | 94 | 72 | 0.2 | 1.7 |
| AB289-2 | 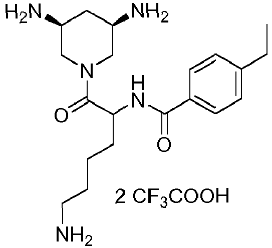 | 154 | 219 | 248 | 113 | 101 | 72 | 0.1 | 0.2 |
| AB289-1 | 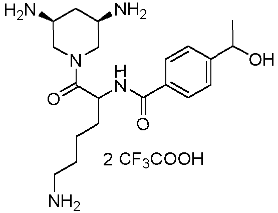 | 157 | 236 | 294 | 103 | 100 | 74 | 0 | 0.8 |
| AB287-F1 | 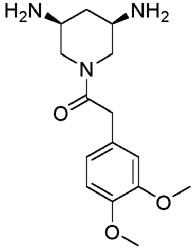 | 137 | 182 | nd | 103 | 92 | nd | 0.1 | 0.4 |
| RA 20 | 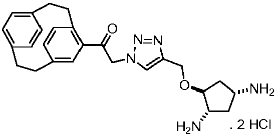 | 167 | 188 | nd | 108 | 97 | nd | 0.1 | 1.1 |
| PDA25 | 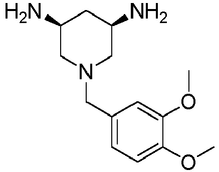 | 183 | 184 | 227 | 97 | 93 | 58 | 0 | 0.2 |
| AB541 | 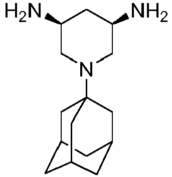 | 158 | 157 | nd | 141 | 123 | nd | 0.1 | 10.5 |
| AB542 | 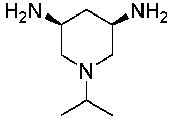 | 113 | 106 | nd | 100 | 99 | nd | 0.1 | 0.7 |
| ECO01-025-C2 | 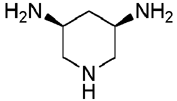 | 166 | 280 | 317 | 104 | 79 | 70 | 0.7 | nd |
| ECO01-026-C2 | 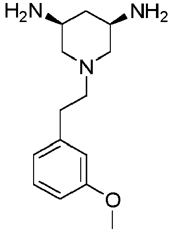 | 116 | 169 | 234 | 106 | 98 | 72 | 3.3 | nd |
| ECO01-028-C | 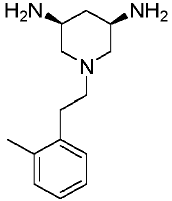 | 108 | 113 | nd | 91 | 94 | nd | 0.1 | 0 |
| ECO01-029-C | 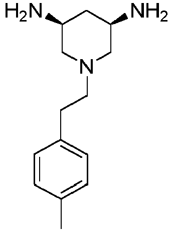 | 126 | 109 | nd | 94 | 89 | nd | 0.1 | 15.8 |
| ECO01-030-C | 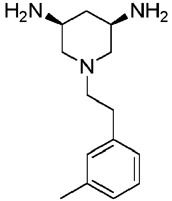 | 125 | 103 | nd | 84 | 82 | nd | 0.1 | 0 |
| ECO01-031-C | 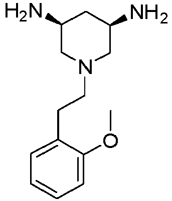 | 88 | 108 | nd | 88 | 82 | nd | 0.1 | 4.8 |
| ECO01-033-C | 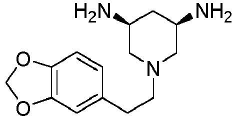 | 103 | 146 | nd | 97 | 94 | nd | 0.1 | 0.2 |
| ECO01-035-C | 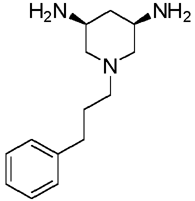 | 169 | 147 | nd | 105 | 88 | nd | 1.5 | 2.8 |
| ECO01-037-C | 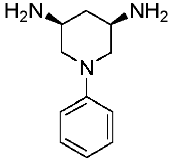 | 103 | 103 | nd | 97 | 106 | nd | 0.6 | 0.2 |
| ECO01-039-C | 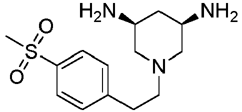 | 164 | 168 | nd | 95 | 108 | nd | 0.1 | 0.1 |
| ECO01-041-C | 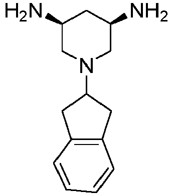 | 114 | 131 | nd | 98 | 84 | nd | 1.0 | 0.1 |
| ECO01-042-C | 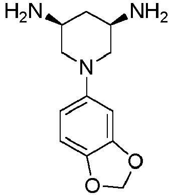 | 146 | 104 | nd | 85 | 60 | nd | 0.2 | 0.4 |
| ECO01-055-C | 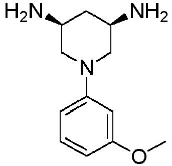 | 111 | 153 | nd | 94 | 94 | nd | 0.4 | 0.1 |
| ECO01-056-C | 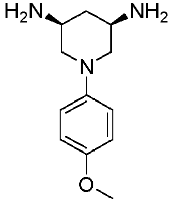 | 94 | 122 | nd | 68 | 81 | nd | 0.1 | 0.2 |
| ECO02-003-C | 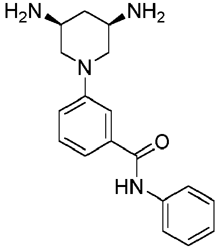 | 140 | 118 | nd | 105 | 60 | nd | 0.4 | 0.5 |
| ECO02-005-C | 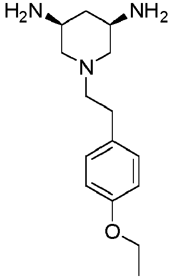 | 127 | 195 | nd | 106 | 97 | nd | 0.5 | 0.1 |
| ECO02-025-C | 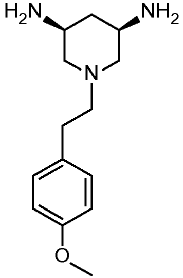 | 90 | 123 | nd | 112 | 82 | nd | 0.3 | 0.3 |
| ECO02-026-C | 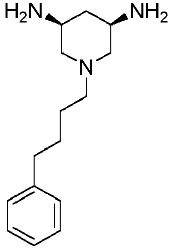 | 30 | 31 | nd | 26 | 26 | nd | 65.0 | nd |
| ECO02-027-C | 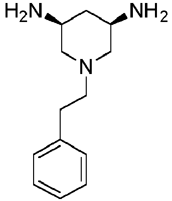 | 75 | 52 | nd | 100 | 45 | nd | 8.7 | 2.3 |
| ECO02-028-C | 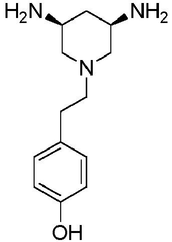 | 107 | 89 | nd | 100 | 83 | nd | 0.3 | 0.2 |
| ECO02-029-C | 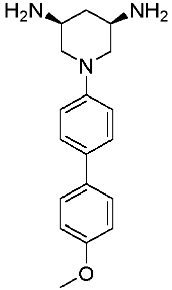 | 29 | 30 | nd | 25 | 25 | nd | 2.4 | nd |
| ECO02-030-C | 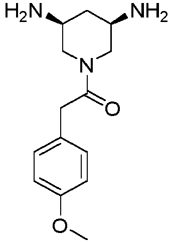 | 139 | 166 | nd | 109 | 107 | nd | 0.5 | 0.1 |
| ECO02-031-C | 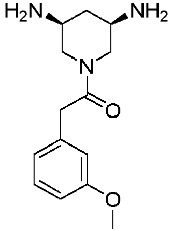 | 146 | 222 | nd | 116 | 110 | nd | 0.2 | 0.1 |
| ECO02-007-C | | 87 | 98 | nd | 97 | 98 | nd | 0.6 | nd |
| ECO02-051-C | | 124 | 200 | 397 | 95 | 96 | 77 | 1.1 | nd |
| ECO02-056-C | | 100 | 79 | nd | 67 | 38 | nd | 1.6 | nd |
| ECO02-058-C | | 110 | 159 | 194 | 105 | 88 | 87 | 1.4 | nd |
| ECO02-059-C | 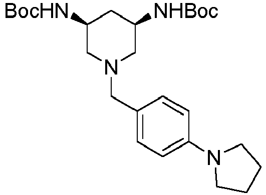 | 198 | 177 | nd | 95 | 67 | nd | 0.5 | nd |
| ECO02-060-C | 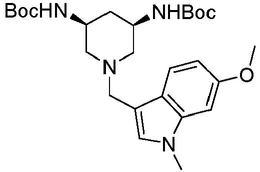 | 178 | 221 | 217 | 69 | 54 | 43 | 0.6 | nd |
| ECO02-062-C | 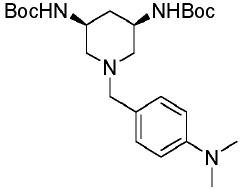 | 239 | 269 | 401 | 91 | 84 | 73 | 0.7 | nd |
| ECO02-063-C | 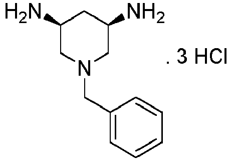 | 85 | 107 | 193 | 108 | 112 | 81 | 2.9 | nd |
| ECO02-064-C | 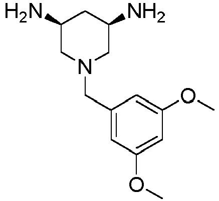 | 109 | 133 | nd | 115 | 107 | nd | 2.4 | nd |
| ECO02-068-C | 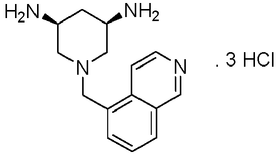 | 105 | 137 | nd | 85 | 83 | nd | 0.4 | nd |
| ECO02-069-B | 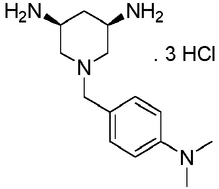 | 86 | 88 | nd | 99 | 94 | nd | 1.3 | nd |
| ECO02-072-C | 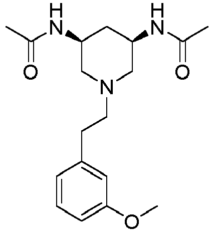 | 107 | 167 | 242 | 111 | 98 | 84 | 2.1 | nd |
| ECO02-065-C | 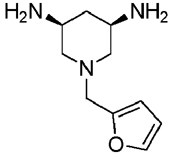 | 108 | 125 | 205 | 91 | 88 | 68 | 0.5 | nd |
| ECO02-073-C | 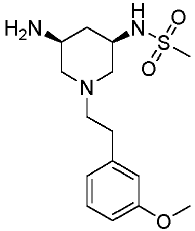 | 151 | 205 | 417 | 100 | 99 | 79 | 0.8 | nd |
| ECO02-073-B2 | 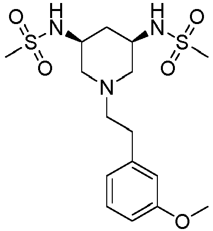 | 103 | 127 | 253 | 96 | 94 | 69 | 0.6 | nd |
| ECO02-074-C | 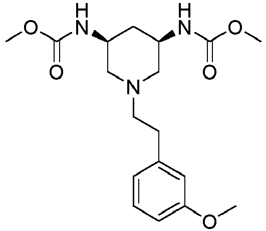 | 139 | 186 | 326 | 101 | 91 | 70 | 0.4 | nd |
| ECO02-078-C | 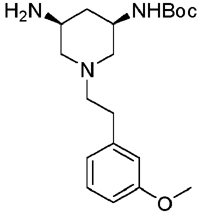 | 251 | 390 | 145 | 105 | 103 | 25 | 0.8 | nd |
| ECO02-081-C | 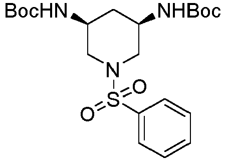 | 196 | 207 | 279 | 91 | 69 | 77 | 0.8 | nd |
| ECO03-001-B | 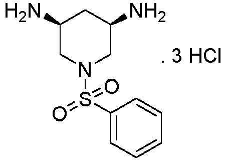 | 118 | 170 | 360 | 97 | 104 | 109 | 0.6 | nd |
| ECO03-002-C | 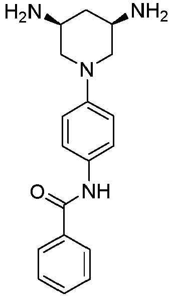 | 120 | 165 | 317 | 115 | 116 | 74 | 0.5 | nd |
| ECO03-03-C | 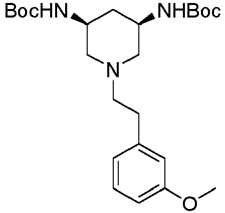 | 148 | 341 | 440 | 105 | 104 | 104 | 0.4 | nd |

### • Example 2 : CD4+ T Cells culture - VIH Patients

### Protocol:

### CD4+T cells

For experiments using primary cells obtained from HIV-infected, antiretroviral-treated, aviremic patients, total CD4+ cells are isolated by a MACS® Whole Blood MicroBead Technology (Miltenyi). Briefly, T CD4+ cells from whole blood are magnetically labeled with MACS MicroBeads and specific antibodies. Cells are separated in a MACS Column placed in a MACS Separator. The flow-through fraction can be collected as the negative fraction depleted of the labeled cells. The column is removed from the separator and the retained cells are eluted as the enriched, positively selected cell fraction. The T CD4+ cells were then cultured with a T cell activation/expansion kit (Miltenyi). The cells are activated for up to 3 days with Anti-Biotin MACSiBead Particle conjugated to monoclonal anti-biotin antibodies (anti CD2-biotin, anti CD3-CD3 and anti CD28-biotin). Expansion is achieved by adding IL-2 and fresh medium for 4 days. Then, these T CD4+ cells are considered as positive culture. A negative control was the same proportion of cells cultured without stimuli like antibodies and IL-2. The T CD4+ cells were cultured with compounds alone or in combination with antibodies or IL-2. The HIV production in cell culture media was measured by real time PCR (Cobas AmpliPrep/Cobas Taqman HIV-1 test, V2.0, Roche). The results are expressed in table 2 as a ratio of the viral load of interest/viral load of negative control or as a ratio of the viral load of interest/viral load of positive control.

### Results:

In the T CD4+ cell model, some compounds were efficient to increase the production of HIV from cells of HIV-infected, ART-treated, aviremic patients. Depending of the compounds, they were efficient alone, or in combination with antibodies or IL2.

**Table 2**

| Compounds | Number of activated patient | Antobodies+IL2(Control +) | Compound | Antobodies + Compound | Compound + IL2 | Compound | Antobodies + Compound | Compound + IL2 |
|---|---|---|---|---|---|---|---|---|
| | | | ratio of the viral load of interest/viral load of negative control | | | ratio of the viral load of interest/viral load of positive control | | |
| **AB77** | 2 | 496 | 0,3 | 258 | 1,5 | 0,0006 | 0,5202 | 0,0030 |
| | | 8,7 | 0,5 | 11,7 | 1 | 0,0575 | 1,3448 | 0,1149 |
| **AB116** | 4 | 2,4 | 1,3 | 2,3 | 0,9 | 0,5417 | 0,9583 | 0,3750 |
| | | 3,2 | 0,8 | 1,1 | 0,9 | 0,2500 | 0,3438 | 0,2813 |
| | | 35,1 | 3 | 5,5 | 27,9 | 0,0855 | 0,1567 | 0,7949 |
| | | 23 | 1,4 | 4,5 | 0,7 | 0,0609 | 0,1957 | 0,0304 |
| **AB120** | 3 | 6 | 12 | 13 | 10 | 2,0000 | 2,1667 | 1,6667 |
| | | 2 | 1 | 1 | 6 | 0,5000 | 0,5000 | 3,0000 |
| | | 3 | 3 | 5 | 3 | 1,0000 | 1,6667 | 1,0000 |
| **AB378** | 2 | 12,8 | 4,3 | 26,3 | 2,8 | 0,3359 | 2,0547 | 0,2188 |
| | | 3,1 | 1 | 2,5 | 1 | 0,3226 | 0,8065 | 0,3226 |
| **AB289-2** | 3 | 2,1 | 1 | 1 | 1 | 0,4762 | 0,4762 | 0,4762 |
| | | 12 | 0,5 | 0,5 | 0,5 | 0,0417 | 0,0417 | 0,0417 |
| | | 4,3 | 1 | 55 | 12,2 | 0,2326 | 12,7907 | 2,8372 |
| **AB289-1** | 3 | 2,2 | 0,2 | 0,2 | 0,6 | 0,0909 | 0,0909 | 0,2727 |
| | | 19,3 | 2,5 | 9,2 | 1,9 | 0,1295 | 0,4767 | 0,0984 |
| | | 2,4 | 5,1 | 3,9 | 2,3 | 2,1250 | 1,6250 | 0,9583 |
| **AB287f1** | 2 | 308 | 1 | 11,6 | 1 | 0,0032 | 0,0377 | 0,0032 |
| | | 69 | 0,6 | 1,9 | 0,5 | 0,0087 | 0,0275 | 0,0072 |
| **PDA25** | 2 | 17 | 1 | 6,8 | 1 | 0,0588 | 0,4000 | 0,0588 |
| | | 7 | 0,9 | 9,9 | 0,7 | 0,1286 | 1,4143 | 0,1000 |
| **ECO01-025** | 3 | 79 | 1 | 248 | 13 | 0,0127 | 3,1392 | 0,1646 |
| | | 38 | 1 | 1 | 1 | 0,0263 | 0,0263 | 0,0263 |
| | | 134 | 1 | 2 | 1 | 0,0075 | 0,0149 | 0,0075 |
| **Eco01-026** | 2 | 504 | 1 | 122 | 2 | 0,0020 | 0,2421 | 0,0040 |
| | | 5 | 0,5 | 1 | 0,5 | 0,1000 | 0,2000 | 0,1000 |
| **ECO02-005** | 2 | 9,4 | 3,8 | 5,3 | 1,9 | 0,4043 | 0,5638 | 0,2021 |
| | | 212 | 2,2 | 3 | 3,9 | 0,0104 | 0,0142 | 0,0184 |
| **ECO02-051** | 2 | 93 | 1 | 157 | 1 | 0,0108 | 1,6882 | 0,0108 |
| | | 4 | 1 | 1 | 0 | 0,2500 | 0,2500 | 0,0000 |
| **ECO02-062** | 2 | 6 | 1 | 3 | 1 | 0,1667 | 0,5000 | 0,1667 |
| | | 3 | 3 | 1 | 2 | 1,0000 | 0,3333 | 0,6667 |

### Example 3 :

The following compounds were assessed:

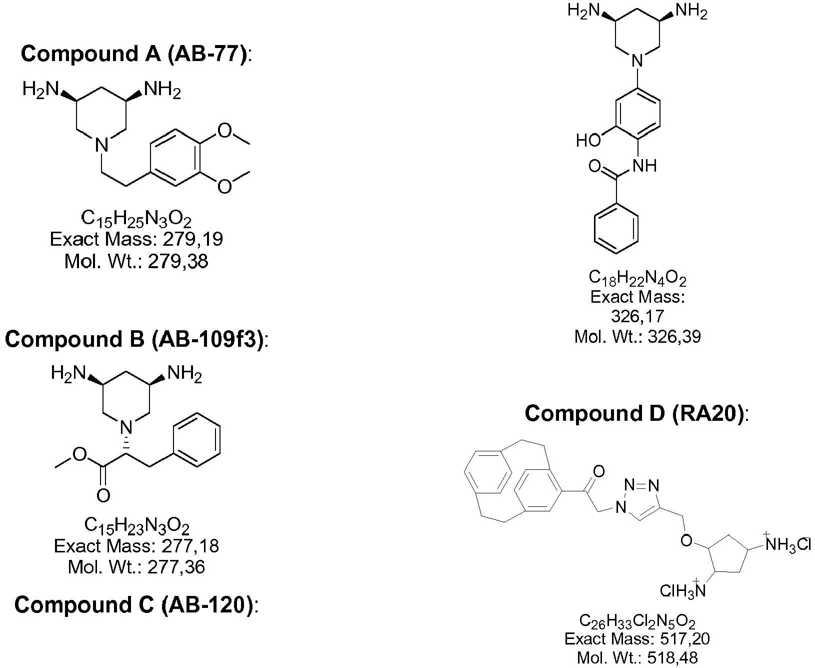

In an experiment, MT2 cells (human lymphocytic cell line) are infected with the Laï virus to a multiplicity of infection of 0.3 and incubated with compounds A, B, C or D at four different concentrations.

After 3 days of culture, real-time PCR quantification of the viral RNA of the supernatant is performed (COBAS Ampliprep/COBAS TaqMan HIV-1 assay, version 2).

The results are expressed as percentage of control (virus without any added compound) in Table 3.

**Table 3:**

| | 10 µM | 25 µM | 50 µM | 100 µM |
|---|---|---|---|---|
| **Compound A** | 130 | 60 | 50 | 73200 |
| **Compound B** | 420 | 40 | 70 | 32400 |
| **Compound C** | 21200 | 83500 | 66400 | 931700 |
| **Compound D** | 14920 | 65300 | 32070 | 280300 |

These results demonstrate that compounds A, B, C and D induce viral replication at an extremely high level in human lymphocytes.

All the above results show that the compounds of formula (I) according to the present invention are capable of increasing viral replication, are not cytotoxic, and are hence useful for eradicating HIV reservoirs.

### • Example 4

### Preparation of compounds:

### Preparation of bicyclic hydrazines

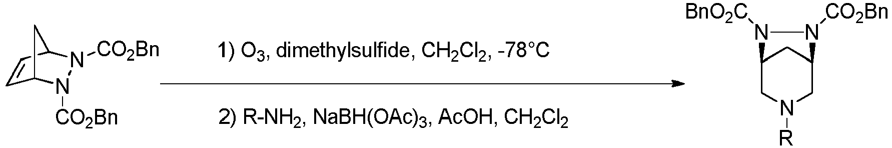

Compounds were prepared according to the Journal of Organic Chemistry 2013, 78, 12236-12242
**ECO01-003-Cdibenzyl 3-(2,3-dihydro-1*H*-inden-1-yl)-3,6,7-triazabicyclo[3.2.1]octane-6,7-dicarboxylate** : 233 mg, 43%, colorless oil ;
   ¹H NMR (500 MHz, (CD₃)₂SO, 70°C) δ 1.86 (s, 2H), 1.94-1.97 (m, 2H), 2.30 (br s, 1H), 2.51 (br s, 1H), 2.70 (bs s, 1H), 2.73-2.78 (m, 1H), 2.82-2.85 (m, 1H), 3.10 (br s, 1H), 4.20 (br s, 1H), 4.31 (br s, 1H), 4.42 (br s, 1H), 5.13-5.26 (m, 4H), 7.17-7.19 (m, 4H), 7.32-73.8 (m, 10H) ; ¹³C NMR (125 MHz, (CD₃)₂SO, 70 °C) δ 24.4, 30.3 (2C), 35.7, 53.6, 55.8, 56.4, 66.9 (2C), 67.8, 124.3 (2C), 125.1, 126.0, 127.3-128.4 (8C), 136.4 (2C), 142.2 (2C), 143.3 (2C), 156.9 (2C) ; HRMS (ESI-Orbitrap) [M+H]⁺ calcd for C₃₀H₃₂N₃O₄, 498.2387, found 498.2381. 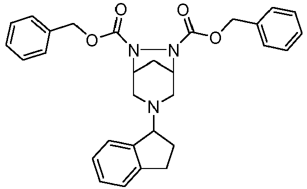
**ECO01-004-C dibenzyl 3-(2-methoxyphenethyl)-3,6,7-triazabicyclo[3.2.1]octane-6,7-dicarboxylate** : 357 mg, 63%, pale yellow oil ;
   ¹H NMR (500 MHz, (CD₃)₂SO, 70°C) δ 1.86 (m, 2H), 2.25 (br s, 2H), 2.50-2.61 (m, 4H), 3.16 (br s, 2H), 3.77 (s, 3H), 4.38 (br s, 2H), 5.14 (s, 4H), 6.86 (t, *J* = 7.3 Hz, 1H), 6.92 (d, *J* = 8.1 Hz, 1H), 7.10 (dd, *J* = 7.4 Hz, 1.3 Hz, 1H), 7.16 (td, *J* = 7.8, 0.85 Hz, 1H), 7.31-7.37 (m, 10H) ; ¹³C NMR (125 MHz, (CD₃)₂SO, 70°C) δ 26.9, 35.7, 55.4, 56.0 (2C), 56.4, 66.0, 66.8 (2C), 110.9, 120.3, 127.0-128.3 (13C), 130.8, 136.5 (2C), 156.8 (2C), 157.2 ; HRMS (ESI-Orbitrap) [M+H]⁺ calcd for C₃₀H₃₄N₃O₅, 516.24930, found 516.24811. 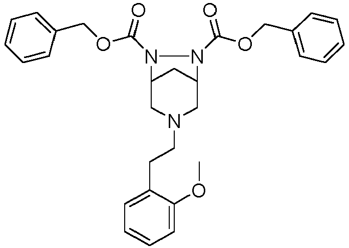
**ECO0-005-C dibenzyl 3-(2,3-dihydro-1H-inden-2-yl)-3,6,7-triazabicyclo[3.2.1]octane-6,7-dicarboxylate** : 168 mg, 30%, orange solid ;
   ¹H NMR (500 MHz, (CD₃)₂SO, 70°C) δ 1.83-1.87 (m, 2H), 2.26 (br s, 2H), 2.60 (d, *J* = 7.7 Hz, 1H), 2.61 (d, *J* = 7.7 Hz, 1H), 2.88 (q, *J* = 7.4 Hz, 1H), 2.90 (d, *J* = 7.4 Hz, 1H), 3.04 (br s, 2H), 3.23 (m, 1H), 4.38 (br s, 2H), 5.10-5.18 (m, 4H), 7.11-7.14 (m, 4H), 7.34 (br s, 10H) ; ¹³C NMR (125 MHz, (CD₃)₂SO, 70°C) δ 35.7, 36.0 (2C), 49.9, 53.8, 55.9 (2C), 64.4, 66.7 (2C), 124.0 (2C), 126.2 (2C), 127.5-128.4 (10C), 136.4 (2C), 141.3 (2C), 156.9 (2C) ; HRMS (ESI-Orbitrap) [M+H]⁺ calcd for C₃₀H₃₂N₃O₄, 498.23873, found 498.23785. 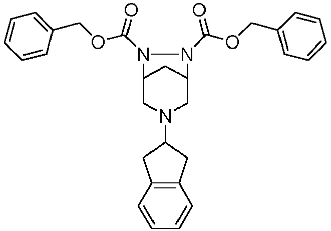
**ECO01-006-C dibenzyl 3-(4-methylphenethyl)-3,6,7-triazabicyclo[3.2.1]octane-6,7-dicarboxylate** : 317 mg, 59%, yellow oil ;
   ¹H NMR (500 MHz, (CD₃)₂SO, 70°C) δ 1.86 (br s, 2H), 2.26 (s, 5H), 2.52 (br s, 3H), 3.17 (br s, 3H), 4.39 (br s, 2H), 5.13 (br s, 4H), 7.02-7.11 (m, 4H), 7.30-7.34 (m, 10H) ; ¹³C NMR (125 MHz, (CD₃)₂SO, 70°C) δ 20.9, 32.1, 35.6, 54.8 (2C), 55.9 (2C), 57.8, 66.8 (2C), 126.4-128.6 (13C), 130.8 (2C), 134.6, 136.5, 137.0, 156.7 (2C) ; HRMS (ESI-Orbitrap) [M+H]⁺ calcd for C₃₀H₃₄N₃O₄, 500.25438, found 500.25446. 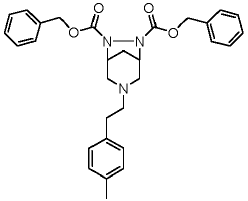
**ECO01-008-C dibenzyl 3-(3-methylphenethyl)-3,6,7-triazabicyclo[3.2.1]octane-6,7-dicarboxylate** : 230 mg, 42%, yellow oil ;
   ¹H NMR (500 MHz, (CD₃)₂SO, 70°C) δ 1.87 (br s, 2H), 2.28 (s, 5H), 2.56 (br s, 3H), 3.16 (br s, 3H), 4.40 (br s, 2H), 5.13 (br s, 4H), 6.94-6.99 (m, 3H), 7.14 (t, *J* = 7.3 Hz, 1H), 7.30-7.35 (m, 10H); ¹³C NMR (125 MHz, (CD₃)₂SO, 70°C) δ 20.9, 32.5, 35.6, 54.8 (2C), 55.9 (2C), 57.8, 66.8 (2C), 125.4, 126.4, 127.1, 127.5-128.4 (11C), 129.1, 136.4, 137.2, 140.0, 156.6 (2C) ; HRMS (ESI-Orbitrap) [M+H]⁺ calcd for C₃₀H₃₄N₃O₄, 500.25438, found 500.25406. 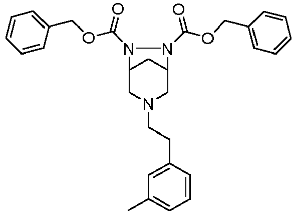
**ECO01-009-C dibenzyl 3-(1,2,3,4-tetrahydronaphthalen-1-yl)-3,6,7-triazabicyclo[3.2.1]octane-6,7-dicarboxylate** : 353 mg, 63%, white solid ;
   ¹H NMR (500 MHz, (CD₃)₂SO, 70°C) δ 1.59-1.70 (m, 2H), 1.80-1.87 (m, 4H), 2.32 (br s, 1H), 2.61-2.73 (m, 4H), 2.99 (br s, 1H), 3.67 (br s, 1H), 4.34 (br s, 1H), 4.43 (br s, 1H), 5.12-5.17 (m, 4H), 7.02-7.07 (m, 3H), 7.31-7.36 (m, 10H), 7.52 (br s, 1H) ; ¹³C NMR (125 MHz, (CD₃)₂SO, 70°C) δ 21.1, 22.1, 29.1, 35.7, 47.8, 53.6, 55.8, 56.7, 60.4, 66.9 (2C), 125.5, 126.1, 126.4, 126.5, 127.4-128.4 (11C), 136.3, 136.9, 137.9, 156.7 (2C) ; HRMS (ESI-Orbitrap) [M+H]⁺ calcd for
   C₃₁H₃₄N₃O₄, 512.25438, found 512.25427. 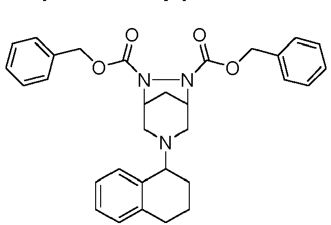
**ECO01-010-C dibenzyl 3-(2-(benzo[d][1,3]dioxol-5-yl)ethyl)-3,6,7-triazabicyclo[3.2.1]octane-6,7-dicarboxylate** : 197 mg, 34%, colorless paste ;
   ¹H NMR (500 MHz, (CD₃)₂SO, 70°C) δ 1.86 (br s, 2H), 2.24 (br s, 2H), 2.50 (br s, 2H), 3.13 (s, 4H), 4.38 (br s, 2H), 5.12 (s, 4H), 5.93 (s, 2H), 6.60 (d, *J* = 3.1 Hz, 1H), 6.72 (d, *J* = 1.4 Hz, 1H), 6.75 (d, *J* = 7.9 Hz, 1H), 7.31-7.34 (m, 10H) ; ¹³C NMR (125 MHz, (CD₃)₂SO, 70°C) δ 32.2, 35.7, 55.0, 55.9 (2C), 57.9 (2C), 66.8 (2C), 100.5, 107.9, 108.8, 121.2, 127.4-127.7 (10 C), 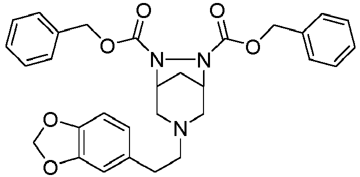 128.2, 134.1, 136.5, 145.3, 147.2, 156.6 (2C);
   HRMS (ESI-Orbitrap) [M+H]⁺ calcd for C₃₀H₃₂N₃O₆ 530.22856, found 530.22937.
**ECO01-011-C dibenzyl 3-(2,2-diphenylethyl)-3,6,7-triazabicyclo[3.2.1]octane-6,7-dicarboxylate** : 216 mg, 35%, white solid ;
   ¹H NMR (500 MHz, (CD₃)₂SO, 70°C) δ 1.80 (s, 2H), 2.22 (br s, 2H), 3.02 (d, *J* = 7.15 Hz, 2H), 3.14 (br s, 2H), 4.17 (t, *J* = 4.2 Hz, 1H), 4.32 (br s, 2H), 4.98 (br s, 4H), 7.13-7.30 (m, 20H) ; ¹³C NMR (125 MHz, (CD₃)₂SO, 70°C) δ 35.6, 48.0, 54.7 (2C), 55.7 (2C), 61.7, 66.7 (2C), 125.9 (2C), 127.3-128.2 (18C), 136.4 (2C), 144.1 (2C), 156.3 (2C) ; HRMS (ESI-Orbitrap) [M+H]⁺ calcd for C₃₅H₃₆N₃O₄, 562.27003, found 562.26874. 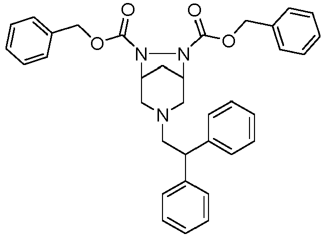
**ECO01-012-C dibenzyl 3-(3-phenylpropyl)-3,6,7-triazabicyclo[3.2.1]octane-6,7-dicarboxylate** : 285 mg, 52%, colorless oil ;
   ¹H NMR (500 MHz, (CD₃)₂SO, 70°C) δ 1.60 (m, 2H),1.85 (br m, 2H), 2.12 (br s, 2H), 2.32 (br s, 2H), 2.52 (t, *J* = 3.9 Hz, 2H), 3.07 (br s, 2H), 4.38 (br s, 2H), 5.13 (s, 4H), 7.17 (d, *J* = 6.9 Hz, 3H), 7.25 (t, *J* = 7.4 Hz, 2H), 7.29-7.36 (m, 10H) ; ¹³C NMR (125 MHz, (CD₃)₂SO, 70°C) δ 27.9, 32.4 (2C), 35.8, 55.2 (2C), 55.9 (2C), 66.9 (2C), 125.5, 127.4-128.2 (14C), 136.4 (2C), 142.1, 156.7 (2C) ; HRMS (ESI-Orbitrap) [M+H]⁺ calcd for C₃₀H₃₄N₃O₄, 500.25438 found 500.25461. 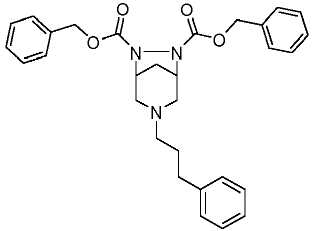
**ECO01-015-C dibenzyl 3-(3-methoxyphenethyl)-3,6,7-triazabicyclo[3.2.1]octane-6,7-dicarboxylate** : 318 mg, 57%, yellow paste ;
   ¹H NMR (500 MHz, (CD₃)₂SO, 70°C) δ 1.86 (s, 2H), 2.23 (br s, 2H), 2.56 (s, 4H), 3.15 (s, 2H), 3.74 (s, 3H), 4.38 (s, 2H), 5.13 (s, 4H), 6.75 (s, 3H), 7.18 (t, *J* = 7.4 Hz, 1H), 7.30-7.35 (m, 10H); ¹³C NMR (125 MHz, (CD₃)₂SO, 70°C) δ 32.7, 35.7, 54.9 (2C), 55.9 (2C), 57.6 (2C), 66.8 (2C), 111.5, 114.2, 120.7, 127.4-129.1 (11C), 136.5 (2C), 141.8, 156.6 (2C), 159.4 ; HRMS (ESI-Orbitrap) [M+H]⁺ calcd for C₃₀H₃₃N₃O₅, 516.24930 found 516.24731. 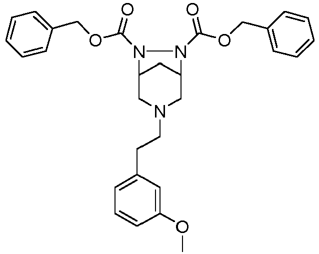
**ECO01-016-C dibenzyl 3-(2-methylphenethyl)-3,6,7-triazabicyclo[3.2.1]octane-6,7-dicarboxylate** : 251 mg, 46%, white paste ;
   ¹H NMR (500 MHz, (CD₃)₂SO, 30°C) δ 1.87 (br s, 2H), 2.11 (br s, 2H), 2.21 (s, 3H), 2.40 (br s, 2H), 3.08 (br s, 2H), 3.44 (br s, 2H), 4.27 (br s, 1H), 4.51 (br s, 1H), 5.13 (s, 4H), 7.10 (s, 4H), 7.28-7.40 (m, 10H) ; ¹³C NMR (125 MHz, (CD₃)₂SO, 30°C, two rotamers) δ 18.8 (2C), 29.6, 30.0, 35.6, 36.0, 51.8 (2C), 53.6 (2C), 55.5 (2C), 55.7 (2C), 56.4, 56.6, 66.3 (2C), 67.2 (2C), 125.8 (2C), 125.9 (2C), 126.9-129.9 (24C), 135.5 (2C), 136.2, 136.3, 136.7 (2C), 138.2 (2C), 155.2 (2C), 157.0, 157.6 ; HRMS (ESI-Orbitrap) [M+H]⁺ calcd for C₃₀H₃₄N₃O₄, 500.25438 found 500.25369. 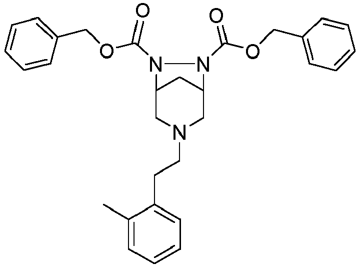
**ECO01-017-C dibenzyl 3-(3,4-dimethylphenethyl)-3,6,7-triazabicyclo[3.2.1]octane-6,7-dicarboxylate** : 291 mg, 52%, white paste ;
   ¹H NMR (500 MHz, (CD₃)₂SO, 30°C) δ 1.86 (br s, 2H), 2.05 (br s, 1H), 2.16 (br s, 6H), 2.33 (br s, 1H), 2.42 (br s, 4H), 3.03 (br s, 1H), 3.41 (br s, 1H), 4.25 (br s, 1H), 4.50 (br s, 1H), 5.03-5.15 (m, 4H), 6.85 (d, *J* = 7.6 Hz, 1H), 6.90 (s, 1H), 7.00 (br s, 1H), 7.27-7.40 (br m, 10H) ; ¹³C NMR (125 MHz, (CD₃)₂SO, 30°C, two rotamers) δ 18.9 (2C), 19.3 (2C), 31.9, 32.0, 35.6, 36.0, 51.8 (2C), 53.7 (2C), 55.7 (2C), 56.5 (2C), 58.0 (2C), 66.2 (2C), 67.2 (2C), 125.7-129.6 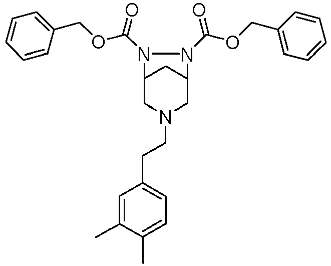 (24C), 133.3 (2C), 135.8 (2C), 136.2 (2C), 136.3 (2C), 136.8 (2C), 137.3 (2C), 155.1 (2C), 156.9, 157.6 ; HRMS (ESI-Orbitrap) [M+H]⁺ calcd for C₃₁H₃₆N₃O₄, 514.27003 found 514.26886.
**ECO01-018-C dibenzyl 3-(4-(methylsulfonyl)phenethyl)-3,6,7-triazabicyclo[3.2.1]octane-6,7-dicarboxylate** : 349 mg, 57%, colorless paste ;
   ¹H NMR (500 MHz, (CD₃)₂SO, 30°C) δ 1.86 (br s, 2H), 2.06 (br d, *J* = 11.5 Hz, 1H), 2.35 (br d, *J* = 11.5 Hz, 1H), 2.51 (s, 1H), 2.63 (br s, 3H), 3.07 (br d, *J* = 10.1 Hz, 2H), 3.16 (s, 3H), 4.26 (br s, 1H), 4.51 (br s, 1H), 5.12 (s, 4H), 7.33 (br s, 10H), 7.44 (d, *J* = 7.9 Hz, 2H), 7.82 (d, *J* = 8.3 Hz, 2H) ; ¹³C NMR (125 MHz, (CD₃)₂SO, 30°C, two rotamers) δ 31.9, 32.0, 35.5, 35.9, 43.6 (2C), 51.8 (2C), 53.8 (2C), 55.6 (2C), 56.5 (2C), 57.1 (2C), 66.2 (2C), 67.2 (2C), 126.9-129.5 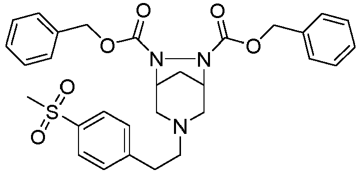 (26C), 136.2 (2C), 136.3 (2C), 136.7 (2C), 138.4 (2C), 146.6 (2C), 155.1 (2C), 156.8, 157.7 ; HRMS (ESI-Orbitrap) [M+H]⁺ calcd for C₃₀H₃₄N₃O₆S, 564.21628 found 564.21625.
**ECO01-019-C dibenzyl 3-(2-phenoxyphenethyl)-3,6,7-triazabicyclo[3.2.1]octane-6,7-dicarboxylate** : 291 mg, 48%, yellow oil ;
   ¹H NMR (500 MHz, (CD₃)₂SO, 60°C) δ 1.81 (br s, 2H), 2.19 (br s, 2H), 2.57 (br s, 2H), 2.60 (br s, 2H), 3.00 (br s, 2H), 4.33 (br s, 2H), 5.08 (br s, 4H), 6.85 (d, *J* = 8.0 Hz, 1H), 6.90 (d, *J* = 8.0 Hz, 2H), 7.05-7.11 (m, 2H), 7.19 (t, *J* = 7.55 Hz, 2H), 7.20-7.28 (m, 12 H) ; ¹³C NMR (125 MHz, (CD₃)₂SO, 60°C) δ 26.7, 35.6, 51.9, 55.0, 55.8 (2C), 56.4, 66.8 (2C), 117.3, 119.4 (2C), 122.6 (2C), 124.1, 126.6-127.9 (10C), 128.2, 129.9, 130.8, 131.5, 136.4 (2C), 153.9, 156.9 (2C), 157.5 ; HRMS (ESI-Orbitrap) [M+H]⁺ calcd for C₃₅H₃₆N₃O₅, 578.26495 found 578.26416. 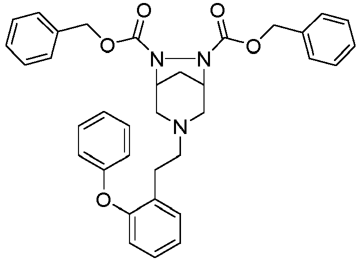
**ECO01-020-C dibenzyl 3-phenyl-3,6,7-triazabicyclo[3.2.1]octane-6,7-dicarboxylate** : 205 mg, 41%, white solid ;
   ¹H NMR (500 MHz, (CD₃)₂SO, 60°C) δ 1.99-2.15 (m, 2H), 2.90 (br s, 2H), 3.81 (br s, 2H), 4.62 (br s, 2H), 5.15 (br s, 4H), 6.74-6.77 (m, 3H), 7.17-7.32 (m, 12 H) ; ¹³C NMR (125 MHz, (CD₃)₂SO, 60°C) δ 34.0, 50.5 (2C), 55.3 (2C), 67.0 (2C), 113.4 (2C), 117.9, 126.6-128.9 (12C), 136.2 (2C), 149.7, 156.3 (2C) ; HRMS (ESI-Orbitrap) [M+H]⁺ calcd for C₂₇H₂₈N₃O₄, 458.2074 found 458.2070. 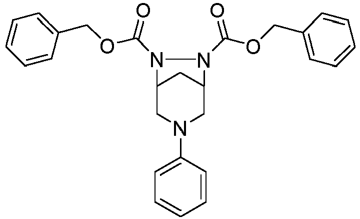
**ECO01-021-C dibenzyl 3-(4-(trifluoromethyl)phenyl)-3,6,7-triazabicyclo[3.2.1]octane-6,7-dicarboxylate** : 168 mg, 30 %, yellow oil;
   ¹H NMR (500 MHz, (CD₃)₂SO, 60°C) δ 2.03-2.06 (m, 1H), 2.14-2.16 (m, 1H), 3.03 (br s, 2H), 3.89 (br s, 2H), 4.66 (br s, 2H), 5.15 (br m, 4H), 6.84 (br s, 2H), 7.17-7.32 (br m, 10H), 7.46 (d, *J* = 8.7 Hz, 2H) ; ¹³C NMR (125 MHz, (CD₃)₂SO, 60°C) δ 33.5, 47.9, 50.3 (2C), 55.0, 67.0 (2C), 112.4 (2C), 117.3 (*J* = 32.0 Hz), 125.5 (*J* = 270.0 Hz), 126.0 (*J* = 3.6 Hz, 2C), 126.2-128.5 (10C), 136.2 (2C), 152.2, 156.0 (2C) ; HRMS (ESI-Orbitrap) [M+H]⁺ calcd for C₂₈H₂₇F₃N₃O₄, 526.1948 found 526.1937. 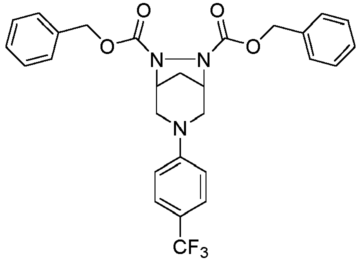
**ECO01-032-C dibenzyl 3-(benzo[*d*][1,3]dioxol-5-yl)-3,6,7-triazabicyclo[3.2.1]octane-6,7-dicarboxylate**: 254 mg, 47%, pale brown solid ;
   ¹H NMR (500 MHz, (CD₃)₂SO, 60°C) δ 1.98-2.05 (m, 2H), 2.83 (br s, 2H), 3.68 (br s, 2H), 4.57 (br s, 2H), 5.16 (br m, 4H), 5.90 (s, 2H), 6.16 (br s, 1H), 6.43 (br s, 1H), 6.73 (d, *J* = 8.5 Hz, 1H), 7.32 (br s, 10 H) ; ¹³C NMR (125 MHz, (CD₃)₂SO, 60°C) δ 34.2, 49.1, 51.8, 55.4 (2C), 67.0 (2C), 97.1, 100.4, 106.2, 108.2, 127.5-128.3 (11C), 136.3, 139.9, 145.7, 148.0, 156.4 (2C) ; HRMS (ESI-Orbitrap) [M+H]⁺ calcd for C₂₈H₂₇N₃O₆, 502.1973 found 502.1991. 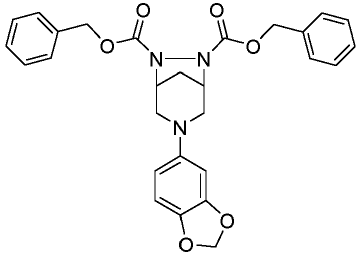
**ECO01-044-C dibenzyl 3-(3-methoxyphenyl)-3,6,7-triazabicyclo[3.2.1]octane-6,7-dicarboxylate** : 312 mg, 59%, orange oil ;
   ¹H NMR (500 MHz, (CD₃)₂SO, 60°C) δ 2.02-2.09 (m, 2H), 2.90 (br s, 2H), 3.72 (s, 3H), 3.81 (br s, 2H), 4.62 (br s, 2H), 5.17 (br s, 4H), 6.30-6.38 (m, 3H), 7.10 (t, *J* = 8.1 Hz, 1H), 7.32 (br s, 10H) ; ¹³C NMR (125 MHz, (CD₃)₂SO, 60°C) δ 33.9, 48.0, 50.7, 54.7, 55.2 (2C), 67.0 (2C), 99.7, 103.5, 106.2, 127.5-128.3 (11C), 129.6, 136.2, 151.1, 156.2 (2C), 160.4 ; HRMS (ESI-Orbitrap) [M+H]⁺ calcd for C₂₈H₃₀N₃O₅, 488.2180 found 488.2184. 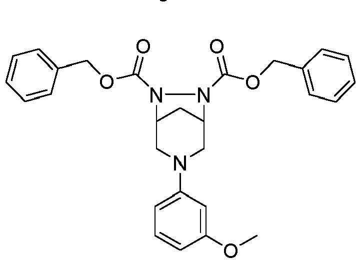
**ECO01-045-C dibenzyl 3-(4-methoxyphenyl)-3,6,7-triazabicyclo[3.2.1]octane-6,7-dicarboxylate** : 335 mg, 63%, brown solid ;
   ¹H NMR (500 MHz, (CD₃)₂SO, 60 °C, δ) 2.00-2.04 (br m, 2H), 2.80 (br s, 2H), 3.56-3.78 (m, 5H), 4.58 (br s, 2H), 5.17 (s, 4H), 6.70-6.82 (br m, 4H), 7.33 (br s, 10H) ; ¹³C NMR (125 MHz, (CD₃)₂SO, 60 °C, δ) 34.4, 49.2, 51.7, 55.4 (3C), 67.0 (2C), 114.5 (2C), 115.5 (2C), 127.6-128.3 (10C), 136.3 (2C), 144.1, 152.5, 156.5 (2C); HRMS (ESI-Orbitrap) [M+H]⁺ calcd for C₂₈H₃₀N₃O₅, 488.2180 found 488.2196. 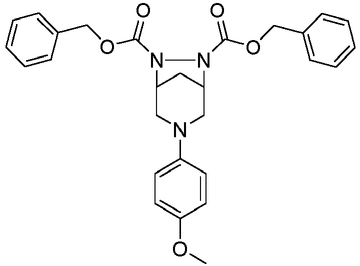
**ECO01-046-C dibenzyl 3-(2-methoxyphenyl)-3,6,7-triazabicyclo[3.2.1]octane-6,7-dicarboxylate** : 335 mg, 63%, brown oil ;
   ¹H NMR (500 MHz, (CD₃)₂SO, 57°C) δ 1.98-2.04 (m, 2H), 2.92 (br s, 2H), 3.56-3.69 (m, 2H), 3.70 (s, 3H), 4.51 (br s, 2H), 5.17 (br s, 4H), 6.86-6.92 (m, 3H), 6.98 (t, *J* = 8.6 Hz, 1H), 7.29-7.32 (m, 10H) ; ¹³C NMR (125 MHz, (CD₃)₂SO, 57°C) δ 35.3, 50.6, 52.9, 55.8 (3C), 66.9 (2C), 112.9, 120.4, 121.0, 123.0, 127.2-128.2 (10C), 136.4 (2C), 139.9, 153.0, 156.4 (2C) ; HRMS (ESI-Orbitrap) [M+H]⁺ calcd for C₂₈H₃₀N₃O₅, 488.2180 found 488.2189. 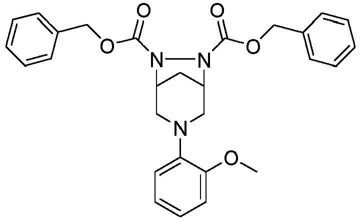
**ECO01-048-C dibenzyl 3-(naphthalen-2-ylmethyl)-3,6,7-triazabicyclo[3.2.1]octane-6,7-dicarboxylate** : 430 mg, 76%, white solid ;
   ¹H NMR (500 MHz, (CD₃)₂SO, 57°C) δ 1.86 (br s, 2H), 2.23 (br s, 2H), 3.01 (br s, 2H), 3.92 (br s, 2H), 4.32 (br s, 2H), 5.01 (s, 4H), 7.27-7.50 (br m, 14H), 7.81 (d, *J* = 8.4 Hz, 1H), 7.88 (d, *J* = 8.9 Hz, 1H), 8.14 (br s, 1H) ; ¹³C NMR (125 MHz, (CD₃)₂SO, 57°C) δ 35.8, 50.6, 52.8 (2C), 55.6 (2C), 58.6, 66.7 (2C), 124.8, 125.5, 125.9, 127.2-128.2 (14C), 131.5, 133.4, 133.7, 136.1, 156.3 (2C) ; HRMS (ESI-Orbitrap) [M+H]⁺ calcd for C₃₂H₃₂N₃O₄, 522.2387 found 522.2397. 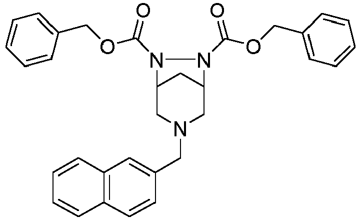
**ECO01-049-C dibenzyl 3-(3-(phenylcarbamoyl)phenyl)-3,6,7-triazabicyclo[3.2.1]octane-6,7-dicarboxylate** : 368 mg, 59%, white solid ;
   ¹H NMR (500 MHz, (CD₃)₂SO, 57°C) δ 2.05-2.13 (m, 2H), 2.99 (br s, 2H), 3.96-4.30 (br s, 2H), 4.66 (br s, 2H), 5.17 (br s, 4H), 6.94-7.36 (br m, 17H), 7.78 (d, *J* = 7.9 Hz, 2H), 10.04 (s, 1H, NH) ; ¹³C NMR (125 MHz, (CD₃)₂SO, 57°C) δ 33.9, 48.1, 50.4, 55.1 (2C), 67.1 (2C), 112.2, 116.2, 117.0, 120.3 (2C), 123.6, 127.6-128.9 (14C), 135.8, 136.2, 139.2, 149.7, 156.1 (2C), 165.9 ; HRMS (ESI-Orbitrap) [M+H]⁺ calcd for C₃₄H₃₃N₄O₅, 577.2445 found 577.2462. 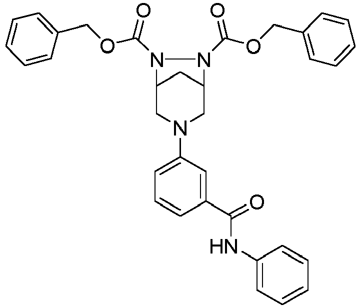
**ECO01-052-C dibenzyl 3-(3-(phenylcarbamoyl)phenyl)-3,6,7-triazabicyclo[3.2.1]octane-6,7-dicarboxylate** : 186 mg, 58 %, white solid ;
   ¹H NMR (500 MHz, (CD₃)₂SO, 57°C) δ 1.30 (t, *J* = 7.0 Hz, 3H), 1.86 (br s, 2H), 2.20 (br s, 2H), 3.19 (s, 6H), 3.98 (q, *J* = 6.9 Hz, 2H), 4.36 (br s, 2H), 5.12 (s, 4H), 6.80 (d, *J* = 8.5 Hz, 2H), 7.05 (d, *J* = 8.5 Hz, 2H), 7.30-7.35 (m, 10H) ; ¹³C NMR (125 MHz, (CD₃)₂SO, 57°C) δ 14.6, 31.6, 35.7, 54.8, 55.8 (2C), 56.4, 58.0, 62.9, 66.8 (2C), 114.4 (2C), 127.6-128.3 (10C), 129.5 (2C), 132.0 (2C), 136.5 (2C), 156.7 (2C) ; HRMS (ESI-Orbitrap) [M+H]⁺ calcd for C₃₁H₃₆N₃O₅, 530.2649 found 530.2643. 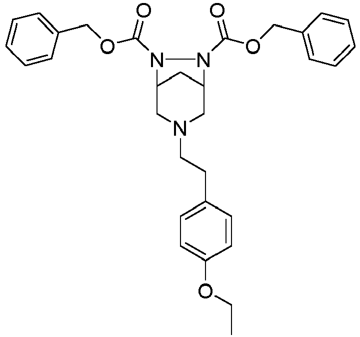
**ECO01-053-C dibenzyl 3-(4-(azepane-1-carbonyl)phenyl)-3,6,7-triazabicyclo[3.2.1]octane-6,7-dicarboxylate** : 301 mg, 56%, white solid ;
   ¹H NMR (500 MHz, (CD₃)₂SO, 57°C) δ 1.56 (br s, 4H), 1.66 (br s, 4H), 2.04 (br s, 1H), 2.10 (br s, 1H), 2.96 (br s, 1H), 3.48 (br s, 5H), 3.74-4.33 (br m, 2H), 4.68 (br s, 2H), 5.17 (br s, 4H), 6.60 (br s, 1H), 6.74 (br s, 1H), 7.10-7.34 (br m, 12H) ; ¹³C NMR (125 MHz, (CD₃)₂SO, 57°C) δ 26.6, 28.0, 32.3, 34.1, 47.6, 50.4, 55.1 (2C), 60.2, 61.3, 62.5, 66.9 (2C), 112.2, 112.9, 124.2, 126.3, 126.8-128.4 (11 C), 136.3 (2C), 150.1, 156.4 (2C), 170.5 ; HRMS (ESI-Orbitrap)[M+H]⁺ calcd for C₃₄H₃₉N₄O₅, 583.2915 found 583.2913. 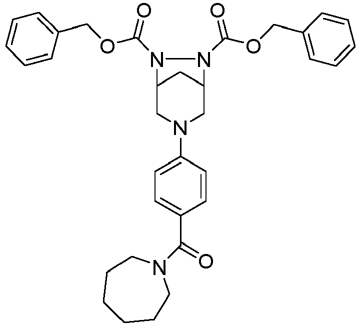
**ECO01-054-C dibenzyl 3-(4-(p-tolyloxy)phenyl)-3,6,7-triazabicyclo[3.2.1]octane-6,7-dicarboxylate** : 403 mg, 66%, white solid ;
   ¹H NMR (500 MHz, (CD₃)₂SO, 57°C) δ 2.05 (br s, 2H), 2.30 (br s, 3H), 2.88 (br s, 2H), 3.77 (br s, 2H), 4.61 (br s, 2H), 5.20 (br s, 4H), 6.85 (br s, 6H), 7.10-7.34 (br m, 12H) ; ¹³C NMR (125 MHz, (CD₃)₂SO, 57°C) δ 20.1, 34.1, 48.4, 48.9, 51.2 (2C), 55.0 (2C), 114.9 (2C), 117.3 (2C), 120.0 (2C), 127.6-128.3 (11C), 130.0, 131.4, 136.3 (2C), 146.2, 148.5, 156.0 (3C) ; HRMS (ESI-Orbitrap) [M+H]⁺ calcd for C₃₄H₃₄N₃O₅, 562.2493 found 564.2488. 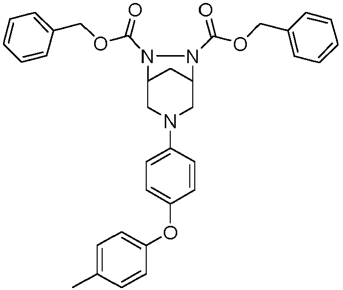
**ECO02-008-C dibenzyl 3-(4-bromophenyl)-3,6,7-triazabicyclo[3.2.1]octane-6,7-dicarboxylate** : 514 mg, 44%, white solid ;
   ¹H NMR (500 MHz, (CD₃)₂SO, 60°C) δ 1.99-2.10 (m, 2H), 2.90 (br s, 2H), 3.78 (br s, 2H), 4.62 (br s, 2H), 5.15 (br s, 4H), 6.67 (br s, 2H), 7.30 (br s, 12H) ; ¹³C NMR (125 MHz, (CD₃)₂SO, 60°C) δ 33.8, 48.6, 50.3, 55.2 (2C), 59.7 (2C), 109.0, 115.2 (2C), 126.4-127.8 (10C), 128.3 (2C), 131.3, 136.2, 148.9, 156.2 (2C); HRMS (ESI-Orbitrap) [M+H]⁺ calcd for C₂₇H₂₇N₃O₄Br, 536.1190 found 536.1177. 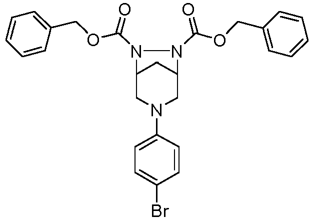
**ECO02-013-C dibenzyl 3-(4-bromophenyl)-3,6,7-triazabicyclo[3.2.1]octane-6,7-dicarboxylate** : 334 mg, 63%, colorless oil;
   ¹H NMR (500 MHz, (CD₃)₂SO, 60°C) δ 1.84 (brs, 2H), 2.21 (br s, 2H), 2.57 (br s, 4H), 3.09 (br s, 2H), 4.39 (br s, 2H), 5.14 (br s, 4H), 7.01-7.36 (m, 15H) ; ¹³C NMR (125 MHz, (CD₃)₂SO, 60°C) δ 32.6, 35.7, 52.2, 54.9, 55.9 (2C), 57.8, 66.8 (2C), 125.5 (2C), 127.0-128.0 (7C), 128.2 (2C), 128.3 (2C), 128.4 (2C), 136.4, 140.3 (2C), 156.8 (2C) ; HRMS (ESI-Orbitrap) [M+H]⁺ calcd for C₂₉H₃₂N₃O₄, 486.2387 found 486.2390. 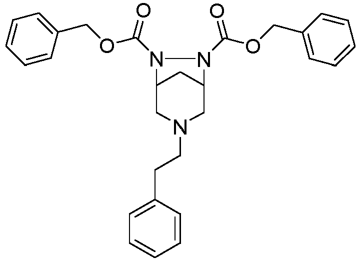
**ECO02-014-C dibenzyl 3-(4-hydroxyphenethyl)-3,6,7-triazabicyclo[3.2.1]octane-6,7-dicarboxylate:** 161 mg, 29%, colorless oil;
   ¹H NMR (500 MHz, (CD₃)₂SO, 60°C) δ 1.83-1.85 (br m, 2H), 2.20 (br s, 2H), 2.47-2.55 (br m, 4H), 3.07 (br s, 2H), 4.37 (br s, 2H), 5.14 (br s, 4H), 6.67 (d, *J* = 8.4 Hz, 2H), 6.94 (d, *J* = 8.4 Hz, 2H), 7.34 (br s, 10H), 8.95 (s, 1H, OH) ; ¹³C NMR (125 MHz, (CD₃)₂SO, 60°C) δ 31.7, 35.7, 52.2, 53.6, 55.9 (2C), 58.2, 66.8 (2C), 115.1 (2C), 127.5-128.3 (11C), 129.2 (2C), 130.3, 136.4, 155.3, 156.8 (2C) ; HRMS (ESI-Orbitrap) [M+H]⁺ calcd for C₂₉H₃₂N₃O₅, 502.2336 found 502.2335. 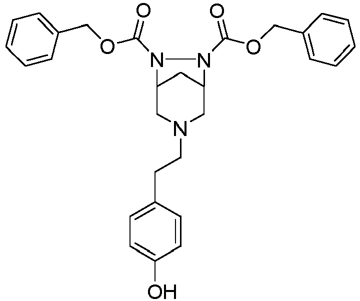
**ECO02-019-C dibenzyl 3-(4-methoxyphenethyl)-3,6,7-triazabicyclo[3.2.1]octane-6,7-dicarboxylate** : 435 mg, 76%, colorless oil;
   ¹H NMR (500 MHz, (CD₃)₂SO, 60°C) δ 1.82-1.90 (m, 2H), 2.12 (br s, 2H), 2.50 (s, 4H), 3.10 (br s, 2H), 3.72 (s, 3H), 4.37 (br s, 2H), 5.13 (s, 4H), 6.82 (d, *J* = 8.4 Hz, 2H), 7.07 (d, *J* = 8.4 Hz, 2H), 7.28-7.38 (m, 10H) ; ¹³C NMR (125 MHz, (CD₃)₂SO, 60°C) δ 31.6, 35.7, 52.5, 54.7, 55.0 (2C), 55.8, 66.8 (2C), 113.8 (2C), 126.6-128.3 (9C), 128.3 (2C), 129.3 (2C), 132.5, 136.4, 156.7 (2C), 157.9 ; HRMS (ESI-Orbitrap) [M+H]⁺ calcd for C₃₀H₃₄N₃O₅, 516.2493 found 516.2489. 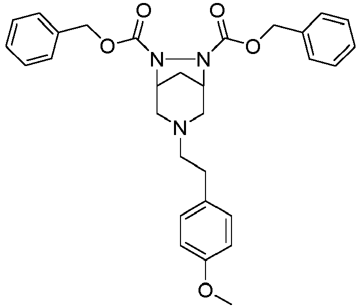
**ECO02-036-C dibenzyl 3-(3-hydroxyphenethyl)-3,6,7-triazabicyclo[3.2.1]octane-6,7-dicarboxylate** : 229 mg, 37%, white solid ;
   ¹H NMR (500 MHz, (CD₃)₂SO, 60 °C, δ) 1.86 (br s, 2H), 2.22 (br s, 2H), 2.51 (br s, 4H), 3.20 (br s, 2H), 4.37 (br s, 2H), 5.14 (br s, 4H), 6.58-6.60 (m, 3H), 7.04 (t, *J*= 7.9 Hz, 1H), 7.35 (br s, 10H), 9.05 (br s, 1H, OH); ¹³C NMR (125 MHz, (CD₃)₂SO, 60 °C, δ) 32.6, 35.7, 52.2, 55.0, 55.8 (2C), 57.8, 66.8 (2C), 112.9, 115.4, 119.1, 127.5-129.0 (11C), 136.5 (2C), 141.5, 157.0 (2C), 157.3 ; HRMS (ESI-Orbitrap) [M+H]⁺ calcd for C₂₉H₃₂N₃O₅, 502.2336 found 502.2352. 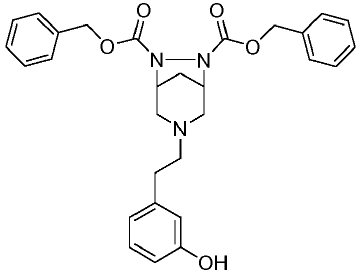
**ECO02-020-C dibenzyl 3-(4-phenylbutyl)-3,6,7-triazabicyclo[3.2.1]octane-6,7-dicarboxylate** : 473 mg, 85%, colorless oil;
   ¹H NMR (500 MHz, (CD₃)₂SO, 60°C) δ 1.33 (m 2H), 1.54 (m, 2H), 1.80-1.84 (m, 2H), 2.20 (br s, 2H), 2.31 (br s, 2H), 2.51-2.56 (m, 2H), 2.99 (br s, 2H), 4.35 (br s, 2H), 5.12 (br s, 4H), 5.71 (s, 4H), 7.14-7.27 (m, 15H) ; ¹³C NMR (125 MHz, (CD₃)₂SO, 60°C) δ 25.7, 28.1, 35.0, 35.8, 52.5, 54.7, 55.8 (3C), 66.8 (2C), 125.3 (2C), 125.5-128.2 (14C), 136.4, 142.4, 156.8 (2C) ; HRMS (ESI-Orbitrap) [M+H]⁺ calcd for C₃₁H₃₅N₃O₄, 514.2700 found 514.2700. 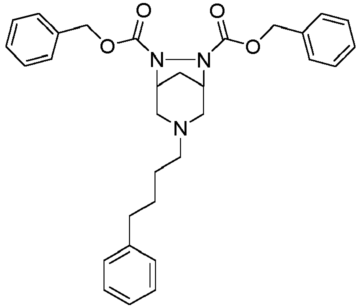
**ECO02-047-C dibenzyl 3-(3-fluorophenethyl)-3,6,7-triazabicyclo[3.2.1]octane-6,7-dicarboxylate** : 360 mg, 66%, colorless oil ;
   ¹H NMR (500 MHz, (CD₃)₂SO, 57°C) δ 1.86 (br s, 2H), 2.22 (br s, 2H), 2.58 (br s, 4H), 3.08 (br s, 2H), 4.37 (br s, 2H), 5.12 (br s, 4H), 6.97-6.99 (m, 3H), 7.25-7.40 (m, 11H) ; ¹³C NMR (125 MHz, (CD₃)₂SO, 57°C) δ 32.1, 35.7, 52.2, 54.1, 55.8 (2C), 57.3, 66.8 (2C), 112.4 (*J* = 20.8 Hz), 115.1 (*J* = 20.8 Hz), 124.5 (*J* = 2.7 Hz), 126.4-129.9 (11C), 136.5 (2C), 143.2 (*J* = 7.5 Hz), 156.7 (2C), 162.2 (*J* = 243.3 Hz) ; HRMS (ESI-Orbitrap) [M+H]⁺ calcd for C₂₉H₃₁N₃O₄F, 504.2293 found 504.2289. 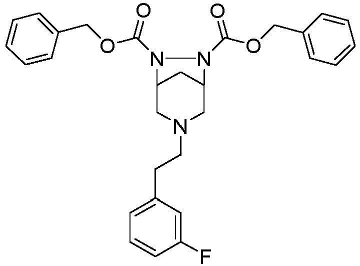
**ECO02-048-C dibenzyl 3-(2-cyclohexylethyl)-3,6,7-triazabicyclo[3.2.1]octane-6,7-dicarboxylate** : 246 mg, 46%, colorless oil ;
   ¹H NMR (500 MHz, (CD₃)₂SO, 57°C) δ 0.83-0.85 (m, 2H), 1.15-1.21 (m, 6H), 1.60-1.62 (m, 5H), 1.83 (br s, 2H), 2.10 (br s, 2H), 2.31 (br s, 2H), 3.02 (br s, 2H), 4.35 (br s, 2H), 5.08-5.18 (m, 4H), 7.34 (br s, 10H) ; ¹³C NMR (125 MHz, (CD₃)₂SO, 57°C) δ 25.6 (2C), 26.1, 32.8 (2C), 33.6, 34.7, 35.8, 52.3, 53.8, 54.1, 55.8 (2C), 66.8 (2C), 126.4-128.3 (10C), 136.4 (2C), 156.7 (2C) ; HRMS (ESI-Orbitrap) [M+H]⁺ calcd for C₂₉H₃₈N₃O₄, 492.2857 found 492.2849. 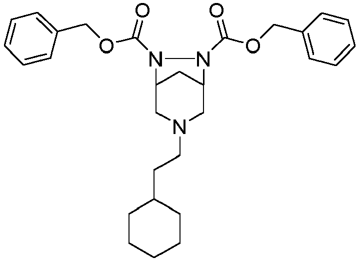
**ECO02-076-C dibenzyl 3-(4-benzamidophenyl)-3,6,7-triazabicyclo[3.2.1]octane-6,7-dicarboxylate** : 436 mg, 69%, pale yellow solid ;
   ¹H NMR (500 MHz, (CD₃)₂SO, 57°C) δ 2.01-2.07 (m, 2H), 2.88 (br s, 2H), 3.81 (br s, 2H), 4.62 (br s, 2H), 4.90-5.19 (m, 4H), 6.75 (br s, 2H), 7.34 (br s, 10H), 7.52-7.62 (m, 5H), 7.98 (d, *J =* 7.0 Hz, 2H), 9.93 (s, 1H); ¹³C NMR (125 MHz, (CD₃)₂SO, 57°C) δ 34.1, 48.5, 50.9, 55.3 (2C), 67.1 (2C), 113.5 (2C), 121.8 (2C), 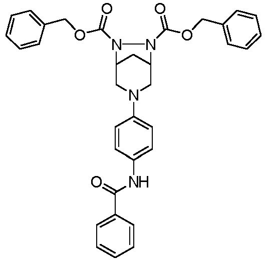 127.5-131.1 (16C), 135.3, 136.3 (2C), 146.3, 156.2 (2C), 164.9 ; HRMS (ESI-Orbitrap) [M+H]⁺ calcd for C₃₄H₃₃N₄O₅, 577.2445 found 577.2435.

### Hydrogenolysis of bicyclic hydrazines using flow hydrogenation

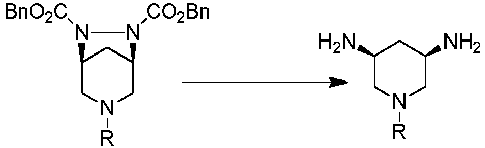

### ECO01-026-C

### Hydrogenation on H-Cube®:

**ECO01-015-C** (100 mg, 0.194 mmol) was solubilised in MeOH (77 mL), c = 0.0025 M. 3 Runs at 35°C, 30 Bars at 1 mL/min. After evaporation, purification Chromatography on silica gel (CH₂Cl₂ to CH₂Cl₂/MeOH/NH₄OH 90/9/1) afforded **ECO01-026-C2** (13 mg, 27%), colorless paste.
**ECO01-026-C2 1-(3-methoxyphenethyl)piperidine-3,5-diamine** : 13 mg, 27%, yellow paste ;
¹H NMR (500 MHz, CD₃OD, 27 °C) δ 0.87 (q, *J* = 11.7 Hz, 1H), 1.72 (t, *J* = 10.4 Hz, 2H), 2.00-2.04 (m, 1H), 2.53-2.57 (m, 2H), 2.67-2.70 (m, 2H), 2.79-2.82 (m, 2H), 2.92 (dd, *J* = 10.4 Hz, *J* = 3.8 Hz, 2H), 3.67 (s, 3H), 6.64-6.69 (m, 3H), 7.07 (t, *J* = 5.33 Hz, 1H); ¹³C NMR (125 MHz, CD₃OD, 27 °C) δ 34.1, 42.5, 48.1 (2C), 55.6, 61.1, 61.3 (2C), 112.5, 115.5, 122.0, 130.4, 142.9, 161.3 ; HRMS (ESI-Orbitrap) [M+H]⁺ calcd for C₁₄H₂₄N₃O, 250.1914 found 250.1910. 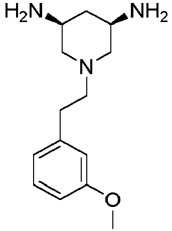
**ECO01-025-C2 piperidine-3,5-diamine** : 3 mg, yellow oil ¹H NMR (500 MHz, CD₃OD, 27 °C) δ 1.15 (q, *J* = 12.0 Hz, 1H), 2.10-2.13 (m, 1H), 2.13 (t, *J* = 10.4 Hz, 2H), 2.82-2.87 (m, 2H), 2.98 (ddd, *J* = 12.3 Hz, *J* = 4.09 Hz, *J* = 1.57 Hz, 2H) ; ¹³C NMR (125 MHz, CD₃OD, 27 °C) δ 39.8 (2C), 49.2, 51.7 (2C) ; HRMS (ESI-Orbitrap) [M+H]⁺ calcd for C₅H₁₄N₃, 116.1182 found 116.1183. 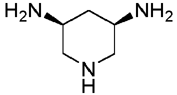
**ECO01-028-C 1-(2-methylphenethyl)piperidine-3,5-diamine** : 13 mg, 28%, white paste ;
   ¹H NMR (500 MHz, CD₃OD, 27 °C) δ 0.99 (q, *J* = 11.5 Hz, 1H), 1.82 (t, *J* = 10.4 Hz, 2H), 2.17 (d, *J* = 10.8 Hz, 1H), 2.34 (s, 3H), 2.57-2.62 (m, 2H), 2.84-2.87 (m, 2H), 2.93-2.95 (m, 2H), 3.08-3.10 (m, 2H), 7.10-7.15 (m, 4H) ; ¹³C NMR (125 MHz, CD₃OD, 27 °C) δ 19.3, 31.2, 42.8, 48.1 (2C), 59.9, 61.5 (2C), 127.2, 127.4, 130.3, 131.3, 137.0, 139.1 ; HRMS (ESI-Orbitrap) [M+H]⁺ calcd for C₁₄H₂₄N₃, 234.1965 found 234.1961. 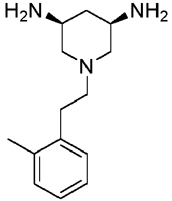
**ECO01-029-C 1-(4-methylphenethyl)piperidine-3,5-diamine** : 17 mg, 36%, white paste ;
   ¹H NMR (500 MHz, CD₃OD, 27 °C) δ 1.16 (q, *J*= 10.8 Hz, 1H), 1.99 (t, *J* = 10.2 Hz, 2H), 2.17 (d, *J* = 12.8 Hz, 1H), 2.34 (s, 3H), 2.70 (t, *J* = 8.7 Hz, 2H), 2.81 (t, *J* = 8.8 Hz, 2H), 3.05-3.07 (m, 4H), 7.10-7.15 (m, 4H) ; ¹³C NMR (125 MHz, CD₃OD, 27 °C) δ 21.1, 33.6, 40.2, 47.9 (2C), 60.1 (2C), 61.1, 129.6 (2C), 130.1 (2C), 136.7, 138.1 ; HRMS (ESI-Orbitrap) [M+H]⁺ calcd for C₁₄H₂₄N₃, 234.1965 found 234.1956. 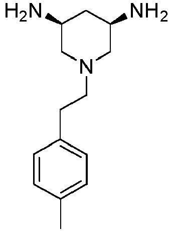
**ECO01-030-C 1-(3-methylphenethyl)piperidine-3,5-diamine** : 12 mg, 26%, colorless paste ;
   ¹H NMR (500 MHz, CD₃OD, 27 °C) δ 1.14 (q, *J* = 10.9 Hz, 1H), 1.99 (t, *J* = 9.4 Hz, 2H), 2.11 (d, *J* = 12.3 Hz, 1H), 2.30 (s, 3H), 2.65-2.68 (m, 2H), 2.76-2.77 (m, 2H), 3.00-3.02 (m, 4H), 6.98-7.03 (m, 3H), 7.14 (t, *J* = 7.5 Hz, 1H) ; ¹³C NMR (125 MHz, CD₃OD, 27 °C) δ 21.4, 33.9, 39.8, 47.9 (2C), 60.1 (2C), 61.1, 126.8, 127.9, 129.4, 130.4, 139.1, 141.2 ; HRMS (ESI-Orbitrap) [M+H]⁺ calcd for C₁₄H₂₄N₃, 234.1965 found 234.1957. 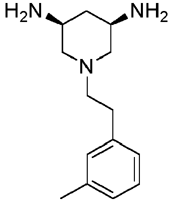
**ECO01-031-C 1-(2-methoxyphenethyl)piperidine-3,5-diamine** : 18 mg, 37%, white solid ;
   ¹H NMR (500 MHz, CD₃OD, 27 °C) δ 1.27 (q, *J* = 11.0 Hz, 1H), 2.08 (t, *J* = 9.0 Hz, 2H), 2.17 (d, *J* = 11.8 Hz, 1H), 2.65 (t, *J* = 8.7 Hz, 2H), 2.82 (t, *J* = 8.8 Hz, 2H), 3.03 (d, *J* = 10.5 Hz, 2H), 3.10 (t, *J* = 9.6 Hz, 2H), 3.82 (s, 3H), 6.85 (t, *J* = 7.1 Hz, 1H), 6.91 (d, *J* = 8.1 Hz, 1H), 7.17 (d, *J* = 7.6 Hz, 1H), 7.12 (t, *J* = 7.3 Hz, 1H); ¹³C NMR (125 MHz, CD₃OD, 27 °C) δ 28.5, 38.4, 47.8, 55.8, 59.1, 59.2 (3C), 111.5, 121.5, 128.7, 129.1, 131.2, 158.9. HRMS (ESI-Orbitrap) [M+H]⁺ calcd for C₁₄H₂₄N₃O, 250.1914 found 250.1903. 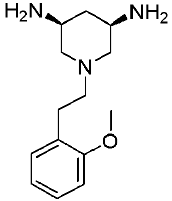
**ECO01-033-C 1-(2-(benzo[d][1,3]dioxol-5-yl)ethyl)piperidine-3,5-diamine** : 27 mg, 54%, pale brown solid ;
   ¹H NMR (500 MHz, CD₃OD, 27 °C) δ 1.05 (q, *J* = 11.2 Hz, 1H), 1.87-1.89 (m, 2H), 2.12 (d, *J* = 11.4 Hz, 1H), 2.60-2.63 (m, 2H), 2.73-2.75 (m, 2H), 2.93-3.01 (m, 4H), 5.88 (s, 2H), 6.67-6.72 (m, 3H) ; ¹³C NMR (125 MHz, CD₃OD, 27 °C) δ 24.2, 33.7, 41.2, 47.9, 60.7 (2C), 61.2, 102.0, 109.1, 110.0, 122.5, 135.0, 147.3, 149.0 ; HRMS (ESI-Orbitrap) [M+H]⁺ calcd for C₁₄H₂₂N₃O₂, 264.1707 found 264.1695. 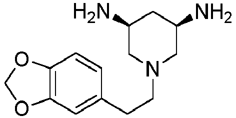
**ECO01-035-C 1-(3-phenylpropyl)piperidine-3,5-diamine** : 22 mg, 48%, brown paste ;
   ¹H NMR (500 MHz, CD₃OD, 27 °C) δ 1.18 (q, *J* = 10.2 Hz, 1H), 1.82 (m, 2H), 1.86 (br s, 1H), 2.13 (d, *J* = 12.2 Hz, 2H), 2.44-2.49 (m, 2H), 2.66 (t, *J* = 7.6 Hz, 2H), 2.92 (d, *J* = 10.4 Hz, 2H), 3.05-3.08 (m, 2H), 7.15-7.21 (m, 3H), 7.25-7.28 (m, 2H) ; ¹³C NMR (125 MHz, CD₃OD, 27 °C) δ 22.5, 28.0, 33.0, 46.5 (2C), 56.9, 58.3 (2C), 125.4 (2C), 127.9 (2C), 141.8 (2C) ; HRMS (ESI-Orbitrap) [M+H]⁺ calcd for C₁₄H₂₄N₃, 234.1965 found 234.1969. 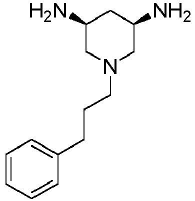
**ECO01-037-C 1-phenylpiperidine-3,5-diamine** : 17 mg, 40%, colorless paste ;
   ¹H NMR (500 MHz, CD₃OD, 27 °C) δ 1.34-1.39 (m, 1H), 2.29-2.32 (m, 1H), 2.68 (t, *J* = 10.8 Hz, 2H), 3.01-3.22 (m, 2H), 3.74-3.77 (m, 2H), 6.94 (t, *J* = 7.3 Hz, 1H), 7.08 (d, *J* = 8.5 Hz, 2H), 7.31-7.34 (m, 2H) ; ¹³C NMR (125 MHz, CD₃OD, 27 °C) δ 38.4, 46.5 (2C), 55.6 (2C), 116.9 (2C), 120.0, 128.7 (2C), 150.8 ; HRMS (ESI-Orbitrap) [M+H]⁺ calcd for C₁₁H₁₈N₃, 192.1495 found 192.1501. 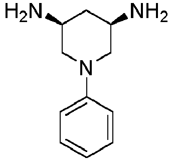
**ECO01-039-C 1-(4-(methylsulfonyl)phenethyl)piperidine-3,5-diamine** : 14 mg, 26%, white paste ;
   ¹H NMR (500 MHz, CD₃OD, 27 °C) δ 1.10 (q, *J* = 11.2 Hz, 1H), 1.94-1.99 (m, 2H), 2.16 (d, *J* = 12.2 Hz, 1H), 2.74-2.77 (m, 2H), 2.99 (t, *J* = 7.6 Hz, 4H), 3.04 (d, *J* = 10.5 Hz, 2H), 3.14 (s, 3H), 7.55 (d, *J* = 8.2 Hz, 2H), 792 (d, *J* = 8.2 Hz, 2H) ; ¹³C NMR (125 MHz, CD₃OD, 27 °C) δ 32.4, 39.9, 43.0, 46.6 (2C), 58.7, 59.3 (2C), 127.0 (2C), 129.4 (2C), 138.4, 146.9 ; HRMS (ESI-Orbitrap) [M+H]⁺ calcd for C₁₄H₂₄N₃O₂S, 298.1584 found 298.1573. 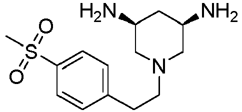
**ECO01-042-C 1-(benzo[d][1,3]dioxol-5-yl)piperidine-3,5-diamine** : 10.6 mg, 22%, colorless oil ;
   ¹H NMR (500 MHz, CD₃OD, 27 °C) δ 1.17 (q, *J* = 8.4 Hz, 1H), 2.20-2.23 (m, 1H), 2.48 (t, *J* = 7.1 Hz, 2H), 3.05-3.10 (m, 2H), 3.50-3.52 (m, 2H), 5.91 (s, 2H), 6.45-6.49 (m, 1H), 6.65-6.67 (m, 1H), 6.73-6.77 (m, 1H); ¹³C NMR (125 MHz, CD₃OD, 27 °C) δ 31.7, 40.1, 46.7 (2C), 58.1, 100.5, 100.7, 107.5, 109.8, 142.0, 146.9, 148.2 ; HRMS (ESI-Orbitrap) [M+H]⁺ calcd for C₁₂H₁₈N₃O₂, 236.1394 found 236.1386. 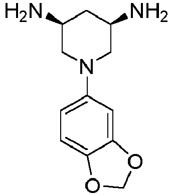
**ECO01-055-C 1-(3-methoxyphenyl)piperidine-3,5-diamine :**
   12 mg, 26%, white paste ;
   ¹H NMR (500 MHz, CD₃OD, 27 °C) δ 1.19 (q, *J* = 11.3 Hz, 1H), 2.20-2.23 (m, 1H), 2.50 (t, *J* = 7.3 Hz, 2H), 3.02-3.07 (m, 2H), 3.69 (dd, *J* = 11.8, 5.3 Hz, 2H), 3.78 (s, 3H), 6.45 (dd, *J* = 8.0, 3.4 Hz, 1H), 6.53-6.55 (m, 1H), 6.60 (dd, *J* = 8.2, 3.5 Hz, 1H), 7.15 (t, *J* = 8.2 Hz, 1H) ; ¹³C NMR (125 MHz, CD₃OD, 27 °C) δ 41.7, 47.9 (2C), 55.7, 57.6 (2C), 104.4, 106.2, 110.8, 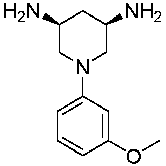 130.9, 153.6, 162.1 ; HRMS (ESI-Orbitrap) [M+H]+ calcd for C₁₂H₂₀N₃O, 222.1601 found 222.1595.
**ECO01-056-C 1-(4-methoxyphenyl)piperidine-3,5-diamine :**
   21 mg, 46%, brown solid ;
   ¹H NMR (500 MHz, CD₃OD, 27 °C) δ 1.13 (q, *J* = 11.3 Hz, 1H), 2.17-2.21 (m, 1H), 2.39 (t, *J* = 10.7 Hz, 2H), 3.03-3.07 (m, 2H), 3.50 (dd, *J* = 11.0, 5.1 Hz, 2H), 3.76 (s, 3H), 6.84 (d, *J* = 9.1 Hz, 2H), 6.97 (d, *J* = 9.1 Hz, 2H); ¹³C NMR (125 MHz, CD₃OD, 27 °C) δ 42.2, 48.5 (2C), 56.2, 59.9 (2C), 115.7 (2C), 120.8 (2C), 146.9, 156.1; HRMS (ESI-Orbitrap) [M+H]+ calcd for C₁₂H₂₀N₃O, 222.1601 found 222.1592. 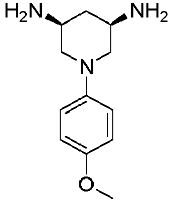
**ECO02-003-C 3-(3,5-diaminopiperidin-1-yl)-N-phenylbenzamide** : 15 mg, 28%, colorless oil ;
   ¹H NMR (500 MHz, CD₃OD, 27 °C) δ 1.20 (q, *J* = 11.5 Hz, 1H), 2.23 (m, 1H), 2.54 (t, *J* = 7.4 Hz, 2H), 3.01-3.06 (m, 2H), 3.83 (dd, *J* = 11.7, 5.3 Hz, 2H), 7.17 (t, *J* = 7.4 Hz, 1H), 7.20-7.24 (m, 1H), 7.36-7.40 (m, 4H), 7.52 (s, 1H), 7.69 (d, *J* = 7.9 Hz, 2H) ; ¹³C NMR (125 MHz, CD₃OD, 27 °C) δ 42.5, 47.9 (2C), 57.4 (2C), 116.6, 119.5, 121.0, 122.5 (2C), 125.7, 129.8 (2C), 130.5, 137.2, 139.8, 152.4, 169.4 ; HRMS (ESI-Orbitrap) [M+H]+ calcd for C₁₈H₂₃N₄O, 311.1866 found 311.1857. 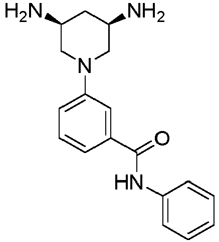
**ECO02-005-C 1-(4-ethoxyphenethyl)piperidine-3,5-diamine :** 15 mg, 38%, white paste ;
   ¹H NMR (500 MHz, CD₃OD, 27 °C) δ 0.97 (q, *J* = 11.8 Hz, 1H), 1.37 (t, *J* = 7.0 Hz, 3H), 1.81 (t, *J* = 10.4 Hz, 2H), 2.13 (d, *J* = 12.5 Hz, 1H), 2.60-2.64 (m, 2H), 2.75-2.78 (m, 2H), 2.90-2.94 (m, 2H), 3.03 (dd, *J* = 11.0, 5.2 Hz, 2H), 4.00 (t, *J* = 7.0 Hz, 2H), 6.83 (d, *J* = 8.5 Hz, 2H), 7.11 (d, *J* = 8.5 Hz, 2H) ; ¹³C NMR (125 MHz, CD₃OD, 27 °C) δ 13.7, 31.7, 40.3, 46.5, 59.5, 59.9, 63.0, 114.1 (2C), 129.1 (2C), 131.7, 157.4; HRMS (ESI-Orbitrap) [M+H]+ calcd for C₁₅H₂₆N₃O, 264.2070 found 264.2059. 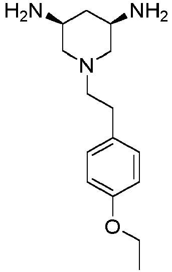
**ECO02-007-C azepan-1-yl(4-(3,5-diaminopiperidin-1-yl)phenyl)methanone** : 15 mg, 28%, colorless oil;
   ¹H NMR (500 MHz, CD₃OD, 27 °C) δ 1.17 (q, *J* = 11.7 Hz, 1H), 1.55 (td, *J* = 12.6, 7.5 Hz, 1H), 1.65 (br s, 6H), 1.86 (br s, 2H), 2.23 (td, *J* = 12.5, 8.0 Hz, 1H), 2.50 (t, *J* = 7.5 Hz, 2H), 2.94-2.99 (m, 2H), 3.23-3.34 (m, 1H), 3.54 (br s, 2H), 3.60 (d, *J* = 5.3 Hz, 2H), 3.89 (dd, *J* = 12.1, 4.0 Hz, 2H), 7.04 (d, *J* = 8.8 Hz, 2H), 7.33 (d, *J* = 8.8 Hz, 2H) ; ¹³C NMR (125 MHz, CD₃OD, 27 °C) δ 25.9, 26.8, 27.5, 29.0, 32.3, 41.6, 46.2, 46.3, 55.4, 62.2, 62.5, 114.8 (2C), 126.2, 127.8 (2C), 151.5, 172.6 ; HRMS (ESI-Orbitrap) [M+H]+ calcd for C₁₈H₂₉N₄O, 317.2336 found 317.2324. 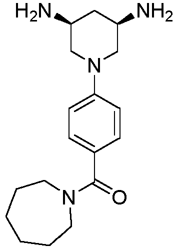
**ECO02-025-C 1-(4-methoxyphenethyl)piperidine-3,5-diamine** : 16 mg, 25%, colorless paste ;
   ¹H NMR (500 MHz, CD₃OD, 27 °C) δ 0.97 (q, *J* = 11.5 Hz, 1H), 1.79 (t, *J* = 10.5 Hz, 2H), 2.10-2.14 (m, 1H), 2.59-2.62 (m, 2H), 2.74-2.77 (m, 2H), 2.86-2.93 (m, 2H), 3.03 (dd, *J* = 10.9, 4.7 Hz, 2H), 3.65 (s, 3H), 6.84 (d, *J* = 8.6 Hz, 2H), 7.11 (d, *J* = 8.6 Hz, 2H) ; ¹³C NMR (125 MHz, CD₃OD, 27 °C) δ 33.2, 42.8, 48.1 (2C), 55.7 (2C), 61.4, 61.5, 115.0 (2C), 130.6 (2C), 133.2, 159.6. 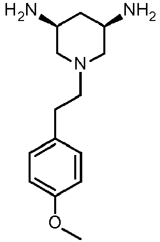
**ECO02-026-C 1-(4-phenylbutyl)piperidine-3,5-diamine :** 12 mg, 25%, white paste ;
   ¹H NMR (500 MHz, CD₃OD, 27 °C) δ 0.97 (q, *J* = 11.4 Hz, 1H), 1.53-1.56 (m, 2H), 1.63-1.75 (m, 4H), 2.11-2.14 (m, 1H), 2.42-2.46 (m, 2H), 2.66 (t, *J* = 7.4 Hz, 2H), 2.88-2.96 (m, 3H), 7.16-7.20 (m, 3H), 7.23-7.28 (m, 2H) ; ¹³C NMR (125 MHz, CD₃OD, 27 °C) δ 25.6, 29.0, 35.2, 40.9 (2C), 46.5, 57.8, 59.9 (2C), 125.3, 127.9 (2C), 128.0 (2C), 142.2.
**ECO02-027-C 1-phenethylpiperidine-3,5-diamine :**
   12 mg, 27%, colorless oil ;
   ¹H NMR (500 MHz, CD₃OD, 27 °C) δ 0.94 (q, *J* = 11.6 Hz, 1H), 1.77 (t, *J* = 10.6 Hz, 2H), 2.11-2.17 (m, 1H), 2.64-2.67 (m, 2H), 2.82-2.91 (m, 4H), 3.04-3.08 (m, 2H), 7.17-7.23 (m, 3H), 7.25-7.30 (m, 2H) ; ¹³C NMR (125 MHz, CD₃OD, 27 °C) δ 32.6, 42.0, 46.6 (2C), 59.8, 60.4 (2C), 125.7, 128.0 (2C), 128.2 (2C), 139.9. 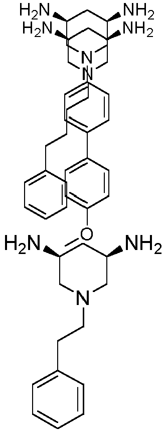
**ECO02-028-C 4-(2-(3,5-diaminopiperidin-1-yl)ethyl)phenol :**
   9.5 mg, 22%, colorless oil ;
   ¹H NMR (500 MHz, CD₃OD, 27 °C) δ 0.96 (q, *J* = 11.6 Hz, 1H), 1.77 (t, *J* = 10.6 Hz, 2H), 2.13-2.16 (m, 1H), 2.60-2.64 (m, 2H), 2.72-2.75 (m, 2H), 2.87-2.92 (m, 2H), 3.03-3.07 (m, 2H), 6.71 (d, *J* = 8.5 Hz, 2H), 7.04 (d, *J* = 8.5 Hz, 2H) ; ¹³C NMR (125 MHz, CD₃OD, 27 °C) δ 31.6, 41.6, 46.6 (2C), 60.2 (3C), 114.8 (2C), 129.1 (2C), 130.5, 155.3. 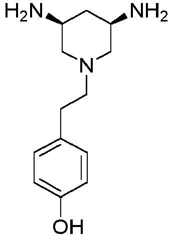
**ECO02-029-C 1-(4'-methoxy-[1,1'-biphenyl]-4-yl)piperidine-3,5-diamine4-(2-((3S,5R)-3,5-diaminopiperidin-1-yl)ethyl)phenol** :
   9 mg, 17%, off-white paste ;
   ¹H NMR (500 MHz, CD₃OD, 27 °C) δ 1.27 (q, *J* = 11.2 Hz, 1H), 2.24-2.27 (m, 1H), 2.58 (dd, *J* = 11.4, 10.3 Hz, 2H), 3.10-3.13 (m, 2H), 3.75 (dd, *J* = 11.5, 5.1 Hz, 2H), 3.85 (s, 3H), 6.98 (d, *J* = 8.7 Hz, 2H), 7.07 (d, *J* = 8.7 Hz, 2H), 7.51 (t, *J* = 8.5 Hz, 4H) ; ¹³C NMR (125 MHz, CD₃OD, 27 °C) δ 39.8, 46.5 (2C), 54.4, 56.0 (2C), 113.8 (2C), 116.9 (2C), 126.7 (2C), 126.9 (2C), 132.4, 133.2, 149.6, 158.7.
**ECO02-030-C 1-(3,5-diaminopiperidin-1-yl)-2-(3-methoxyphenyl)ethanone :** 13 mg, 26%, colorless paste ;
   ¹H NMR (500 MHz, CD₃OD, 27 °C) δ 1.10 (q, *J* = 11.6 Hz, 1H), 2.12-2.15 (m, 1H), 2.28 (dd, *J* = 12.5, 10.9 Hz, 1H), 2.47-2.53 (m, 1H), 2.60-2.71 (m, 2H), 3.78 (s, 2H), 3.80 (s, 3H), 3.98-4.01 (m, 1H), 4.57-4.60 (m, 1H), 6.84-6.87 (m, 3H), 7.24-7.27 (m, 1H) ; ¹³C NMR (125 MHz, CD₃OD, 27 °C) δ 40.1, 41.7, 45.6, 47.1, 48.8, 52.8, 54.2, 112.0, 114.0, 120.6, 129.4, 136.3, 160.1, 170.7. 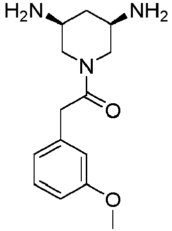
**ECO02-031-C 1-(3,5-diaminopiperidin-1-yl)-2-(3-methoxyphenyl)ethanone** : 7.5 mg, 15%, colorless oil ;
   ¹H NMR (500 MHz, CD₃OD, 27 °C) δ 1.10 (q, *J* = 12.2 Hz, 1H), 2.08-2.15 (m, 1H), 2.23-2.24 (m, 1H), 2.42-2.48 (m, 1H), 2.60-2.68 (m, 2H), 3.74 (br s, 2H), 3.80 (s, 3H), 3.99-4.02 (m, 1H), 4.57-4.59 (m, 1H), 6.90 (d, *J* = 8.1 Hz, 2H), 7.20 (d, *J* = 8.4 Hz, 2H) ; ¹³C NMR (125 MHz, CD₃OD, 27 °C) δ 39.2, 41.8, 46.4, 47.1, 48.8, 52.7, 54.2, 113.8 (2C), 126.8, 129.3 (2C), 158.7, 171.2. 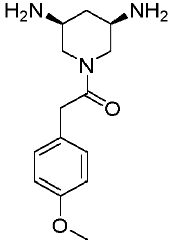
**ECO03-002-C N-(4-(3,5-diaminopiperidin-1-yl)phenyl)benzamide** : 9 mg, 17 % colorless oil;
   ¹H NMR (500 MHz, CD₃OD , 27 °C) δ 1.09 (q, *J* = 11.4 Hz, 1H), 2.20-2.23 (m, 1H), 2.40 (t, *J* = 11.0 Hz, 2H), 2.96-3.03 (m, 2H), 3.69-3.73 (m, 2H), 7.01 (d, *J* = 9.0 Hz, 2H), 7.51 (t, *J* = 7.5 Hz, 2H), 7.56-7.58 (m, 3H), 7.93 (d, *J* = 7.3 Hz, 2H) ; ¹³C NMR (125 MHz, CD₃OD, 27 °C) δ 41.5, 46.6 (2C), 56.9 (2C), 116.7 (2C), 122.3 (2C), 127.1 (2C), 128.2 (2C), 130.7, 131.3, 134.9, 148.1, 167.2 ; HRMS (ESI- Orbitrap) [M+H]⁺ calcd for C₁₈H₂₃N₄O, 311.1866 found 311.1864.

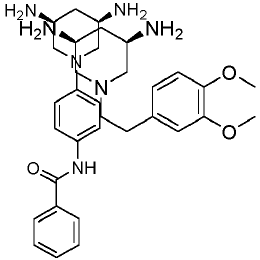

Hydrogenolysis under batch conditions according to the Journal of Organic Chemistry 2013, 78, 12236-12242 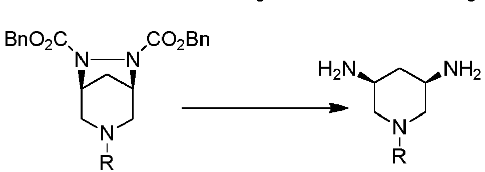
**ECO01-041-C 1-(2,3-dihydro-1H-inden-2-yl)piperidine-3,5-diamine** : 18 mg, 77%, beige solid ;
   ¹H NMR (500 MHz, CD₃OD, 27 °C) δ 1.00 (q, *J* = 11.7 Hz, 1H), 1.82 (t, *J* = 10.7 Hz, 2H), 2.18-2.21 (m, 1H), 2.92-2.97 (m, 4H), 3.12-3.21 (m, 4H), 3.31 (quint, *J* = 7.9 Hz, 1H), 7.16-7.18 (m, 2H), 7.23-7.24 (m, 2H); ¹³C NMR (125 MHz, CD₃OD, 27 °C) δ 37.8 (2C), 43.3, 48.0 (2C), 59.9 (2C), 67.7, 125.4 (2C), 127.7 (2C), 142.3 (2C) ; HRMS (ESI-Orbitrap) [M+H]+ calcd for C₁₄H₂₂N₃, 232.1808 found 232.1800. 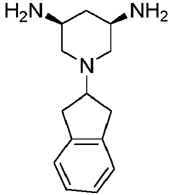
**AB-77 (3*S*,5*R*)-1-(3,4-dimethoxyphenethyl)piperidine-3,5-diamine :**
   62 mg, 88%, colorless oil; ¹H NMR (400 MHz, CDCl₃, δ) 0.89 (q, *J=* 11.7 Hz, 1H), 1.25 (br s, 4H), 1.71 (t, *J=* 10.1 Hz, 2H), 2.17 (d, *J=* 11.7 Hz, 1H), 2.60-2.71 (m, 2H), 2.75-2.85 (m, 2H), 2.92-3.03 (m, 2H), 3.06 (d, *J=* 10.1 Hz, 2H), 3.90 (s, 3H), 3.91 (s, 3H), 6.77-6.85 (m, 3H); ¹³C NMR (100 MHz, CDCl₃, δ) 33.1, 44.9, 47.6, 55.8, 55.9, 60.3, 62.3, 111.1, 111.9, 120.5, 132.8, 147.2, 148.7; HRMS (ESI-TOF)[M+H]⁺ calcd for C₁₅H₂₆N₃O₂ 280.2025, found 280.2029
**AB-81 (S)-methyl 2-((3S,5R)-3,5-diaminopiperidin-1-yl)-3-phenylpropanoate :** 39 mg, 98%, colorless oil; ¹H NMR (400 MHz, CDCl₃, δ) 0.85 (q, *J* = 11.4 Hz, 1H), 1.47 (br s, 4H), 1.89 (t, *J* = 10.3 Hz, 1H), 2.05 (t, *J* = 10.3 Hz, 1H), 2.12 (d, *J* = 11.4 Hz, 1H), 2.80-2.99 (m, 4H), 3.04-3.15 (m, 2H), 3.50 (dd, *J* = 7.9 Hz, *J* = 7.0 Hz, 1H), 3.64 (s, 3H), 7.15-7.35 (m, 5H); ¹³C NMR (75 MHz, CDCl₃, δ) 35.6, 44.8, 47.7, 47.9, 51.1, 56.3, 61.2, 69.1, 126.4, 128.3, 129.1, 138.1, 171.7; HRMS (ESI-TOF)[M+H]⁺ calcd for C₁₅H₂₄N₃O₂ 278.1869, found 278.1864; [α]_{D}²⁰ -19.0 (c 1.0, CH₃OH) 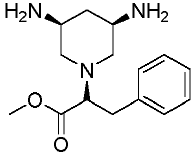
**AB-84 (*3S*,*5R*)-1-(2-(5-methoxy-1H-indol-3-yl)ethyl)piperidine-3,5-diamine:** 29 mg, quantitative, colorless oil; ¹H NMR (400 MHz, CDCl₃, δ) 0.90 (q, *J* = 11.4 Hz, 1H), 1.30 (br s, 4H), 1.75 (t, *J* = 10.3 Hz, 2H), 2.19 (d, *J* = 11.4 Hz, 1H), 2.74-2.81 (m, 2H), 2.95-3.07 (m, 4H), 3.13 (dd, *J* = 10.3 Hz, *J* = 3.2 Hz, 2H), 3.90 (s, 3H), 6.90 (d, *J* = 8.8 Hz, 1H), 7.04 (s, 1H), 7.08 (s, 1H), 7.30 (d, *J* = 8.8 Hz, 1H), 8.30 (s, 1H); ¹³C NMR (100 MHz, CDCl₃, δ) 22.9, 45.0, 47.7, 56.0, 58.9, 62.4, 100.6, 111.9, 112.1, 113.9, 122.4, 127.8, 131.4, 153.9; HRMS (ESI-TOF)[M+H]⁺ calcd for C₁₆H₂₅N₄O 289.2028, found 289.2021 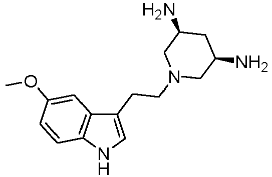
**AB-86 (*3S,5R*)-1-(1H-indol-5-yl)piperidine-3,5-diamine:** 22 mg, 79%, colorless oil; ¹H NMR (300 MHz, CDCl₃, δ) 0.96 (q, *J* = 11.6 Hz, 1H), 1.39 (br s, 4H), 2.24 (d, *J* = 11.6 Hz, 1H), 2.33 (dd, *J* = 11.1 Hz, *J* = 10.3 Hz, 2H), 3.12 (m, 2H), 3.60 (dd, *J* = 11.1 Hz, *J* = 4.4 Hz, 2H), 6.48 (br s, 1H), 6.97 (dd, *J* = 8.8 Hz, *J* = 2.4 Hz, 1H), 7.17 (t, *J* = 2.7 Hz, 1H), 7.19 (d, *J* = 2.4 Hz, 1H), 7.29 (d, *J* = 8.8 Hz, 1H), 8.34 (br s, 1H); ¹³C NMR (75 MHz, CDCl₃, δ) 44.7, 47.8, 61.3, 102.3, 108.5, 111.5, 116.4, 124.7, 128.3, 131.4, 145.6; HRMS (ESI-TOF)[M+H]⁺ calcd for C₁₃H₁₉N₄ 231.1610, found 231.1606 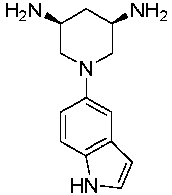
**AB-103 (3S,3'R,3"S,5R,5'S,5"R)-1'-(3,4-dimethoxyphenethyl)-[1,3':5',1"-terpiperidine]-3,3",5,5"-tetraamine:** 44 mg, quantitative, colorless oil; ¹H NMR (500 MHz, CD₃OD, δ) 1.55-1.70 (m, 3H), 2.12 (d, *J* = 10.9 Hz, 1H), 2.45-2.56 (m, 6H), 2.60-2.69 (m, 2H), 2.95-3.11 (m, 6H), 3.15-3.26 (m, 4H), 3.30-3.37 (m, 6H), 3.82 (s, 3H), 3.86 (s, 3H), 6.84 (d, *J* = 8.5 Hz, 1H), 6.91 (d, *J* = 8.5 Hz, 1H), 6.94 (s, 1H); ¹³C NMR (125 MHz, CD₃OD, δ) 28.7, 32.3, 34.5, 48.0, 48.1, 52.8, 53.5, 55.2, 56.7, 60.0, 60.8, 113.5, 114.0, 122.2, 132.4, 149.4, 150.6; HRMS (ESI-TOF)[M+Na]⁺ calcd for C₂₅H₄₅N₇O₂Na 498.3532, found 498.3526 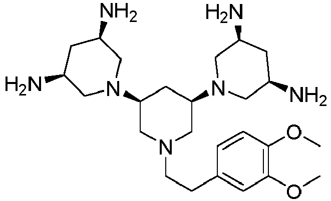
**AB-109 F3 (R)-methyl 2-((3S,5R)-3,5-diaminopiperidin-1-yl)-3-phenylpropanoate:** 13 mg, 52%, colorless oil; ¹H NMR (400 MHz, CDCl₃, δ) 0.85 (q, *J* = 11.4 Hz, 1H), 1.47 (br s, 4H), 1.89 (t, *J* = 10.3 Hz, 1H), 2.05 (t, *J* = 10.3 Hz, 1H), 2.12 (d, *J* = 11.4 Hz, 1H), 2.80-2.99 (m, 4H), 3.04-3.15 (m, 2H), 3.50 (dd, *J* = 7.9 Hz, *J* = 7.0 Hz, 1H), 3.64 (s, 3H), 7.15-7.35 (m, 5H); ¹³C NMR (75 MHz, CDCl₃, δ) 35.6, 44.8, 47.7, 47.9, 51.1, 56.3, 61.2, 69.1, 126.4, 128.3, 129.1, 138.1, 171.7; HRMS (ESI-TOF)[M+H]⁺ calcd for C₁₅H₂₄N₃O₂ 278.1869, found 278.1864; [α]_{D}²⁰ = + 21.1(c = 1.0, MeOH) 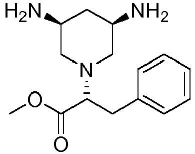
**AB-116 (3S,5R)-1-(3,4,5-trimethoxyphenyl)piperidine-3,5-diamine** : 45 mg, 88%, colorless oil; ¹H NMR (300 MHz, CD₃OD, δ) 1.06 (q, *J* = 11.4 Hz, 1H), 2.20 (br d, *J* = 11.4 Hz, 1H), 2.35 (dd, *J* = 11.6 Hz, *J* = 10.4 Hz, 2H), 2.95 (m, 2H), 3.65 (dd, *J* = 11.6 Hz, *J* = 4.3 Hz, 2H), 3.71 (s, 3H), 3.84 (s, 6H), 6.27 (s, 2H); ¹³C NMR (75 MHz, CD₃OD, δ) 41.9, 46.8, 55.1, 57.5, 59.9, 95.0, 131.5, 148.2, 153.4; HRMS (ESI-TOF)[M+H]⁺ calcd for C₁₄H₂₄N₃O₃ 282.1818, found 282.1815 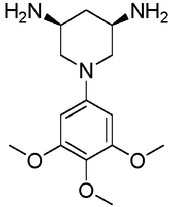
**AB-120 N-(4-((3*S*,5*R*)-3,5-diaminopiperidin-1-yl)-2-hydroxyphenyl)benzamide** : 21 mg, 95%, orange oil; ¹H NMR (300 MHz, CD₃OD, δ) 1.45 (q, *J* = 11.0 Hz, 1H), 2.26 (d, *J* = 11.0 Hz, 1H), 2.74 (dd, *J* = 11.9 Hz, *J* = 9.2 Hz, 2H), 3.22 (m, 2H), 3.66 (dd, *J* = 11.9 Hz, *J* = 2.9 Hz, 2H), 6.57 (dd, *J* = 8.8 Hz, *J* = 2.3 Hz, 1H), 6.62 (d, *J* = 2.3 Hz, 1H), 7.52-7.60 (m, 4H), 7.97 (d, *J* = 8.0 Hz, 2H); ¹³C NMR (75 MHz, CD₃OD, δ) 36.4, 46.3, 54.6, 105.1, 108.4, 118.8, 124.3, 127.2, 128.4, 131.6, 134.2, 149.5, 150.2, 167.3; HRMS (ESI-TOF)[M+H]⁺ calcd for C₁₈H₂₃N₄O₂ 327.1821, found 327.1821 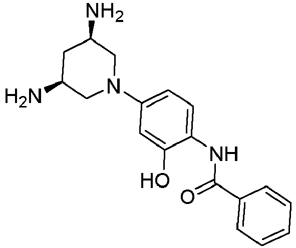
**AB-378 (*3S,5R*)-1-(4-methoxybenzyl)piperidine-3,5-diamine** 40 mg, quantitative, colorless oil; ¹H NMR (250 MHz, CD₃OD, δ) 0.84 (q, *J* = 11.6 Hz, 1H), 1.62 (t, *J* = 10.4 Hz,2H), 2.09 (d, *J* = 11.6 Hz, 1H), 2.75-2.87 (m, 2H), 2.94 (dd, *J* = 10.4 Hz, *J* = 4.4 Hz, 2H), 3.50 (s, 2H), 3.77 (s, 3H), 6.86 (d, *J* = 8.5 Hz, 2H), 7.23 (d, *J* = 8.5 Hz, 2H); ¹³C NMR (125 MHz, CD₃OD, δ) 43.3, 48.1, 55.8, 61.5, 63.0, 114.8, 130.4, 131.9, 160.6; HRMS (ESI-Orbitrap)[M+H]⁺ calcd for C₁₃H₂₂N₃O 236.1762, found 236.1756 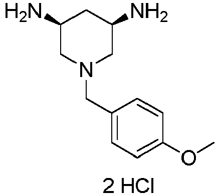
**AB289-2 N-(6-amino-1-(3,5-diaminopiperidin-1-yl)-1-oxohexan-2-yl)-4-ethylbenzamide** 1H NMR (500MHz, (CD3)2SO, 70 °C) δ = 1.27 (t, *J* = 7.6, 3H) ; 1.44-1.67 (m, 2H) ; 1.72-1.80 (m, 2H) ; 1.85-1.96 (m, 3H) ; 2.62 (broad d, *J* = 10.8, 1H) ; 2.72 (q, *J* = 7.6, 2H) ; 2.70-2.78 (m, 1H) ; 2.97 (t, *J* = 7.3, 2H) ; 3.27-3.37 (m, 2H) ; 3.51-3.69 (m, 1H) ; 4.45-4.61 (m, 1H) ; 4.84-4.91 (m, 1H) ; 4.91-5.04 (m, 1H) ; 7.33 (d, *J* = 8.0, 2H) ; 7.80 (d, *J* = 8.0, 2H) 13C NMR (125MHz, (CD3)2SO, 70 °C) δ = 15.8, 24.0, 28.3, 29.8, 31.8, 34.3, 40.5, 45.2, 46.2, 47.0, 49.9, 51.5, 128.7 (2C), 129.1 (2C), 132.1, 150.3, 170.6, 173.6. HRMS (ES+) m/z [M+H]+ Calcd for C20H34N5O2 376.27125, Found 376.27057. 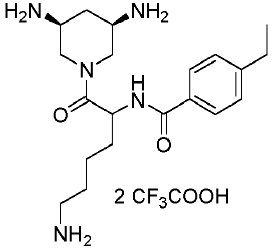
**AB289-1N-(6-amino-1-(3,5-diaminopiperidin-1-yl)-1-oxohexan-2-yl)-4-(1- hydroxyethyl)benzamide** ¹H NMR (500MHz, (CD3)2SO, 70 °C) δ = 1.46 (d, *J* = 6.4, 3H) ; 1.54 1.60 (2 broad s, 2H) ; 1.76 (broad s, 2H) ; 1.85-1.92 (m, 3H, H4) ; 2.62 (broad s, 1H) ; 2.70-2.78 (m, 1H) ; 2.97 (broad s, 2H) ; 3.30-3.37 (m, 2H) ; 3.55-3.68 (m, 1H) ; 4.45-4.59 (m, 1H) ; 4.87-5.05 (m, 3H) ; 7.49 (d, *J* = 8.0, 2H) ; 7.86 (d, *J* = 8.0, 2H) ¹³C NMR (125MHz, (CD3)2SO, 70 °C) δ = 23.9, 25.6, 28.3, 31.8, 34.2, 40.5, 45.2, 46.2, 47.0, 48.9, 51.6, 70.3, 126.6 (2C), 128.7 (2C), 133.4, 152.2, 170.4, 173.6 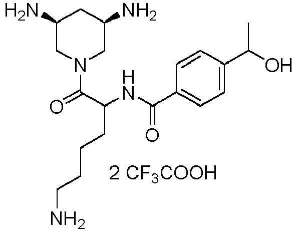
**AB287-F1 1-((3S,5R)-3,5-diaminopiperidin-1-yl)-2-(3,4-dimethoxyphenyl)ethanone** : 16 mg, 76%, colorless oil; ¹H NMR (500 MHz, CD₃OD, δ) 1.43 (q, *J* = 11.8 Hz, 1H), 2.21 (d, *J* = 11.8 Hz, 1H), 2.80-2.87 (m, 2H), 2.95-3.10 (m, 2H), 3.76 (d, *J* = 4.9 Hz, 2H), 3.81 (s, 3H), 3.82 (s, 3H), 3.99 (d, *J* = 13.5 Hz, 1H), 4.41 (d, *J* = 13.5 Hz, 1H), 6.82 (d, *J* = 8.2 Hz, 1H), 6.84 (s, 1H), 6.86 (d, *J* = 8.2 Hz, 1H); ¹³C NMR (125 MHz, CD₃OD, δ) 38.2, 41.0, 47.0, 47.5, 48.0, 52.4, 56.7, 113.4, 114.0, 122.5, 128.9, 149.8, 150.9, 173.2; HRMS (ESI-Orbitrap)[M+H]⁺ calcd for C₁₅H₂₄N₃O₃ 294.1817, found 294.1815

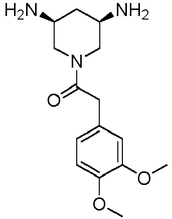

### Synthesis of RA20

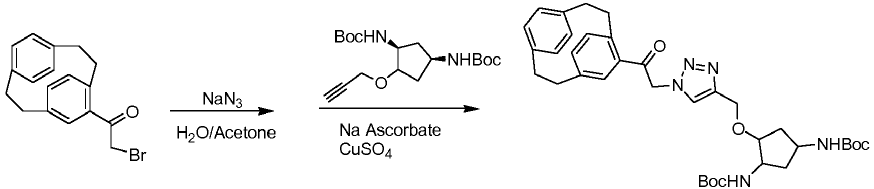

2-Bromo-1-tricyclo[8.2.2.24,7]hexadeca-1(13),4,6,10(14),11,15-hexaen-5-yl-ethanone (150 mg, 0.45 mmol) and NaN₃ (31 mg, 0.47 mmol) were stirred at room temperature in H₂O/acetone (1:2; 4.5 mL) for 3 h. (4-tert-Butoxycarbonylamino-2-prop-2-ynyloxy-cyclopentyl)-carbamic acid tert-butyl ester (135 mg, 0.38 mmol) in acetone (1.5 mL) was then added, followed by the addition of sodium ascorbate (1 M, 0.19 mL) and CuSO₄ (1 M, 0.19 mL). The resultant mixture was then stirred at room temperature until complete consumption of the alkyne and was monitored by TLC. After addition of water the product was extracted with EtOAc, dried over Na₂SO₄ and concentrated *in vacuo.* The crude product was purified flash chromatography on silica gel (cyclohexane:AcOEt 50:50) to give 203 mg (83 %) of the product.
**¹H NMR (400 MHz, CDCl₃):** 1.44 (19H, bs), 1.87 (1H, bs), 2.04 (1H, bs), 2.55 (1H, m), 2.85 (1H, m), 2.99-3.08 (2H, m), 3.12-3.25 (4H, m), 3.85 (1H, m), 3.93 (1H, s), 3.97 (1H, s), 4.08 (1H, bs), 4.77 (2H, m, H-6), 5.29 (1H, d, 17.6, H-9), 5.85 (1H, d, 17.6, H-9), 6.38 (1H, d, 7.8), 6.43 (1H, d, 7.8), 6.51 (1H, d, 7.8), 6.55 (1H, d, 7.8), 6.59 (1H, d, 7.8), 6.74 (1H, d, 7.8), 7.02 (1H, s), 7.75 (1H, s, H-8).
**¹³C NMR (75 MHz, CDCl₃):** 28.5 (6)(CH₃), 34.8 (CH₂), 35.1 (2)(CH₂), 35.9 (CH₂), 37.2 (2)(CH₂), 49.2 (CH), 55.8 (CH), 56.3 (CH₂), 62.8 (CH₂) 79.2 (2)(C), 83.5 (CH), 124.6 (CH), 131.2 (CH), 132.2 (CH), 132.9 (CH), 133.0 (CH), 133.1 (CH), 134.3 (C), 136.9 (CH), 137.8 (CH), 139.3 (C),140.1 (C), 140.4 (C), 142.7 (C), 145.7 (C), 155.5 (2)(C), 192.1 (C).
**MS (ES):** 646 (MH⁺), 688 (MNa⁺).

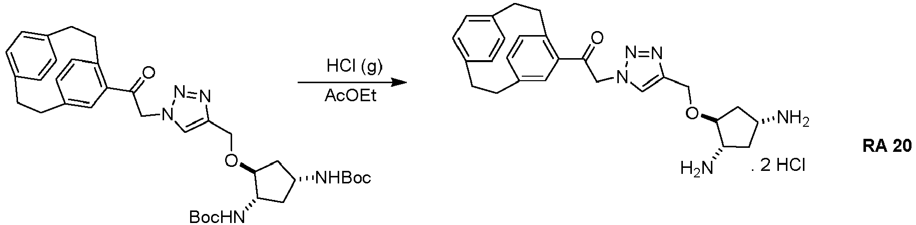

A solution of the Boc-protected triazole derivative in AcOEt (173 mg, 0.26 mmol) was cooled in an ice-bath and HCI (g) was bubbled inside until the formation of a white precipitate (few minutes). The solution was then filtered and rinsed with AcOEt and DCM to obtain 130 mg (96 %) of **RA 20 hydrochloride.**
**¹H NMR (300 MHz, CD₃OD):** 1.86 (1H, m), 2.27 (2H, t, 7.6), 2.68 (1H, dt), 2.92 (1H, m), 3.04-3.28 (6H, m), 3.65 (1H, m), 3.89 (2H, m), 4.35 (1H, m), 4.76 (1H, d, 12.2), 4.84 (1H, d, 12.2), 5.70 (1H, d, 17.8, H-9), 6.16 (1H, d, 17.8, H-9), 6.46 (1H, d, 7.8), 6.55-6.63 (3H, m), 6.79 (1H, d, 7.8), 6.86 (1H, d, 7.8), 7.29 (1H, s), 8.16 (1H, s, H-8).
**¹³C NMR (75 MHz, CD₃OD):** 32.7 (CH₂), 34.0 (CH₂), 34.2 (CH₂), 34.5 (CH₂), 34.6 (CH₂), 35.5 (CH₂), 47.4 (CH), 55.1 (CH), 58.1 (CH₂), 61.5 (CH₂), 80.4 (CH), 83.5 (CH), 128.5 (CH), 131.0 (CH), 132.1 (CH), 132.8 (CH), 133.2 (CH), 133.7 (CH), 133.9 (C), 136.8 (CH), 137.9 (CH), 139.6 (C), 139.8 (C), 140.8 (C), 142.6 (C), 191.5 (C).
**MS (ES):** 446(MH⁺), 468 (MNa⁺).
**PDA25 1-(3,4-dimethoxybenzyl)piperidine-3,5-diamine** ¹H NMR (500MHz, CD3OD) δ = 1.55 (q, *J* = 11.9, 1H, H4) ; 2.13 (t, *J* = 11.0, 2H) ; 2.49 (d, *J* = 11.9, 1H) ; 3.21 (dd, *J* = 11.0, *J* = 4.3, 2H) ; 3.45-3.47 (m, 2H) ; 3.68 (s, 2H) ; 3.77-3.85 (m, 6H) ; 6.88-6.95 (m, 3H) ¹³C NMR (125MHz, CD3OD) δ = 32.4, 45.8 (2C) ; 53.9 (2C), 55.0, 55.1, 61.0, 111.5, 112.6, 121.6, 128.8, 148.9, 149.3. HRMS (APCI+-Orbitrap)[M+H]⁺ calcd for C₁₄H₂₄N₃O₂ 266.18685, Found 266.18570 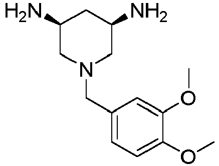
**AB541 1-(adamantan-1-yl)piperidine-3,5-diamine** ¹H NMR (500MHz, CD3OD) δ = 0.86 (q, *J*5 = 11.4) ; 1.62-1.78 (m, 12H) ; 1.81 (d, *J* = 10.5, 2H) ; 2.04-2.15 (m, 4H) ; 2.71-2.82 (m, 2H) ; 3.19 (dd, *J* = 10.5, *J* = 4.3, 2H) ; ¹³C NMR (125MHz, CD3OD) δ = 31.4 (3C), 38.0, 39.6 (6C), 44.1, 48.9 (2C), 53.5, 55.7. HRMS (ESI-Orbitrap)[M+H]⁺ calcd for C₁₅H₅₈N₃ 250.22832, Found 250.22810 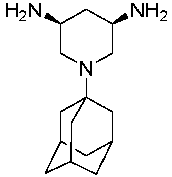
**AB542 1-isopropylpiperidine-3,5-diamine** ¹H NMR (500MHz, CD3OD) δ = 0.88 (q, *J* = 11.6, 1H) ; 1.07 1.08 (2s, 2x3H) ; 1.83 (t, *J* = 10.7, 2H) ; 2.07-2.16 (m, 1H) ; 2.73-2.86 (m, 3H) ; 2.91 (dd, *J* = 10.7, *J* = 4.1, 2H) ¹³C NMR (125MHz, CD3OD) δ = 18.4 (2C), 43.3, 48.5 (2C), 55.8 (C7), 57.2 (2C)

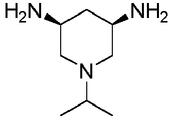

### Acylation of byclic hydrazines

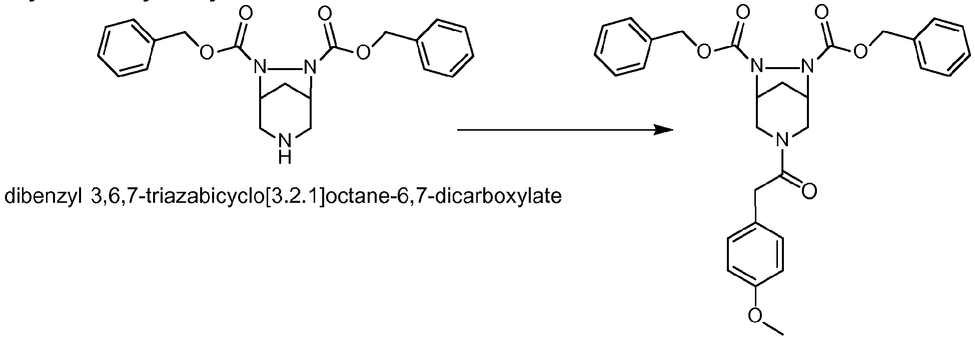

ECO02-018-C dibenzyl 3,6,7-triazabicyclo[3.2.1]octane-6,7-dicarboxylate (350 mg, 0.918 mmol) was solubilized in dry CH₂Cl₂ (5 mL) and in dry DMF (0.2 mL). methoxyphenylacetic acid (168 mg, 1.01 mmol), triethylamine (0.87 ml, 6.43 mmol) and EDC (264 mg, 1.38 mmol) were added. Mixture was stirred at RT under Ar overnight. The reaction was monitored by TLC until disappearance of the initial product. The solution was quenched with NaHCO₃, extracted with CH₂Cl₂. The organic layer was dried over MgSO₄, filtered and evaporated. Flash chromatography (Cyclohexane to Cyclohexane/ Ethyl acetate 4/6) afforded **ECO02-018-C** (178 mg, 37%), colorless oil.
**ECO02-018-C dibenzyl 3-(2-(3 methoxyphenyl)acetyl)-3,6,7-triazabicyclo[3.2.1]octane-6,7-dicarboxylate :** 178 mg, 37%, colorless oil ;
   ¹H NMR (500 MHz, (CD₃)₂SO, 60 °C) δ 1.95-1.97 (m, 1H), 2.09 (d, *J* = 11.6 Hz, 1H), 2.86 (br s, 1H), 3.20 (s, 1H), 3.48 (br s, 1H), 3.58 (br s, 1H), 3.72 (s, 3H), 4.08 (br s, 1H), 4.50 (br s 3H), 5.11 (s, 4H), 6.70 -6.73 (m, 2H), 6.79 (dd, *J* = 7.8, 3.3 Hz, 1H), 7.18 (t, *J* = 7.8 Hz, 1H), 7.35 (br s, 10H) ; ¹³C NMR (125 MHz, (CD₃)₂SO, 60 °C) δ 35.0, 40.6, 46.5, 49.9, 55.5 (2C), 55.9, 68.2 (2C), 112.9, 115.8, 122.2, 128.4-130.1 (12C), 137.0, 137.7, 156.8 (2C), 160.2, 171.6 ; HRMS (ESI-Orbitrap) [M+H]⁺ calcd for C₃₀H₃₂N₃O₆, 530.2286 found 530.2283. 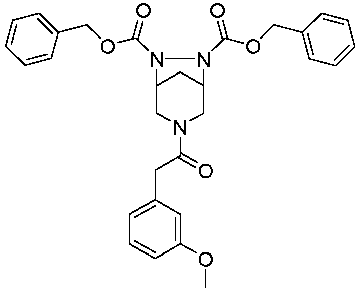
**ECO02-021-C dibenzyl 3-(2-(4-methoxyphenyl)acetyl)-3,6,7-triazabicyclo[3.2.1]octane-6,7-dicarboxylate** : 167 mg, 34%, colorless oil ;
   ¹H NMR (500 MHz, (CD₃)₂SO, 60 °C) δ 1.94-1.98 (m, 1H), 2.08 (d, *J* = 11.5 Hz, 1H), 2.84 (br s, 1H), 3.20-3.25 (m, 1H), 3.44 (br s, 1H), 3.53 (br s, 1H), 3.72 (s, 3H), 4.06 (br s, 1H), 4.50 (br s 3H), 5.11 (s, 4H), 6.84 (d, *J* = 8.2 Hz, 2H), 7.06 (d, *J* = 8.2 Hz, 2H), 7.35 (br s, 10H) ; ¹³C NMR (125 MHz, (CD₃)₂SO, 60 °C) δ 34.0, 38.8, 45.7, 48.6, 54.5 (2C), 55.1, 67.2 (2C), 113.7 (2C), 127.2-128.3 (12C), 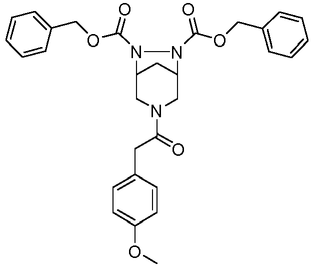 130.2, 136.1 (2C), 156.0 (2C), 158.0, 171.0 ; HRMS (ESI-Orbitrap) [M+H]⁺ calcd for C₃₀H₃₂N₃O₆, 530.2286 found 530.2278.

### Suzuki coupling of bicyclic hydrazines

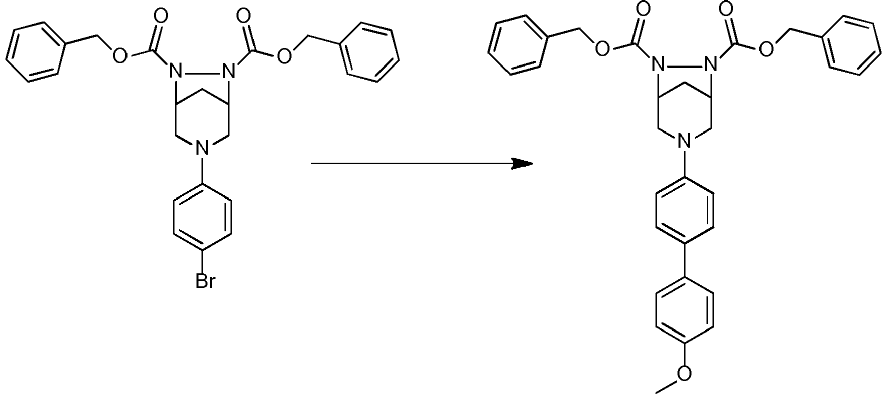

**ECO02-008-C** (414 mg, 0.774 mmol), was solubilized in DMF (4 mL) and water (1.2 mL). 4-methoxyphenylboronic acid (141 mg, 0.929 mmol), K₂CO₃(374 mg, 2.71 mmol), and Pd(Ph₃)₄ (44.7 mg, 0.0387 mmol) were added under Ar. Mixture was stirred at 90°C, overnight under Ar. The reaction was monitored by TLC until disappearance of the initial product. The solution was quenched with NaHCO₃, extracted with CH₂Cl₂. The organic layer was dried over MgSO₄, filtered and evaporated. Flash chromatography (Cyclohexane to Cyclohexane/ Ethyl acetate 7/3) afforded **ECO02-023-C** (273 mg, 48%), white solid.
**ECO02-023-C dibenzyl 3-(4'-methoxy-[1,1'-biphenyl]-4-yl)-3,6,7-triazabicyclo[3.2.1]octane-6,7-dicarboxylate** : 273 mg, 48%, white solid ;
   ¹H NMR (500 MHz, (CD₃)₂SO, 60 °C) δ 2.01-2.05 (m, 1H), 2.08-2.12 (m, 1H), 2.91 (br s, 2H), 3.79 (s, 3H), 3.84 (br s, 2H), 4.63 (br s 2H), 5.11-5.19 (s, 4H), 6.76-6.79 (m, 2H), 6.99 (d, *J* = 8.8 Hz, 2H), 7.32 (br s, 10H), 7.79 (d, *J* = 8.7 Hz, 2H), 7.51 (d, *J* = 7.9 Hz, 2H) ; ¹³C NMR (125 MHz, (CD₃)₂SO, 60 °C) δ 34.0, 48.4, 50.5, 55.2 (3C), 67.0 (2C), 113.7, 114.4, 126.6-128.3 (16C), 129.6, 131.4, 132.8, 136.2, 148.9, 156.2 (2C), 158.2 ; HRMS (ESI-Orbitrap) [M+H]⁺ calcd for C₃₄H₃₄N₃O₅, 564.2493 found 564.2480.

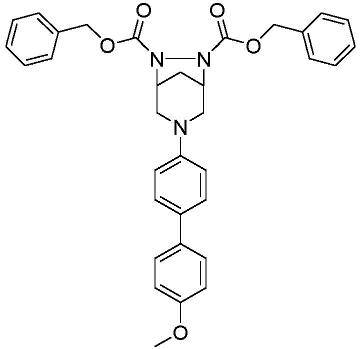

### Acetylation of bicyclic hydrazine

**ECO02-045-C dibenzyl 3-(3-acetoxyphenethyl)-3,6,7-triazabicyclo[3.2.1]octane-6,7-dicarboxylate :** 105 mg, quant., transparent oil ;
¹H NMR (500 MHz, (CD₃)₂SO, 60 °C, δ) 1.60 (br s, 2H), 2.25 (br s, 5H), 2.57 (br s, 2H), 3.07 (br s, 2H), 3.20 (br s, 2H), 4.37 (br s, 2H), 5.13 (br s, 4H), 6.92 (br s, 2H), 7.04 (br s, 1H), 7.34 (br s, 11H) ; ¹³C NMR (125 MHz, (CD₃)₂SO, 60 °C, δ) 20.8, 28.9, 35.7, 52.1, 54.7, 55.8 (2C), 57.4, 66.8 (2C), 119.2, 121.6, 125.8, 127.5-129.1 (11C), 136.4 (2C), 141.9, 150.6, 156.8 (2C), 168.9 ; HRMS (ESI-Orbitrap) [M+H]⁺ calcd for C₃₁H₃₄N₃O₆, 544.2442 found 544.2440.

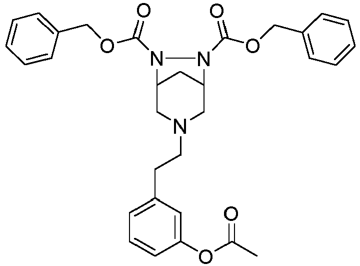

### Reductive amination of protected diaminopiperidines

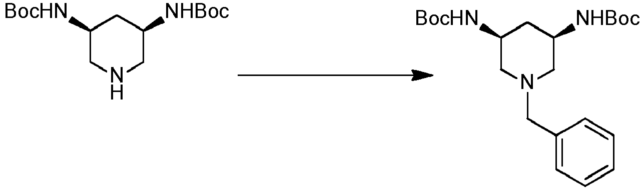

ECO02-056-C
ECO02-051-C (65 mg, 0.206 mmol) was solubilized in MeOH (1.5 mL) and DCM (1.5 mL). Freshly distilled benzaldehyde (328 mg, 0.309 mmol) and MgSO₄ (tip of spatula) were added. Mixture was stirred 1h under Ar at RT. NaBH(OAc)₃ (131 mg, 0.618 mmol), was added and mixture was stirred 1h. NaBH(OAc)₃ (87 mg, 0.412 mmol) was added and mixture was stirred overnight under Ar at RT. The reaction was monitored by TLC (not completed reaction). The solution was quenched with water, extracted with CH₂Cl₂. The organic layer was dried over MgSO₄, filtered and evaporated. Flash chromatography (CH₂Cl₂ to CH₂Cl₂/MeOH/NH₄OH 90/9/1) afforded **ECO02-056-C** (31 mg, 40%), white solid.
**ECO02-056-C di-tert-butyl (1-benzylpiperidine-3,5-diyl)dicarbamate :** 31 mg, 44%, white solid ;
   ¹H NMR (500 MHz, CD₃OD, 27 °C) δ 0.98 (q, *J* = 12.0 Hz, 1H), 1.30 (br s, 18H), 1.63 (br s, 2H), 1.91-1.96 (m, 1H), 2.82-2.86 (m, 2H), 3.45-3.49 (m, 4H), 7.10-7.17 (m, 1H), 7.21 (br s, 4H) ; ¹³C NMR (125 MHz, CD₃OD, 27 °C, δ) 28.7 (6C), 38.2, 47.7 (2C), 58.9 (2C), 63.4, 80.0 (2C), 128.4, 129.4 (2C), 130.4 (2C), 138.8, 157.6 (2C) ; HRMS (ESI-Orbitrap) [M+H]⁺ calcd for C₂₂H₃₆N₃O₄, 406.2700 found 406.2706. 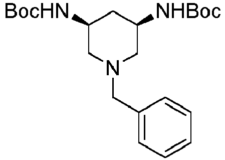
**ECO02-058-C di-tert-butyl (1-(furan-2-ylmethyl)piperidine-3,5-diyl)dicarbamate** : 41 mg, 51%, pale yellow solid ;
   ¹H NMR (500 MHz, CD₃OD, 27 °C) δ 1.07 (q, *J* = 11.8 Hz, 1H), 1.43 (br s, 18H), 1.74-1.83 (m, 2H), 2.03 (br s, 1H), 2.93-2.99 (m, 2H), 3.56-3.66 (m, 4H), 6.27-6.30 (m, 1H), 6.36-6.39 (m, 1H), 7.46 (s, 1H) ; ¹³C NMR (125 MHz, CD₃OD, 27 °C) δ 27.3 (6C), 36.4, 46.2 (2C), 53.4, 56.9 (2C), 78.6 (2C), 108.8, 109.8, 142.1, 151.0, 156.1 (2C); HRMS (ESI-Orbitrap) [M+H]⁺ calcd for C₂₀H₃₄N₃O₅, 396.2488 found 396.2493. 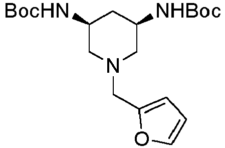
**ECO02-059-C di-tert-butyl (1-(4-(pyrrolidin-1-yl)benzyl)piperidine-3,5-diyl)dicarbamate :** 41 mg, 55%, off-white solid ;
   ¹H NMR (500 MHz, CD₃OD, 27 °C) δ 1.07 (q, *J* = 11.8 Hz, 1H), 1.42 (br s, 18H), 1.71 (br s, 2H), 1.97-2.08 (m, 5H), 2.90-2.99 (m, 2H), 3.23-3.28 (m, 4H), 3.46 (br s, 2H), 3.61 (br s, 2H), 6.55 (d, *J* = 8.5 Hz, 2H), 7.11 (d, *J* = 8.5 Hz, 2H) ; ¹³C NMR (125 MHz, CD₃OD, 27 °C) δ 24.9 (2C), 27.3 (6C), 36.8, 46.2 (2C), 48.1, 48.2, 57.1 (2C), 61.6, 78.6 (2C), 111.2 (2C), 123.1, 130.1 (2C), 147.7, 156.1 (2C); HRMS (ESI-Orbitrap) [M+H]⁺ calcd for C₂₆H₄₃N₄O₄, 475.3279 found 475.3278. 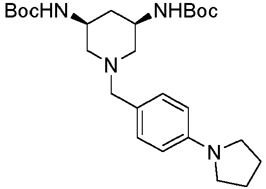
**ECO02-060-C di-tert-butyl (1-((6-methoxy-1-methyl-1H-indol-3-yl)methyl)piperidine-3,5-diyl)dicarbamate** : 25 mg, 32%, white solid ;
   ¹H NMR (500 MHz, CDCl₃, 27 °C) δ 1.42 (br s, 18H), 1.98 (br s, 2H), 2.20 (br s, 2H), 2.76 (br s, 2H), 3.56-3.79 (m, 7H), 3.89 (s, 3H), 4.67 (br s, 2H, NH), 6.76 (d, *J* = 2.2 Hz, 2H), 6.79 (d, *J* = 2.2 Hz, 1H), 6.80 (d, *J* = 2.2 Hz, 1H), 6.85 (br s, 1H), 7.55 (d, *J* = 8.9 Hz, 1H) ; ¹³C NMR (125 MHz, CDCl₃, 27 °C) δ 28.3 (6C), 32.7, 46.2 (2C), 53.1, 55.8, 57.8 (3C), 79.2 (2C), 92.9, 109.1, 110.5, 120.2, 122.7, 127.2, 137.8, 155.1 (2C), 156.5 ; HRMS (ESI-Orbitrap) [M+H]⁺ calcd for C₂₆H₄₁N₄O₅, 489.3071 found 489.3077. 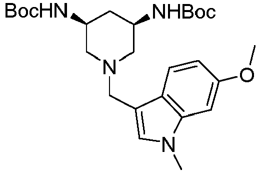
**ECO02-062-C di-tert-butyl 1-(4-(dimethylamino)benzyl)piperidine-3,5-diyl)dicarbamate** : 26 mg, 28%, white solid ;
   ¹H NMR (500 MHz, CDCl₃, 27 °C) δ 1.20-1.45 (m, 18H), 1.67 (br s, 1H), 2.01 (br s, 3H), 2.70 (br s, 2H), 2.91 (s, 6H), 3.45 (br s, 2H), 3.72 (br s, 2H), 4.60 (br s, 2H, NH), 6.67 (d, *J* = 8.3 Hz, 2H), 7.12 (d, *J* = 8.3 Hz, 2H) ; ¹³C NMR (125 MHz, CDCl₃, 27 °C) δ 28.4 (6C), 36.2, 40.7 (2C), 46.2 (2C), 57.7 (2C), 61.8, 79.3 (2C), 112.5 (2C), 125.2, 130.0 (2C), 150.0, 155.0 (2C) ; HRMS (ESI-Orbitrap) [M+H]⁺ calcd for C₂₄H₄₁N₄O₄, 449.3122 found 449.3127.

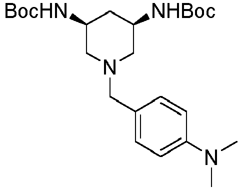

### Deprotection of diaminopiperidines

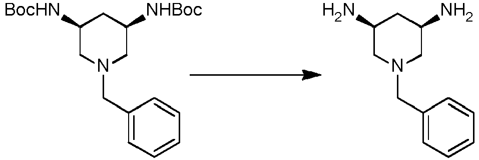

**ECO02-056-C** (31 mg, 0.074 mmol) was solubilized in HCl 4M/Dioxane (1 mL). Mixture was stirred under Ar, 0.5h at RT. Evaporation of the solvents crude gives **ECO02-063-C** (28 mg, quant.), white solid._ECO02-064-C and ECO02-065-C were purified by preparative HPLC using a C18 Hypersil column (elution gradient H₂O/MeCN 80/20 to 20/80).
**ECO02-063-C 1-benzylpiperidine-3,5-diamine trihydrochloride :** 28 mg, quant., white solid ;
   ¹H NMR (500 MHz, CD₃OD, 27 °C) δ 2.02 (q, *J* = 12.1 Hz, 1H), 2.66 (d, *J* = 11.7 Hz, 1H), 3.26 (t, *J* = 11.7 Hz, 2H), 3.67 (d, *J* = 4.6 Hz, 2H), 3.85-3.90 (m, 2H), 4.55 (s, 2H), 7.51-7.52 (m, 3H), 7.68-7.69 (m, 2H) ; ¹³C NMR (125 MHz, CD₃OD, 27 °C) δ 30.4, 43.4 (2C), 51.0 (2C), 60.8, 128.9 (2C), 129.9 (2C), 131.1 (2C); HRMS (ESI-Orbitrap) [M+H]⁺ calcd for C₁₂H₂₀N₃, 206.1652 found 206.1658. 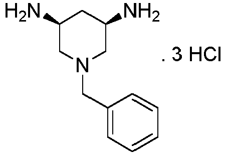
**ECO02-064-C 1-(3,5-dimethoxybenzyl)piperidine-3,5-diamine :** 21 mg, 95%, white solid ;
   ¹H NMR (500 MHz, CD₃OD , 27 °C) δ 1.56 (q, *J* = 11.8 Hz, 1H), 2.11 (t, *J* = 10.9 Hz, 2H), 2.52 (d, *J* = 11.6 Hz, 1H), 3.18-3.22 (m, 2H), 3.40-3.45 (m, 2H), 3.65 (s, 2H), 3.78 (s, 6H), 6.40-6.43 (m, 1H), 6.53-6.54 (m, 2H) ; ¹³C NMR (125 MHz, CD₃OD, 27 °C) δ 34.0, 47.5 (2C), 55.7 (2C), 55.8 (2C), 62.9, 110.4, 108.1 (2C), 140.5, 162.6 (2C) ; HRMS (ESI-Orbitrap) [M+H]⁺ calcd for C₁₄H₂₄N₃O₂, 266.1863 found 266.1862. 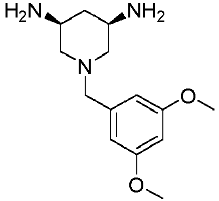
**ECO02-068-C 1-(isoquinolin-5-ylmethyl)piperidine-3,5-diamine trihydrochloride :** 6 mg, 83%, yellow paste ;
   ¹H NMR (500 MHz, CD₃OD , 27 °C) δ 1.63 (q, *J* = 11.8 Hz, 1H), 2.34 (t, *J* = 10.5 Hz, 1H), 2.52 (d, *J* = 11.7 Hz, 2H), 3.26-3.29 (m, 2H), 3.44-3.46 (m, 2H), 4.33 (s, 2H), 8.06 (t, *J* = 8.2, 7.2 Hz, 1H), 8.28 (d, *J* = 7.1 Hz, 1H), 8.52 (d, *J* = 8.3 Hz, 1H), 8.66 (d, *J* = 6.7 Hz, 1H), 8.90 (d, *J* = 6.6 Hz, 1H), 9.84 (s, 1H) ; ¹³C NMR (125 MHz, CD₃OD, 27 °C) δ 32.3, 45.8 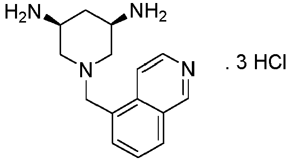 (2C), 54.0 (2C), 58.0, 123.1, 128.3, 130.5, 130.6, 131.0, 132.0, 138.0, 138.3, 147.2 ; HRMS (ESI-Orbitrap) [M+H]⁺ calcd for C₁₅H₂₁N₄, 257.1765 found 257.1761.
**ECO02-069-C 1-(4-(dimethylamino)benzyl)piperidine-3,5-diamine trihydrochloride** : 20 mg, quant., beige solid ;
   ¹H NMR (500 MHz, D₂O, 27 °C) δ 1.86 (q, *J* = 12.1 Hz, 1H), 2.67-2.70 (m, 1H), 2.92 (t, *J* = 11.7 Hz, 2H), 3.36 (s, 6H), 3.63 (s, 6H), 3.65-3.69 (m, 2H), 3.72-3.77 (m, 2H), 4.39 (s, 2H), 7.76 (s, 4H) ; ¹³C NMR (125 MHz, D₂O, 27 °C) δ 43.2 (2C), 43.7, 46.4 (2C), 60.3 (2C), 62.9, 120.5 (2C), 129.2 (2C), 132.9, 143.0 ; HRMS (ESI-Orbitrap) [M+H]⁺ calcd for C₁₄H₂₅N₄, 249.2078 found 249.2074. 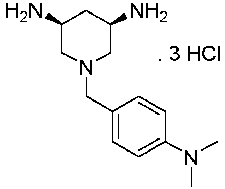
**ECO03-001-B 1-(phenylsulfonyl)piperidine-3,5-diamine :** 43 mg, quant., colorless oil ;
   ¹H NMR (500 MHz, CD₃OD , 27 °C) δ 1.67 (q, *J* = 11.9 Hz, 1H), 2.49 (t, *J* = 11.3 Hz, 2H), 2.57 (d, *J* = 11.8 Hz), 3.52-3.55 (m, 2H), 4.15-4.19 (m, 2H), 7.67-7.71 (m, 2H), 7.76 (t, *J* = 7.4 Hz, 1H), 7.87 (d, *J* = 7.5 Hz, 2H) ; ¹³C NMR (125 MHz. CD₃OD. 27 °C) δ 31.8 45.6 (2C), 46.9 (2C), 127.2 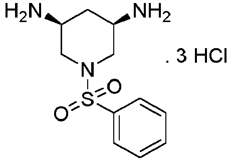 (2C), 128.4 (2C), 133.5, 136.1 ; HRMS (ESI-Orbitrap) [M+H]⁺ calcd for C₁₁H₁₈N₃O₂S, 366.2023 found 366.2020.

### Acylation of diaminopiperidines

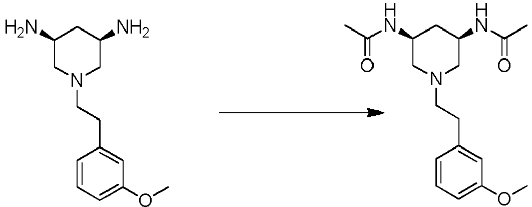

**ECO01-026-C2** (100 mg, 0.401 mmol) was solubilized in acetic anhydride (2.30 mL). Anhydrous pyridine (0.30 mL) and DMAP (2.4 mg, 0.020 mmol) were added. Mixture was stirred under Ar, 3h at RT. The reaction was monitored by TLC until disappearance of the initial product. In an ice bath, the solution was quenched with NaHCO₃, extracted with Ethyl Acetate. The organic layer was dried over MgSO₄, filtered and evaporated. Flash chromatography (CH₂Cl₂ to CH₂Cl₂/MeOH/NH₄OH 90/9/1) afforded **ECO02-072-C** (26 mg, 19%), white solid.
**ECO02-072-C N,N'-(1-(3-methoxyphenethyl)piperidine-3,5-diyl)diacetamide :** 26 mg, 19%, white solid ;
   ¹H NMR (500 MHz, CD₃OD , 27 °C) δ 1.21 (q, *J* = 10.5 Hz, 1H), 1.86 (t, *J* = 10.7 Hz, 2H), 1.94 (s, 6H), 2.11-2.13 (m, 1H), 2.66-2.69 (m, 2H), 2.77-2.80 (m, 2H), 3.11 (dd, *J* = 10.5, 4.0 Hz, 2H), 3.79 (s, 3H), 3.93-3.99 (m, 2H), 6.75 (dd, *J* = 8.1, 2.6 Hz, 1H), 6.79-6.80 (m, 2H), 7.18 (t, *J* = 8.1 Hz, 1H) ; ¹³C NMR (125 MHz, CD₃OD, 27 °C) δ 21.2 (2C), 32.8, 35.8, 45.2 (2C), 54.2, 57.0 (2C), 59.4, 111.2, 114.0, 120.7, 129.0, 141.5, 159.9, 171.3 (2C) ; HRMS (ESI-Orbitrap) [M+H]⁺ calcd for C₁₈H₂₈N₃O₃, 334.2125 found 334.2125.

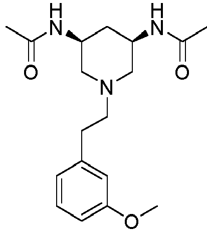

### Sulfonylation of diaminopiperidines

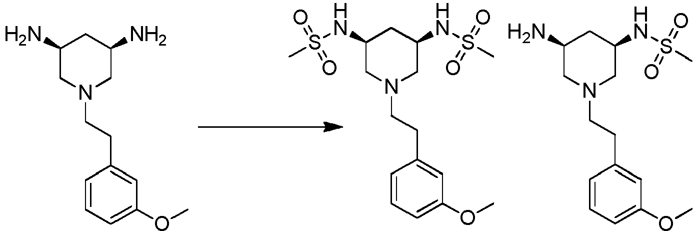

**ECO02-072-C** (100 mg, 0.401 mmol) was solubilized in anhydrous CH₂Cl₂ (1 mL). Anhydrous pyridine (36 µL, 0.441 mmol) was added. At 0°C, methane sulfonyl chloride (68 µL, 0.882 mmol) was added. Mixture was stirred 10 min at 0°C and at RT for 7h under Ar. The reaction was monitored by TLC until disappearance of the initial product. Mixture was quenched with water and NaOH 3M, extracted with CH₂Cl₂. The organic layer was washed with a saturated aqueous solution of NaCl, dried over MgSO₄, filtered and evaporated. Flash chromatography (CH₂Cl₂ to CH₂Cl₂/MeOH/NH₄OH 90/9/1) afforded mono-substitued compound **ECO02-073-C** (5.5 mg) as a yellow oil. The aqueous layer was adjusted at pH = 7 with HCI 3M and extracted with Ethyl Acetate. The organic layer was washed with NaCl sat, dried over over MgSO₄, filtered and evaporated. Crude product was obtained to give **ECO02-073-B2** (84 mg, 52%), white solid.
**ECO02-073-C *rac*-*cis*-N-(5-amino-1-(3-methoxyphenethyl)piperidin-3-yl) methanesulfonamide** : 5.5 mg, yellow oil;
   ¹H NMR (500 MHz, CD₃OD , 27 °C) δ 1.11 (q, *J* = 11.8 Hz, 1H), 1.78 (t, *J* = 10.6 Hz, 1H), 1.90 (t, *J* = 10.7 Hz, 1H), 2.21-2.24 (m, 1H), 2.67-2.69 (m, 2H), 2.79-2.80 (m, 2H), 2.90-2.94 (m, 1H), 2.97 (s, 3H), 3.04-3.08 (m, 1H), 3.15-3.19 (m, 1H), 3.41-3.46 (m, 1H), 3.79 (s, 3H), 6.75-6.81 (m, 3H), 7.19 (t, *J* = 8.0 Hz, 1H) ; ¹³C NMR (125 MHz, CD₃OD, 27 °C) δ 34.1, 41.4, 41.5, 48.1, 50.5, 55.6, 60.3, 60.8, 61.2, 112.6, 115.4, 122.1, 130.5, 142.9, 161.3 ; HRMS (ESI-Orbitrap) [M+H]⁺ calcd for C₁₅H₂₆N₃O₃S, 328.1689 found 328.1688. 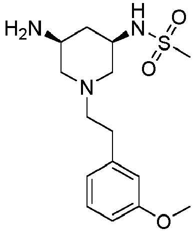
**ECO02-073-B2 N,N'-(1-(3-methoxyphenethyl)piperidine-3,5-diyl)dimethanesulfonamide :** 84 mg, 52%, white solid ;
   ¹H NMR (500 MHz, CD₃OD , 27 °C) δ 1.23 (q, *J* = 12.2 Hz, 1H), 1.90 (t, *J* = 10.8 Hz, 2H), 2.31-2.33 (m, 1H), 2.69-2.72 (m, 2H), 2.79-2.82 (m, 2H), 2.99 (s, 6H), 3.14-3.18 (m, 2H), 3.43-3.48 (m, 2H), 3.79 (s, 3H), 6.75-6.77 (m, 1H), 6.80-6.82 (m, 2H), 7.19 (t, *J* = 8.1 Hz, 1H) ; ¹³C NMR (125 MHz, CD₃OD, 27 °C) δ 32.8, 38.7, 40.0 (2C), 50.5 (2C), 54.2, 58.5 (2C), 59.1, 111.3, 114.0, 120.7, 129.0, 141.5, 160.0 ; HRMS (ESI-Orbitrap) [M+H]⁺ calcd for C₁₆H₂₈N₃O₅S₂, 406.1465 found 406.1466.

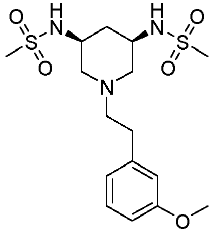

### Carbamoylation of diaminopiperidines

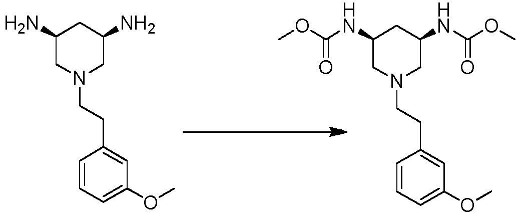

**ECO01-026-C2** (100 mg, 0.401 mmol) was solubilized in anhydrous pyridine (0.8 mL). At 0°C, methyl chloroformate was added (94 µL, 1.2 mmol). Mixture was stirred 10 min at 0°C and at RT for 4h under Ar. The reaction was monitored by TLC until disappearance of the initial product. Mixture was quenched with water, extracted with CH₂Cl₂. The organic layer with a saturated aqueous solution of NaCl, dried over MgSO₄, filtered and evaporated. Flash chromatography (Cyclohexane to Ethyl Acetate) afforded **ECO02-074-C** (87 mg, 59 %), white solid.
**ECO02-074-C dimethyl (1-(3-methoxyphenethyl)piperidine-3,5-diyl)dicarbamate** : 87 mg, 59%, white solid ;
   ¹H NMR (500 MHz, CD₃OD , 27 °C) δ 1.19 (q, *J* = 11.8 Hz, 1H), 1.84 (t, *J* = 10.7 Hz, 2H), 2.12-2.14 (m, 1H), 2.66-2.68 (m, 2H), 2.77-2.79 (m, 2H), 3.09-3.11 (m, 2H), 3.60-3.72 m, 8H), 3.79 (s, 3H), 6.75-6.77 (m, 1H), 6.79-6.80 (m, 2H), 7.19 (t, *J* = 8.1 Hz, 1H) ; ¹³C NMR (125 MHz, CD₃OD, 27 °C) δ 34.2, 37.9, 48.2 (2C), 52.4 (2C), 55.6, 58.9 (2C), 60.9, 112.6, 115.4, 122.1, 130.4, 142.9, 158.8, 161.3 (2C) ; HRMS (ESI-Orbitrap) [M+H]⁺ calcd for C₁₈H₂₈N₃O₅, 366.2023 found 366.2020.

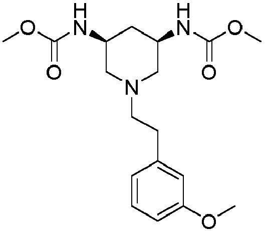

### Monocarbamoylation of diaminopiperidines

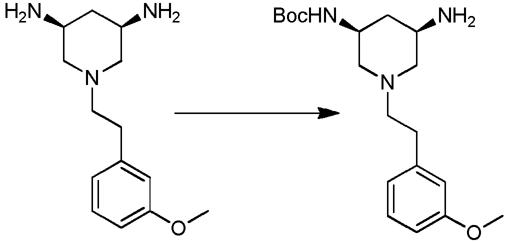

**ECO01-026-C2** (100 mg, 0.401 mmol), was solubilized in THF (4 mL) and NaOH 1M (4 mL). BoC₂O (219 mg, 1.00 mmol) was added. Mixture was stirred 2h under Ar at RT. The reaction was monitored by TLC until disappearance of the initial product. THF was evaporated. Mixture was extracted with Ethyl Acetate. The organic layer with a saturated aqueous solution of NaCl, dried over MgSO₄, filtered and evaporated. Crude product was purified on SiO₂ (CH₂Cl₂ to CH₂Cl₂/MeOH/NH₄OH 90/9/1) afforded **ECO02-078-C** (24 mg), colorless oil.
**ECO02-078-C tert-butyl 5-amino-1-(3-methoxyphenethyl)piperidin-3-yl)carbamate** : 24 mg, byproduct, colorless oil ;
   ¹H NMR (500 MHz, CD₃OD , 27 °C) δ 1.07 (q, *J* = 11.8 Hz, 1H), 1.46 (s, 9H), 1.82 (td, *J* = 10.5, 4.8 Hz, 2H), 2.13-2.17 (m, 1H), 2.66-2.69 (m, 2H), 2.78-2.81 (m, 2H), 2.93-2.99 (m, 1H), 3.05-3.11 (m, 2H), 3.62-.365 (m, 1H), 3.79 (s, 3H), 6.75-6.80 (m, 3H), 7.19 (t, *J* = 8.1 Hz, 1H) ; ¹³C NMR (125 MHz, CD₃OD, 27 °C) δ 28.8 (3C), 34.1, 39.9, 47.6, 48.1, 55.6, 58.9, 60.7, 60.9, 80.2, 112.6, 115.5, 122.1, 130.4, 142.9, 157.7, 161.3; HRMS (ESI-Orbitrap) [M+H]⁺ calcd for C₁₉H32N₃O₃, 350.2438 found 350.2439.

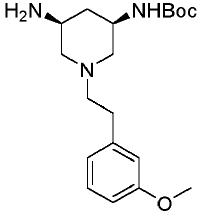

### Sulfonylation of protected diaminopiperidines

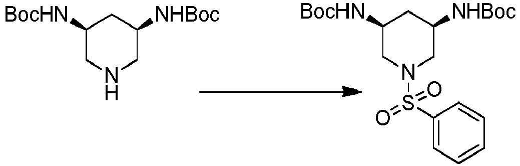

ECO02-051-C (79 mg, 0.251 mmol) was suspended in CH₂Cl₂ (5 mL) and anhydrous pyridine (22 µL, 0.276 mmol). At 0 °C, benzenesulfonylchloride (35 µL, 0.276 mmol) was added. Mixture was stirred overnight under Ar at RT. At 0°C, benzenesulfonylchloride (35 µL, 0.276 mmol) was added and mixture was stirred 6h under Ar at RT. Water and NaOH 2M were added. The aqueous layer extracted with Ethyl Acetate. The organic layer with a saturated aqueous solution of NaCl, dried over MgSO₄, filtered and evaporated. Flash chromatography (Cyclohexane to Cyclohexane/Ethyl Acetate 5/5) afforded **ECO02-081-C** (60 mg, 52%), white solid.
**ECO02-081-C di-tert-butyl (1-(phenylsulfonyl)piperidine-3,5-diyl)dicarbamate** : 60 mg, 52%, white solid ;
   ¹H NMR (500 MHz, CD₃OD , 27 °C) δ 1.11 (q, *J* = 12.1 Hz, 1H), 1.46 (s, 18H), 1.98-2.02 (m, 3H), 3.57 (br s, 2H), 3.80-3.90 (m, 2H), 7.62-7.65 (m, 2H), 7.65-7.70 (m, 1H), 7.83 (d, *J* = 7.3 Hz, 2H) ; ¹³C NMR (125 MHz, CD₃OD, 27 °C) δ 27.2 (6C), 35.8; 46.0 (2C), 49.5 (2C), 79.0 (2C), 127.2 (2C), 129.0 (2C), 132.8 , 136.8, 156.0 (2C)

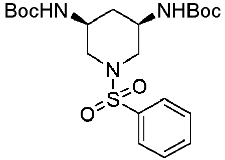

### Preparation of ECO03-03-C

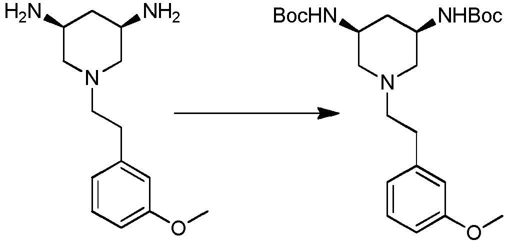

**ECO01-026-C2** (100 mg, 0.401 mmol) was solubilized in THF (4 mL) and NaOH 1M (4 mL). BoC₂O (394 mg, 1.80 mmol) was added. Mixture was stirred 2h under Ar at RT. The reaction was monitored by TLC until disappearance of the initial product. THF was evaporated. Mixture was extracted with Ethyl Acetate. The organic layer with a saturated aqueous solution of NaCl, dried over MgSO₄, filtered and evaporated. Flash chromatography (Cyclohexane to Cyclohexane/ Ethyl Acetate 6/4) afforded **ECO03-03-C** (110 mg, 61%).
**ECO03-03-C di-tert-butyl (1-(3-methoxyphenethyl)piperidine-3,5-diyl)dicarbamate** : 110 mg, 61%, white solid ;
   ¹H NMR (500 MHz, CD₃OD , 27 °C) δ 1.14 (q, *J* = 11.8 Hz, 1H), 1.45 (s, 18H), 1.82 (t, *J* = 10.6 Hz, 2H), 2.03-2.11 (m, 1H), 2.64-2.67 (m, 2H), 2.77-2.81 (m, 2H), 3.04-3.09 (m, 2H), 3.62-3.65 (m, 2H), 3.79 (s, 3H), 6.73-6.77 (m, 1H), 6.79-6.81 (m, 2H), 7.18 (t, *J* = 8.1 Hz, 1H) ; ¹³C NMR (125 MHz, CD₃OD, 27 °C) δ 28.8 (6C), 34.2, 38.0, 47.7 (2C), 55.6, 59.0 (2C), 60.9, 80.1 (2C), 112.6, 115.4, 122.1, 130.4, 143.0, 157.7, 161.3 (2C) ; HRMS (ESI-Orbitrap) [M+H]⁺ calcd for C₂₄H₄₀N₃O₅, 450.2962 found 450.2949.

### Preparation of ECO02-045-C

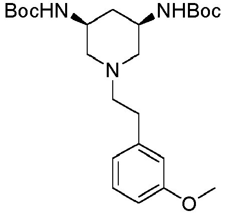

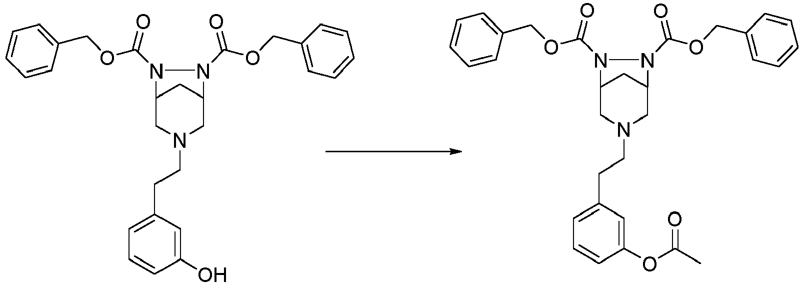

**ECO02-045-C dibenzyl 3-(3-acetoxyphenethyl)-3,6,7-triazabicyclo[3.2.1]octane-6,7-dicarboxylate** : 105 mg, quant., transparent oil ;
¹H NMR (500 MHz, (CD₃)₂SO, 60 °C, δ) 1.60 (br s, 2H), 2.25 (br s, 5H), 2.57 (br s, 2H), 3.07 (br s, 2H), 3.20 (br s, 2H), 4.37 (br s, 2H), 5.13 (br s, 4H), 6.92 (br s, 2H), 7.04 (br s, 1H), 7.34 (br s, 11H) ; ¹³C NMR (125 MHz, (CD₃)₂SO, 60 °C, δ) 20.8, 28.9, 35.7, 52.1, 54.7, 55.8 (2C), 57.4, 66.8 (2C), 119.2, 121.6, 125.8, 127.5-129.1 (11C), 136.4 (2C), 141.9, 150.6, 156.8 (2C), 168.9 ; HRMS (ESI-Orbitrap) [M+H]⁺ calcd for C₃₁H₃₄N₃O₆, 544.2442 found 544.2440.

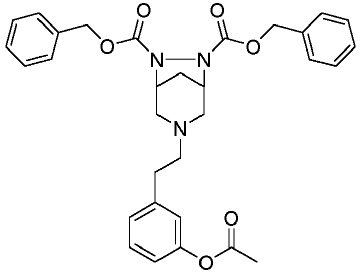

## Claims

1. A compound of formula (I), a pharmaceutically acceptable salt, solvate or hydrate thereof, enantiomers, mixture of enantiomers, diastereoisomers and mixture of diasteroisomers thereof: 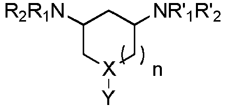 wherein:
n is 0 or 1,
X is CH or N,
Y is OR₃; NR₄R₅, or R₆,
R₁ and R'₁ are H, or R₁ and R₂ and/or R'₁ and R'₂ form together a (C₃-C₈)heterocyclyl,
R₂ and R'₂ are independently one from the other H, (C₁-C₆)alkyl, aryl, heteroaryl, (C₃-C₈)heterocyclyl, (C₃-C₈)carbocyclyl, (C₁-C₆)alkyl-aryl, (C₁-C₆)alkyl-heteroaryl, C(O)-Q, or SO₂-Z,
Q is H, (C₁-C₆)alkyl, aryl, heteroaryl, (C₃-C₈)carbocyclyl, (C₃-C₈)heterocyclyl, (C₁-C₆)alkyl-aryl, (C₁-C₆)alkyl-heteroaryl, (C₁-C₆)alkyl-(C₃-C₈)heterocyclyl, (C₁-C₆)alkyl-(C₃-C₈)carbocyclyl, NRₐR_{b} or OR_{c},
Rₐ and R_{b} are independently one from the other H, (C₁-C₆)alkyl, aryl, heteroaryl, (C₃-C₈)heterocyclyl, (C₃-C₈)carbocyclyl, (C₁-C₆)alkyl-aryl, (C₁-C₆)alkyl-heteroaryl, or Rₐ and R_{b} form together a (C₃-C₈)heterocyclyl,
R_{c} is (C₁-C₆)alkyl, (C₃-C₈)heterocyclyl, (C₃-C₈)carbocyclyl, aryl, heteroaryl, (C₁-C₆)alkyl-aryl, or (C₁-C₆)alkyl-heteroaryl,
Z is (C₁-C₆)-alkyl, aryl, heteroaryl, NRₐR_{b}, or CF₃,
R₃ is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, or (C₁-C₆)alkyl-heteroaryl-(C₁-C₆)alkyl-C(O)-aryl,
R₄ and R₅ are independently one from the other H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-V,
R₄ and R₅ form together a (C₃-C₈)heterocyclyl or a heteroaryl, or one of R₄ or R₅ is - CH(R₇)-CO-V,
V is H, (C₁-C₆)alkyl, aryl, heteroaryl, (C₃-C₈)carbocyclyl, (C₃-C₈)heterocyclyl, (C₁-C₆)alkyl-aryl, (C₁-C₆)alkyl-heteroaryl, (C₁-C₆)alkyl-(C₃-C₈)heterocyclyl, (C₁-C₆)alkyl-(C₃-C₈)carbocyclyl, NR_{f}R_{g}, OR₁₀ or CH(R₁₁)-NH-COR₁₂,
R₁₀ is as defined for R_{c},
R₇ is the side chain of an amino-acid,
R₁₁ is (C1-C6) alkylamine,
R₁₂ is aryl,
R₆ is H, (C₁-C₆)alkyl, (C₃-C₈)heterocyclyl, (C₃-C₈)carbocyclyl, (C₉-C₁₀)carbocyclyl, aryl, heteroaryl, (C₁-C₆)alkyl-aryl, (C₁-C₆)alkyl-heteroaryl, , NR_{d}Rₑ, OR₉, C(O)-V, SO₂-W, or-CH(R₇)-CO-V,
R_{d} and Rₑ are independently one from the other H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, C(O)-(C₁-C₆)alkyl-(C₃-C₈)carbocyclyl, or R_{d} and Rₑ form together a (C₃-C₈)heterocyclyl,
R_{f} is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, or C(O)-(C₁-C₆)alkyl-(C₃-C₈)carbocyclyl,
R_{g} is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, or R_{f} and R_{g} form together a (C₃-C₈)heterocyclyl,
R₉ is H, (C₁-C₆)alkyl, (C₃-C₈)heterocyclyl, (C₃-C₈)carbocyclyl, aryl, heteroaryl, (C₁-C₆)alkyl-aryl, (C₁-C₆)alkyl-heteroaryl, C(O)-(C₁-C₆)alkyl, C(O)-aryl, C(O)-heteroaryl, C(O)-(C₃-C₈)carbocyclyl C(O)-(C₃-C₈)heterocyclyl, C(O)-(C₁-C₆)alkyl-aryl, C(O)-(C₁-C₆)alkyl-heteroaryl, C(O)-(C₁-C₆)alkyl-(C₃-C₈)heterocyclyl, or C(O)-(C₁-C₆)alkyl-(C₃-C₈)carbocyclyl,
W is as defined for Z,
for all radicals R₁ to R₁₂, R_{1'}, R_{2'}, Rₐ to R_{g}, Q, V, W and Z: said (C₃-C₈)heterocyclyl can be substituted by one or more groups selected from methyl, ethyl, isopropyl, hydroxy, methoxy, amino, fluoro, chloro, bromo and iodo,
said aryl may be substituted with one or more groups independently selected from the group consisting of alkyl, alkoxy, halogen, hydroxyl, amino, nitro, cyano, trifluoro, carboxylic acid or carboxylic ester,
said heteroaryl may be substituted with one or more groups independently selected from the group consisting of alkyl, alkoxy, halogen, hydroxyl, amino, nitro, cyano, trifluoro, carboxylic acid or carboxylic ester, and
said carbocyclyl may be substituted with one or more groups selected from the group consisting of methyl, ethyl, isopropyl, hydroxy, methoxy, amino, fluoro, chloro, bromo and iodo,
for use in the treatment of HIV infection.

2. The compound for use according to claim 1, wherein NR₁R₂ and NR'₁R'₂ are in *cis* configuration.

3. The compound for use according to claim 1 or 2, wherein R₁, R'₁, R₂ and R'₂ are H.

4. The compound for use according to any of claims 1 to 3, wherein n = 0, X is CH and Y is OR₃ or NR₄R₅, advantageously OR₃.

5. The compound for use according to claim 4 wherein R₃ is aryl, (C₁-C₆)alkyl-heteroaryl-(C₁-C₆)alkyl-C(O)-aryl, advantageously (C₁)alkyl-heteroaryl-(C₁)alkyl-C(O)-aryl.

6. The compound for use according to any of claims 1 to 3, wherein n = 1, X is N and Y is R₆.

7. The compound for use according to claim 6 wherein R₆ is H; aryl; heteroaryl; (C₁-C₆)alkyl-aryl; (C₁-C₆)alkyl-heteroaryl; C(O)-(C₁-C₆)alkyl-aryl, C(O)-OR₁₀, where R₁₀ is (C₁-C₆)alkyl, advantageously *tert*-butyl, or (C₁-C₆)alkyl-aryl, advantageously benzyl; C(O)-V, or -CH(R₇)-CO-V, where R₇ is as defined in claim 1, advantageously benzyl.

8. The compound for use according to claim 7, wherein the aryl is chosen from among methoxy-phenyl, ethoxyphenyl, di-methoxy-phenyl, tri-methoxy-phenyl, 9,9'-Spirobi[9H-fluorene], p-cyclophanyl, (hydroxy-phenyl)amide, ethylphenyl and a phenylethanol; or the heteroaryl is a 3- or 5-indolyl, advantageously substituted with a methoxy group.

9. The compound for use according to any of the preceding claims selected in the list consisting of: 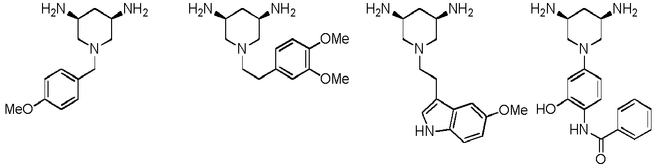 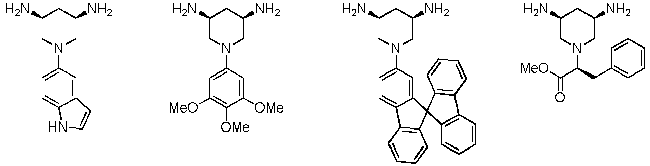 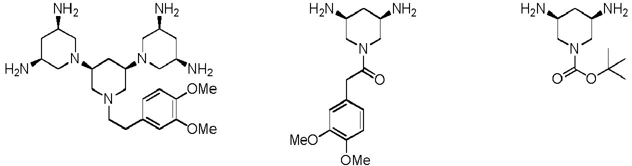 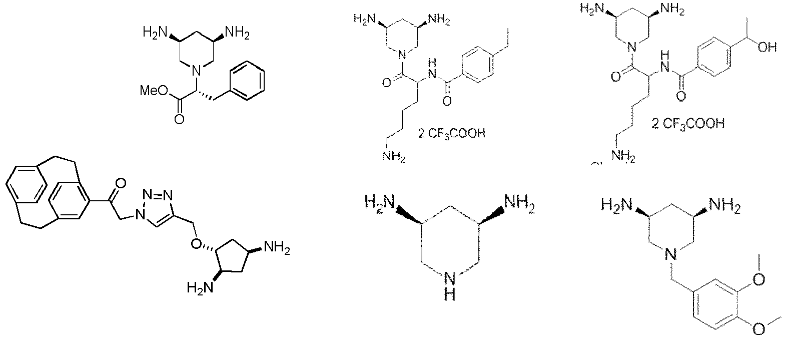 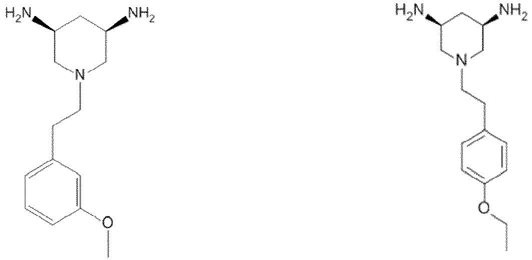 , and

10. The compound for use according to claim 1 or 2, wherein R₁ and R'₁ are H and at least one of R₂ and R'₂ is H, C(O)-Q or SO₂-Z wherein Q is OR_{c}, R_{c} is (C₁-C₆)alkyl, advantageously tert-butyl and wherein Z is (C₁-C₆)alkyl.

11. The compound for use according to claim 10, wherein n = 1, X is N and Y is R₆.

12. The compound for use according to claim 11 wherein R₆ is H; (C₁-C₆)alkyl-aryl;, (C₁-C₆)alkyl-heteroaryl or SO₂-W wherein W is aryl, advantageously phenyl, and advantageously, the compound is selected in the list consisting of:

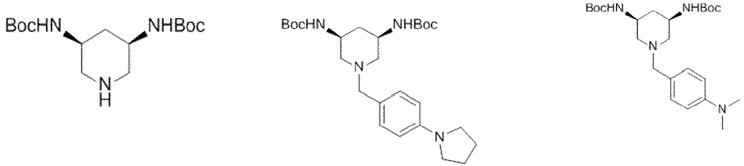

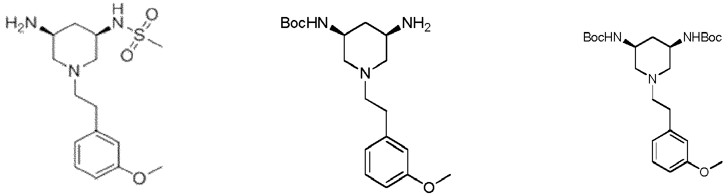

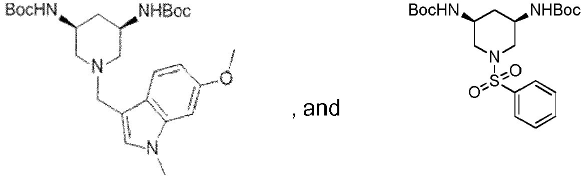

13. The compound for use according to any of the preceding claims in combination with an HIV therapy chosen from among immunotherapy, vaccines, antiretrovirals, or Highly Active Antiretroviral Therapy (HAART).

14. The compound for use according to claim 13, wherein the HIV therapy is chosen from among Lamivudine, Emtricitabine, Abacavir, Zidovudine, Didanosine, Stavudine, Adefovir, Tenofovir, Efavirenz, Etravirine, Nevirapine, Rilpivirine, Amprenavir, Fosamprenavir, Tipranavir, Lopinavir, Ritonavir, Indinavir, Saquinavir, Darunavir, Atazanavir, Nelfinavir, Raltegravir, Eviltegravir, Dolutégravir, Enfuvirtide, Maraviroc and combinations thereof.

15. A combination product comprising:
(i) at least one compound of formula (I) as defined in any of claims 1 to 12,
(ii) at least one antiretroviral of HIV,
for simultaneous, separate or sequential use as a medicament.

16. The combination product according to claim 15 for simultaneous, separate or sequential use in the treatment of HIV infection.

17. The combination product according to claim 16, wherein the at least one antiretroviral of HIV is chosen from among Lamivudine, Emtricitabine, Abacavir, Zidovudine, Didanosine, Stavudine, Adefovir, Tenofovir, Efavirenz, Etravirine, Nevirapine, Rilpivirine, Amprenavir, Fosamprenavir, Tipranavir, Lopinavir, Ritonavir, Indinavir, Saquinavir, Darunavir, Atazanavir, Nelfinavir, Raltégravir, Eviltegravir, Dolutégravir, Enfuvirtide, Maraviroc.

18. The compound of formula (I) as defined in claim 1 for use in the treatment of HIV infection in combination with one or more HIV-1 inducers chosen from among DNA methylation inhibitors, histone deacetylase inhibitors, and NF-kappa-B-inducers.

## Patentansprüche

1. Verbindung der Formel (I), ein pharmazeutisch verträgliches Salz, Solvat oder Hydrat davon, Enantiomere, Mischungen von Enantiomeren, Diastereoisomere und Mischungen von Diastereoisomeren davon: 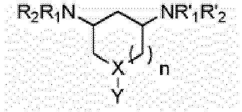 wobei:
n 0 oder 1 ist,
X CH oder N ist,
Y OR₃; NR₄R₅ oder R₆ ist,
R₁ und R'₁ H sind, oder R₁ und R₂ und/oder R'₁ und R'₂ zusammen ein (C₃-C₈)Heterocyclyl bilden,
R₂ and R'₂ unabhängig voneinander H, (C₁-C₆)Alkyl, Aryl, Heteroaryl, (C₃-C₈)Heterocyclyl, (C₃-C₈)Carbocyclyl, (C₁-C₆)Alkyl-aryl, (C₁-C₆)Alkyl-heteroaryl, C(O)-Q, oder SO₂-Z sind,
Q H, (C₁-C₆)Alkyl, Aryl, Heteroaryl, (C₃-C₈)Carbocyclyl, (C₃-C₈)Heterocyclyl, (C₁-C₆)Alkyl-aryl, (C₁-C₆)Alkyl-heteroaryl, (C₁-C₆)Alkyl-(C₃-C₈)heterocyclyl, (C₁-C₆)Alkyl-(C₃-C₈)carbocyclyl, NRₐR_{b} oder OR_{c} ist,
Rₐ und R_{b} unabhängig voneinander H, (C₁-C₆)Alkyl, Aryl, Heteroaryl, (C₃-C₈)Heterocyclyl, (C₃-C₈)Carbocyclyl, (C₁-C₆)Alkyl-aryl, (C₁-C₆)Alkyl-heteroaryl sind, oder Rₐ und R_{b} zusammen ein (C₃-C₈)Heterocyclyl bilden,
R_{c} (C₁-C₆) Alkyl, (C₃-C₈)Heterocyclyl, (C₃-C₈)Carbocyclyl, Aryl, Heteroaryl, (C₁-C₆)Alkyl-aryl, oder (C₁-C₆)Alkyl-heteroaryl ist,
Z (C₁-C₆)-Alkyl, Aryl, Heteroaryl, NRₐR_{b}, oder CF₃ ist,
R₃ H, (C₁-C₆) Alkyl, (C₃-C₈)Heterocyclyl, (C₃-C₈)Carbocyclyl, Aryl, Heteroaryl, (C₁-C₆)Alkyl-aryl, (C₁-C₆)Alkyl-heteroaryl, oder (C₁-C₆)Alkyl-heteroaryl- (C₁-C₆)alkyl-C(O)-aryl ist,
R₄ und R₅ unabhängig voneinander H, (C₁-C₆) Alkyl, (C₃-C₈)Heterocyclyl, (C₃-C₈)Carbocyclyl, Aryl, Heteroaryl, (C₁-C₆)Alkyl-aryl, (C₁-C₆)Alkyl-heteroaryl, C(O)-V sind,
R₄ und R₅ zusammen ein (C₃-C₈)Heterocyclyl oder ein Heteroaryl bilden, oder eines aus R₄ oder R₅ -CH(R₇)-CO-V ist,
V H, (C₁-C₆)Alkyl, Aryl, Heteroaryl, (C₃-C₈) Carbocyclyl, (C₃-C₈)Heterocyclyl, (C₁-C₆)Alkyl-aryl, (C₁-C₆)Alkyl-heteroaryl, (C₁-C₆)Alkyl-(C₃-C₈)Heterocyclyl, (C₁-C₆)Alkyl-(C₃-C₈) carbocyclyl, NR_{f}R_{g}, OR₁₀ oder CH(R₁₁)-NH-COR₁₂ ist,
R₁₀ wie für R_{c} definiert ist,
R₇ die Seitenkette einer Aminosäure ist,
R₁₁ (C1-C6)Alkylamin ist,
R₁₂ Aryl ist,
R₆ H, (C₁-C₆)Alkyl, (C₃-C₈)Heterocyclyl, (C₃-C₈)Carbocyclyl, (C₉-C₁₀)Carbocyclyl, Aryl, Heteroaryl, (C₁-C₆)Alkyl-aryl, (C₁-C₆)Alkyl-heteroaryl, NR_{d}Rₑ, OR₉, C(O)-V, SO₂-W, oder -CH(R₇)-CO-V ist,
R_{d} und Rₑ unabhängig voneinander H, (C₁-C₆)Alkyl, (C₃-C₈)Heterocyclyl, (C₃-C₈)Carbocyclyl, Aryl, Heteroaryl, (C₁-C₆)Alkyl-aryl, (C₁-C₆)Alkyl-heteroaryl, C(O)-(C₁-C₆)Alkyl, C(O)-Aryl, C(O)-Heteroaryl, C(O)-(C₃-C₈)Carbocyclyl C(O)-(C₃-C₈)Heterocyclyl, C(O)-(C₁-C₆)Alkyl-aryl, C(O)-(C₁-C₆)Alkyl-heteroaryl, C(O)-(C₁-C₆)Alkyl-(C₃-C₈)heterocyolyl, C(O)-(C₁-C₆)Alkyl-(C₃-C₈)carbocyclyl sind, oder R_{d} und Rₑ zusammen ein (C₃-C₈)Heterocyclyl bilden,
R_{f} H, (C₁-C₆)Alkyl, (C₃-C₈)Heterocyclyl, (C₃-C₈)Carbocyclyl, Aryl, Heteroaryl, (C₁-C₆)Alkyl-aryl, (C₁-C₆)Alkyl-heteroaryl, C(O)-(C₁-C₆)Alkyl, C(O)-Aryl, C(O)-Heteroaryl, C(O)-(C₃-C₈)Carbocyclyl C(O)-(C₃-C₈)Heterocyclyl, C(O)-(C₁-C₆)Alkyl-aryl, C(O)-(C₁-C₆)Alkyl-heteroaryl, C(O)-(C₁-C₆)Alkyl- (C₃-C₈)heterocyclyl, oder C(O)-(C₁-C₆)Alkyl-(C₃-C₈)carbocyclyl ist,
R_{g} H, (C₁-C₆)Alkyl, (C₃-C₈)Heterocyclyl, (C₃-C₈)Carbocyclyl, Aryl, Heteroaryl, (C₁-C₆)Alkyl-aryl, (C₁-C₆)Alkyl-heteroaryl ist, oder R_{f} und R_{g} zusammen ein (C₃-C₈)Heterocyclyl bilden,
R₉ H, (C₁-C₆)Alkyl, (C₃-C₈)Heterocyclyl, (C₃-C₈) Carbocyclyl, Aryl, Heteroaryl, (C₁-C₆)Alkyl-aryl, (C₁-C₆)Alkyl-heteroaryl, C(O)-(C₁-C₆)Alkyl, C(O)-Aryl, C(O)-Heteroaryl, C(O)-(C₃-C₈)CarbocyclylC(O)-(C₃-C₈) Heterocyclyl, C(O)-(C₁-C₆)Alkyl-aryl, C(O)-(C₁-C₆)Alkyl-heteroaryl, C(O)-(C₁-C₆) Alkyl-(C₃-C₈)heterocyclyl, oder C(O)-(C₁-C₆)Alkyl-(C₃-C₈)carbocyclyl ist,
W wie für Z definiert ist,
bei allen Radikalen R₁ bis R₁₂, R_{1'}, R_{2'}, Rₐ bis R_{g}, Q, V, W und Z:
das (C₃-C₈)Heterocyclyl durch eine oder mehrere Gruppen substituiert sein kann, ausgewählt aus Methyl, Ethyl, Isopropyl, Hydroxy, Methoxy, Amino, Fluor, Chlor, Brom und Jod,
das Aryl mit einer oder mehreren Gruppen substituiert sein kann, unabhängig ausgewählt aus der Gruppe bestehend aus Alkyl, Alkoxy, Halogen, Hydroxyl, Amino, Nitro, Cyano, Trifluor, Carbonsäure oder Carbonsäureester,
das Heteroaryl mit einer oder mehreren Gruppen substituiert sein kann, unabhängig ausgewählt aus der Gruppe bestehend aus Alkyl, Alkoxy, Halogen, Hydroxyl, Amino, Nitro, Cyano, Trifluor, Carbonsäure oder Carbonsäureester, und
das Carbocyclyl mit einer oder mehreren Gruppen substituiert sein kann, ausgewählt aus der Gruppe bestehend aus Methyl, Ethyl, Isopropyl, Hydroxy, Methoxy, Amino, Fluor, Chlor, Brom und Jod,
zur Verwendung in der Behandlung von HIV-Infektion.

2. Verbindung zur Verwendung nach Anspruch 1, wobei NR₁R₂ und NR'₁R'₂ in cis-Konfiguration sind.

3. Verbindung zur Verwendung nach Anspruch 1 oder 2, wobei R₁, R'₁, R₂ und R'₂ H sind.

4. Verbindung zur Verwendung nach einem der Ansprüche 1 bis 3, wobei n = 0, X CH ist und Y OR₃ oder NR₄R₅, vorteilhafterweise OR₃ ist.

5. Verbindung zur Verwendung nach Anspruch 4, wobei R₃ Aryl, (C₁-C₆)Alkyl-heteroaryl-(C₁-C₆)alkyl-C(O)-aryl, vorteilhafterweise (C₁)Alkyl-heteroaryl-(C₁)alkyl-C(O)-aryl ist.

6. Verbindung zur Verwendung nach einem der Ansprüche 1 bis 3, wobei n = 1, X N ist und Y R₆ ist.

7. Verbindung zur Verwendung nach Anspruch 6, wobei R₆ H; Aryl; Heteroaryl; (C₁-C₆)Alkyl-aryl; (C₁-C₆)Alkyl-heteroaryl; C(O)-(C₁-C₆)Alkyl-aryl, C(O)-OR₁₀, worin R₁₀ (C₁-C₆)Alkyl, vorteilhafterweise tert-Butyl, oder (C₁-C₆)Alkyl-aryl, vorteilhafterweise Benzyl ist; C(O)-V, oder -CH(R₇)-CO-V ist, worin R₇ wie in Anspruch 1 definiert, vorteilhafterweise Benzyl ist.

8. Verbindung zur Verwendung nach Anspruch 7, wobei das Aryl ausgewählt ist aus Methoxy-phenyl, Ethoxyphenyl, Di-methoxy-phenyl, Tri-methoxy-phenyl, 9,9'-Spirobi[9H-fluoren], p-Cyclophanyl, (Hydroxy-phenyl)amid, Ethylphenyl und einem Phenylethanol; oder das Heteroaryl ein 3- oder 5-Indolyl, vorteilhafterweise mit einer Methoxygruppe substituiert ist.

9. Verbindung zur Verwendung nach einem der vorstehenden Ansprüche, ausgewählt aus der Liste bestehend aus:

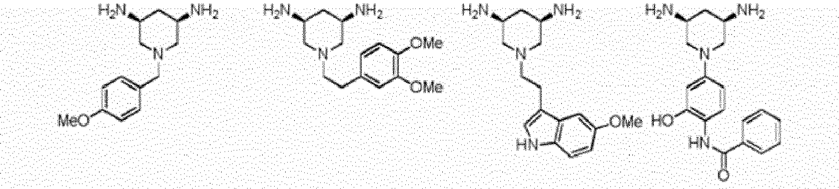

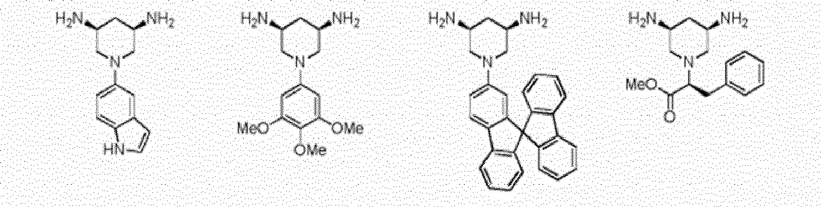

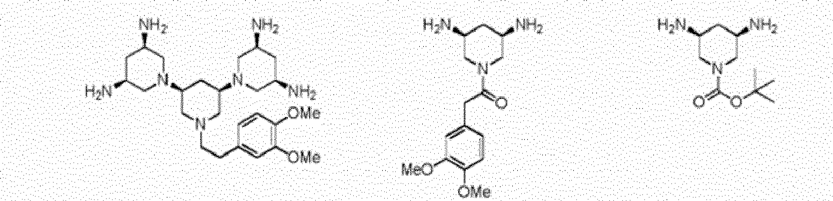

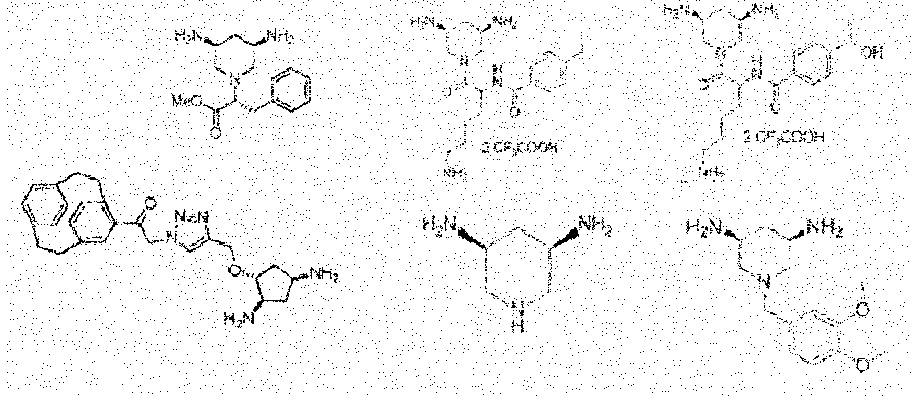

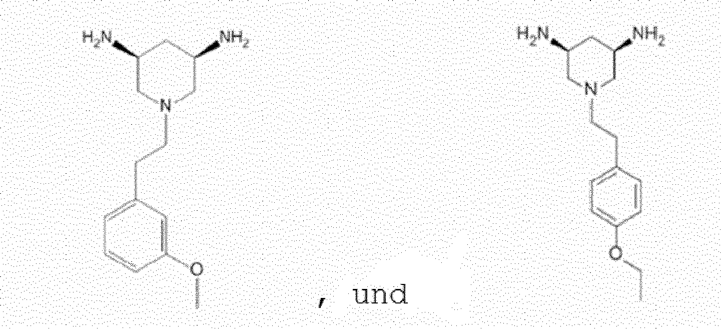

10. Verbindung zur Verwendung nach Anspruch 1 oder 2, wobei R₁ und R'₁ H sind und mindestens eines aus R₂ und R'₂ H, C(O)-Q oder SO₂-Z ist, wobei Q OR_{c} ist, R_{c} (C₁-C₆)Alkyl, vorteilhafterweise tert-Butyl ist, und wobei Z (C₁-C₆)Alkyl ist.

11. Verbindung zur Verwendung nach Anspruch 10, wobei n = 1, X N ist und Y R₆ ist.

12. Verbindung zur Verwendung nach Anspruch 11, wobei R₆ H; (C₁-C₆)Alkyl-aryl, (C₁-C₆)Alkyl-heteroaryl oder SO₂-W ist, wobei W Aryl, vorteilhafterweise Phenyl ist, und vorteilhafterweise die Verbindung ausgewählt ist aus der Liste bestehend aus:

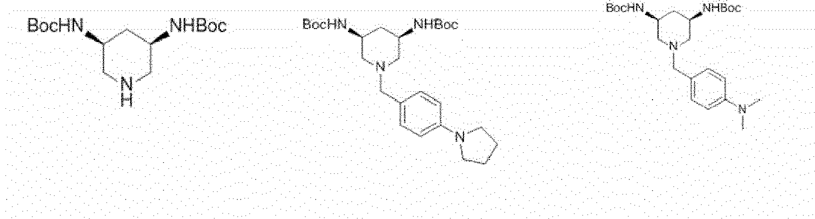

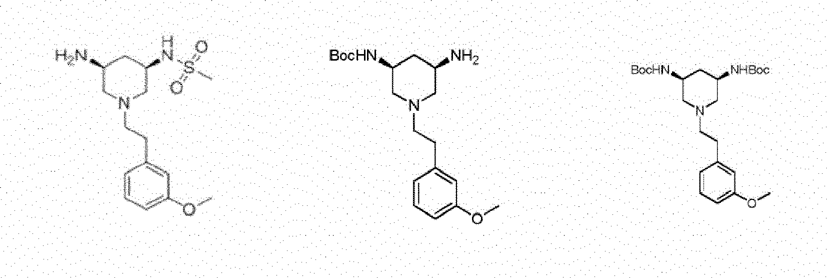

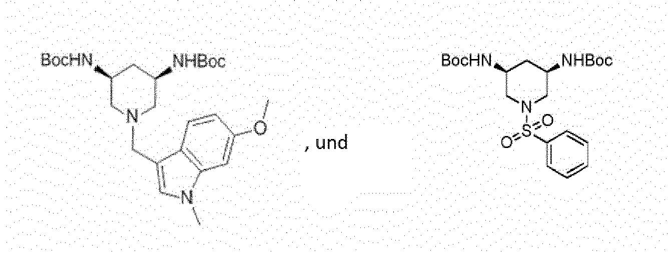

13. Verbindung zur Verwendung nach einem der vorstehenden Ansprüche in Kombination mit einer HIV-Therapie, ausgewählt aus Immuntherapie, Impfstoffen, antiretroviralen Wirkstoffen, oder hochaktiver antiretroviraler Therapie (HAART).

14. Verbindung zur Verwendung nach Anspruch 13, wobei die HIV-Therapie ausgewählt ist aus Lamivudin, Emtricitabin, Abacavir, Zidovudin, Didanosin, Stavudin, Adefovir, Tenofovir, Efavirenz, Etravirin, Nevirapin, Rilpivirin, Amprenavir, Fosamprenavir, Tipranavir, Lopinavir, Ritonavir, Indinavir, Saquinavir, Darunavir, Atazanavir, Nelfinavir, Raltegravir, Eviltegravir, Dolutegravir, Enfuvirtid, Maraviroc und Kombinationen davon.

15. Kombinationsprodukt, umfassend:
(i) mindestens eine Verbindung der Formel (I), wie in einem der Ansprüche 1 bis 12 definiert,
(ii) mindestens einen antiretroviralen HIV-Wirkstoff,
zur gleichzeitigen, getrennten oder sequentiellen Verwendung als ein Medikament.

16. Kombinationsprodukt nach Anspruch 15 zur gleichzeitigen, getrennten oder sequentiellen Verwendung in der Behandlung von HIV-Infektion.

17. Kombinationsprodukt nach Anspruch 16, wobei der mindestens eine antiretrovirale HIV-Wirkstoff ausgewählt ist aus Lamivudin, Emtricitabin, Abacavir, Zidovudin, Didanosin, Stavudin, Adefovir, Tenofovir, Efavirenz, Etravirin, Nevirapin, Rilpivirin, Amprenavir, Fosamprenavir, Tipranavir, Lopinavir, Ritonavir, Indinavir, Saquinavir, Darunavir, Atazanavir, Nelfinavir, Raltegravir, Eviltegravir, Dolutegravir, Enfuvirtid, Maraviroc.

18. Verbindung der Formel (I) wie in Anspruch 1 definiert zur Verwendung in der Behandlung von HIV-Infektion in Kombination mit einem oder mehreren HIV-1-Induktoren, ausgewählt aus DNA-Methylierungshemmern, Histon-Deacetylase-Hemmern, und NF-kappa-B-Induktoren.

## Revendications

1. Composé de formule (I), un sel, solvate ou hydrate pharmaceutiquement acceptables de celui-ci, les énantiomères, mélange d'énantiomères, les diastéréoisomères et mélange de diastéréoisomères de celui-ci : 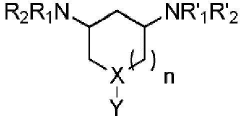 dans lequel :
n est 0 ou 1,
X est CH ou N,
Y est OR₃ ; NR₄R₅, ou R₆,
R₁ et R'₁ sont H, ou R₁ et R₂ et/ou R'₁ et R'₂ forment ensemble un (C₃-C₈)hétérocyclyle, R₂ et R'₂ sont indépendamment l'un de l'autre H, (C₁-C₆)alkyle, aryle, hétéroaryle, (C₃-C₈)hétérocyclyle, (C₃-C₈)carbocyclyle, (C₁-C₆)alkyl-aryle, (C₁-C₆)alkyl-hétéroaryle, C(O)-Q, ou SO₂-Z,
Q est H, (C₁-C₆)alkyle, aryle, hétéroaryle, (C₃-C₈)carbocyclyle, (C₃-C₈)hétérocyclyle, (C₁-C₆)alkyl-aryle, (C₁-C₆)alkyl-hétéroaryle, (C₁-C₆)alkyl-(C₃-C₈)hétérocyclyle, (C₁-C₆)alkyl-(C₃-C₈)carbocyclyle, NRₐR_{b} ou OR_{c},
Rₐ et R_{b} sont indépendamment l'un de l'autre H, (C₁-C₆)alkyle, aryle, hétéroaryle, (C₃-C₈)hétérocyclyle, (C₃-C₈)carbocyclyle, (C₁-C₆)alkyl-aryle, (C₁-C₆)alkyl-hétéroaryle, ou Rₐ et R_{b} forment ensemble un (C₃-C₈) hétérocyclyle, R_{c} est (C₁-C₆) alkyle, (C₃-C₈) hétérocyclyle, (C₃-C₈) carbocyclyle, aryle, hétéroaryle, (C₁-C₆) alkyl-aryle, ou (C₁-C₆) alkyl-hétéroaryle,
Z est (C₁-C₆) -alkyle, aryle, hétéroaryle, NRₐR_{b}, ou CF₃,
R₃ est H, (C₁-C₆) alkyle, (C₃-C₈)hétérocyclyle, (C₃-C₈)carbocyclyle, aryle, hétéroaryle, (C₁-C₆)alkyl-aryle, (C₁-C₆) alkyl-hétéroaryle, ou (C₁-C₆) alkyl-hétéroaryl-(C₁-C₆)alkyl-C(O)-aryle, R₄ et R₅ sont indépendamment l'un de l'autre H, (C₁-C₆) alkyle, (C₃-C₈) hétérocyclyle, (C₃-C₈)carbocyclyle, aryle, hétéroaryle, (C₁-C₆) alkyl-aryle, (C₁-C₆) alkyl-hétéroaryle, C(O)-V, R₄ et R₅ forment ensemble un (C₃-C₈)hétérocyclyle ou un hétéroaryle, ou l'un de R₄ ou R₅ est -CH(R₇)-CO-V,
V est H, (C₁-C₆) alkyle, aryle, hétéroaryle, (C₃-C₈) carbocyclyle, (C₃-C₈) hétérocyclyle, (C₁-C₆) alkyl-aryle, (C₁-C₆) alkyl-hétéroaryle, (C₁-C₆)alkyl-(C₃-C₈) hétérocyclyle, (C₁-C₆) alkyl-(C₃-C₈) carbocyclyle, NR_{f}R_{g}, OR₁₀ ou CH(R₁₁)-NH-COR₁₂,
R₁₀ est tel que défini pour R_{c},
R₇ est la chaîne latérale d'un acide aminé,
R₁₁ est (C1-C6)alkylamine,
R₁₂ est aryle,
R₆ est H, (C₁-C₆) alkyle, (C₃-C₈) hétérocyclyle, (C₃-C₈)carbocyclyle, (C₉-C₁₀)carbocyclyle, aryle, hétéroaryle, (C₁-C₆) alkyl-aryle, (C₁-C₆)alkyl-hétéroaryle, NR_{d}Rₑ, OR₉, C(O)-V, SO₂-W, ou -CH(R₇)-CO-V,
R_{d} et Rₑ sont indépendamment l'un de l'autre H, (C₁-C₆)alkyle, (C₃-C₈)hétérocyclyle, (C₃-C₈) carbocyclyle, aryle, hétéroaryle, (C₁-C₆) alkyl-aryle, (C₁-C₆) alkyl-hétéroaryle, C(O)-(C₁-C₆)alkyle, C(O)-aryle, C(O)-hétéroaryle, C(O)-(C₃-C₈)carbocyclyl C(O)-(C₃-C₈)hétérocyclyle, C(O)-(C₁-C₆)alkyl-aryle, C(O)-(C₁-C₆)alkyl-hétéroaryle, C(O)-(C₁-C₆)alkyl-(C₃-C₈)hétérocyclyle, C(O)-(C₁-C₆)alkyl-(C₃-C₈)carbocyclyle, ou R_{d} et Rₑ forment ensemble un (C₃-C₈)hétérocyclyle,
R_{f} est H, (C₁-C₆) alkyle, (C₃-C₈) hétérocyclyle, (C₃-C₈)carbocyclyle, aryle, hétéroaryle, (C₁-C₆)alkyl-aryle, (C₁-C₆)alkyl-hétéroaryle, C(O)-(C₁-C₆)alkyle, C(O)-aryle, C(O)-hétéroaryle, C(O)-(C₃-C₈)carbocyclyl C(O)-(C₃-C₈)hétérocyclyle, C(O)-(C₁-C₆)alkyl-aryle, C(O)-(C₁-C₆)alkyl-hétéroaryle, C(O)-(C₁-C₆)alkyl-(C₃-C₈)hétérocyclyle, ou C(O)-(C₁-C₆)alkyl-(C₃-C₈)carbocyclyle,
R_{g} est H, (C₁-C₆) alkyle, (C₃-C₈) hétérocyclyle, (C₃-C₈)carbocyclyle, aryle, hétéroaryle, (C₁-C₆)alkyl-aryle, (C₁-C₆) alkyl-hétéroaryle, ou R_{f} et R_{g} forment ensemble un (C₃-C₈) hétérocyclyle,
R₉ est H, (C₁-C₆) alkyle, (C₃-C₈) hétérocyclyle, (C₃-C₈)carbocyclyle, aryle, hétéroaryle, (C₁-C₆) alkyl-aryle, (C₁-C₈)alkyl-hétéroaryle, C(O)-(C₁-C₆)alkyle, C(O)-aryle, C(O)-hétéroaryle, C(O)-(C₃-C₈)carbocyclyl C(O)-(C₃-C₆)hétérocyclyle, C(O)-(C₁-C₆)alkyl-aryle, C(O)-(C₁-C₆)alkyl-hétéroaryle, C(O)-(C₁-C₆)alkyl-(C₃-C₈)hétérocyclyle, ou C(O)-(C₁-C₆)alkyl-(C₃-C₈)carbocyclyle,
W est tel que défini pour Z,
pour tous les radicaux R₁ à R₁₂, R₁', R₂', Rₐ à R_{g}, Q, V, W et Z :
ledit (C₃-C₈)hétérocyclyle peut être substitué par un ou plusieurs groupes tels que méthyle, éthyle, isopropyle, hydroxy, méthoxy, amino, fluoro, chloro, bromo et iodo,
ledit aryle peut être substitué par un ou plusieurs groupes indépendamment sélectionnés dans le groupe constitué d'un alkyle, alcoxy, halogène, hydroxyle, amino, nitro, cyano, trifluoro, acide carboxylique ou ester carboxylique,
ledit hétéroaryle peut être substitué par un ou plusieurs groupes indépendamment sélectionnés dans le groupe constitué d'un alkyle, alcoxy, halogène, hydroxyle, amino, nitro, cyano, trifluoro, acide carboxylique ou ester carboxylique, et
ledit carbocyclyle peut être substitué par un ou plusieurs groupes indépendamment sélectionnés dans le groupe constitué d'un méthyle, éthyle, isopropyle, hydroxy, méthoxy, amino, fluoro, chloro, bromo et iodo,
pour son utilisation dans le traitement d'une infection par le VIH.

2. Composé pour son utilisation selon la revendication 1, dans lequel NR₁R₂ et NR'₁R'₂ sont en configuration *cis.*

3. Composé pour son utilisation selon la revendication 1 ou 2, dans lequel R₁, R'₁, R₂ et R'₂ sont H.

4. Composé pour son utilisation selon l'une quelconque des revendications 1 à 3, dans lequel n = 0, X est CH et Y est OR₃ ou NR₄R₅, avantageusement OR₃.

5. Composé pour son utilisation selon la revendication 4, dans lequel R₃ est aryle, (C₁-C₆)alkyl-hétéroaryl-(C₁-C₆)alkyl-C(O)-aryle, avantageusement (C₁)alkyl-hétéroaryl-(C₁)alkyl-C(O)-aryle.

6. Composé pour son utilisation selon l'une quelconque des revendications 1 à 3, dans lequel n = 1, X est N et Y est R₆.

7. Composé pour son utilisation selon la revendication 6 dans lequel R₆ est H ; aryle ; hétéroaryle ; (C₁-C₆) alkyl-aryle ; (C₁-C₆)alkyl-hétéroaryle ; C(O)-(C₁-C₆) alkyl-aryle, C(O)-OR₁₀, où R₁₀ est un (C₁-C₆)alkyle, avantageusement tert-butyle, ou (C₁-C₆)alkyl-aryle, avantageusement benzyle ; C(O)-V, ou -CH(R₇)-CO-V, où R₇ est tel que défini comme dans la revendication 1, avantageusement benzyle.

8. Composé pour son utilisation selon la revendication 7, dans lequel l'aryle est choisi parmi les méthoxy-phényle, éthoxyphényle, di-méthoxy-phényle, tri-méthoxy-phényle, 9,9'-spirobi[9H-fluorène], p-cyclophanyle, (hydroxy-phényl)amide, éthylphényle et phényléthanol ; ou l'hétéroaryle est un 3- ou 5-indolyle, avantageusement substitué par un groupe méthoxy.

9. Composé pour son utilisation selon l'une quelconque des revendications précédentes, sélectionné dans la liste constituée de :

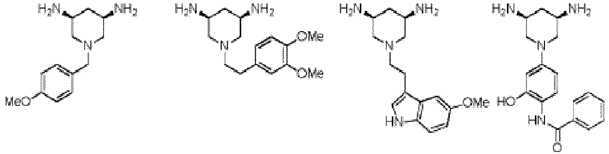

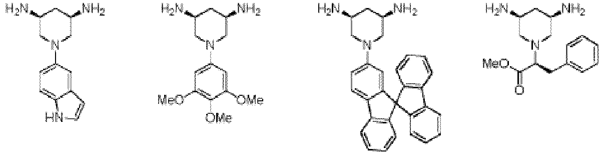

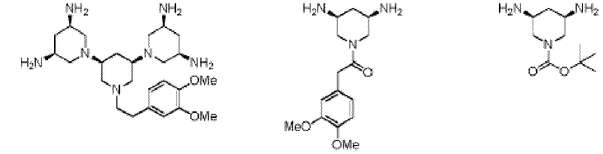

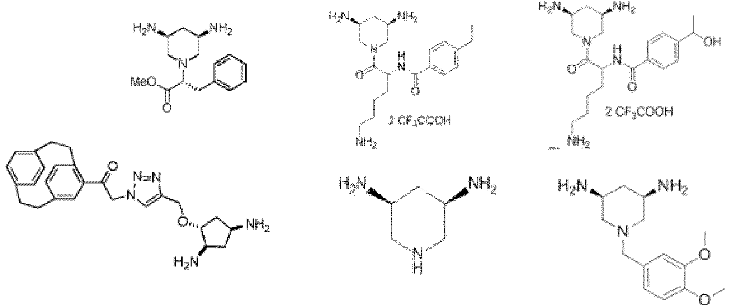

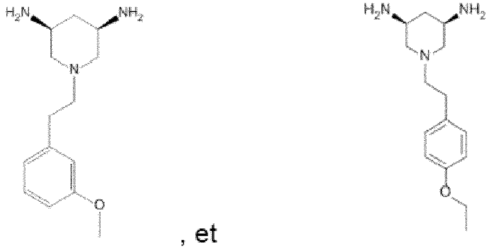

10. Composé pour son utilisation selon la revendication 1 ou 2, dans lequel R₁ et R'₁ sont H et au moins l'un de R₂ et R'₂ est H, C(O)-Q ou SO₂-Z dans lequel Q est OR_{c}, R_{c} est (C₁-C₆) alkyle, avantageusement tert-butyle et dans lequel Z est (C₁-C₆)alkyle.

11. Composé pour son utilisation selon la revendication 10, dans lequel n = 1, X est N et Y est R₆.

12. Composé pour son utilisation selon la revendication 11, dans lequel R₆ est H ; (C₁-C₆)alkyl-aryle ; (C₁-C₆) alkyl-hétéroaryle ou SO₂-W dans lequel W est aryle, avantageusement phényle, et avantageusement, le composé est sélectionné dans la liste constituée de :

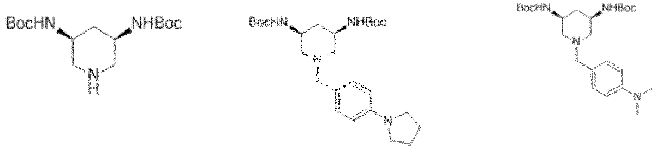

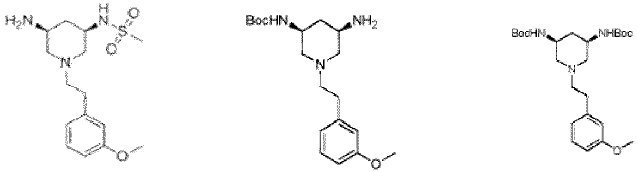

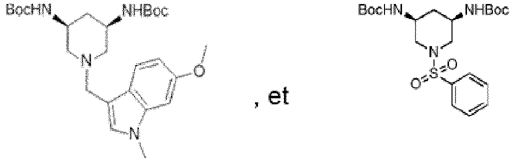

13. Composé pour son utilisation selon l'une quelconque des revendications précédentes en combinaison avec une thérapie contre le VIH choisie parmi une immunothérapie, des vaccins, des antirétroviraux, ou une thérapie antirétrovirale hautement active (HAART).

14. Composé pour son utilisation selon la revendication 13, dans laquelle la thérapie contre le VIH est choisie parmi la lamivudine, l'emtricitabine, l'abacavir, la zidovudine, la didanosine, la stavudine, l'adéfovir, le ténofovir, l'éfavirenz, l'étravirine, la névirapine, la rilpivirine, l'amprénavir, le fosamprenavir, le tipranavir, le lopinavir, le ritonavir, l'indinavir, le saquinavir, le darunavir, l'atazanavir, le nelfinavir, le raltegravir, l'éviltégravir, le dolutégravir, l'enfuvirtide, le maraviroc et des combinaisons de ceux-ci.

15. Produit de combinaison qui comprend :
(i) au moins un composé de formule (I) tel que défini dans l'une quelconque des revendications 1 à 12,
(ii) au moins un antirétroviral du VIH,
pour une utilisation simultanée, séparée ou séquentielle en tant que médicament.

16. Produit de combinaison selon la revendication 15 pour son utilisation simultanée, séparée ou séquentielle dans le traitement d'une infection par le VIH.

17. Produit de combinaison selon la revendication 16, dans lequel l'au moins un antirétroviral du VIH est choisi parmi la lamivudine, l'emtricitabine, l'abacavir, la zidovudine, la didanosine, la stavudine, l'adéfovir, le ténofovir, l'éfavirenz, l'étravirine, la névirapine, la rilpivirine, l'amprénavir, le fosamprenavir, le tipranavir, le lopinavir, le ritonavir, l'indinavir, le saquinavir, le darunavir, l'atazanavir, le nelfinavir, le raltegravir, l'éviltégravir, le dolutégravir, l'enfuvirtide, le maraviroc.

18. Composé de formule (I) tel que défini dans la revendication 1, pour son utilisation dans le traitement d'une infection par le VIH en combinaison avec un ou plusieurs inducteurs du VIH-1 choisis parmi des inhibiteurs de la méthylation de l'ADN, des inhibiteurs de l'histone désacétylase, et des inducteurs de NF-κB.
